# EUROPEAN PATENT APPLICATION

(11) **EP 3 730 489 A1**
(43) Date of publication of application: **28.10.2020**
(21) Application number: 19170974.0
(22) Date of filing: 25.04.2019
(51) Int. Cl.: C07D 409/04, A01N 43/76, A01N 43/78, C07D 413/04, C07D 417/04, C07D 471/04, C07D 487/04

(54) **HETEROARYL COMPOUNDS AS AGROCHEMICAL FUNGICIDES**

(71) Applicant: BASF SE, 67056 Ludwigshafen am Rhein (DE)
(72) Inventor: The designation of the inventor has not yet been filed
(74) Representative: BASF IP Association

(57) **Abstract**

The present invention relates to heteroaryl compounds of formula (I') or a compound in the form of a stereoisomer, an agriculturally acceptable salt, a tautomer, an isotopic form, a N-oxide or a S-oxide thereof. In addition, the present invention further relates to the use of a compound of formula (I) or an agriculturally acceptable salt, a stereoisomer, a tautomer, an isotopic form, a derivative or mixture thereof, as phyto fungicide.

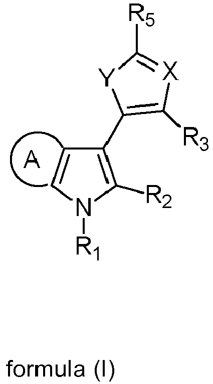

## Description

### FIELD OF THE INVENTION

The present invention relates to heteroaryl compounds of formula (I') or a compound in the form of a stereoisomer, an agriculturally acceptable salt, a tautomer, an isotopic form, a N-oxide or a S-oxide thereof. In addition, the present invention further relates to the use of a compound of formula (I) or an agriculturally acceptable salt, a stereoisomer, a tautomer, an isotopic form, a derivative or mixture thereof, as a phytofungicide.

### BACKGROUND OF THE INVENTION

The control of plant diseases and crop damage caused by fungal plant pathogens is extremely pertinent to achieve high crop yield and efficiency.

EP 0,697,172 A1 relates to a method for the control of insect or acarid pests by contacting said pests with a pesticidally effective amount of an indole compound.

US 2011207732 A1 discloses azaindole derivatives which are useful as medicaments.

One typical problem arising in the field of fungi control lies in the need to reduce the dosage rates of the active ingredient to reduce or avoid unfavourable environmental or toxicological effects whilst still allowing effective fungi control. Another problem that is being encountered concerns the need to have available fungicidal active agents which are effective against a broad spectrum of fungi. There is a continuous need for developing new fungicidal compounds which are effective in terms of activity spectrum, selectivity, sites of action, application rate, environmental safety while retarding or combatting resistance development. In many cases, in particular at low application rates, the fungicidal activity of known phytofungicidal compounds is unsatisfactory.

The inventors have surprisingly found that the novel heteroaryl compounds of formula (I) have improved antifungal activity. The compounds are particularly effective as agrochemical fungicides and effective against a broad spectrum of phytopathogenic fungi.

### DETAILED DESCRIPTION OF THE INVENTION

Accordingly, the present invention relates to the use of a compound of formula (I) 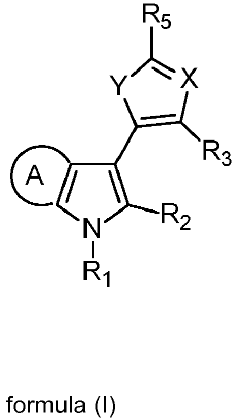 wherein,
X denotes CH or N;
Y denotes NH, O or S;
A, together with two carbon atoms of the pyrrole ring, is selected from the group consisting of; 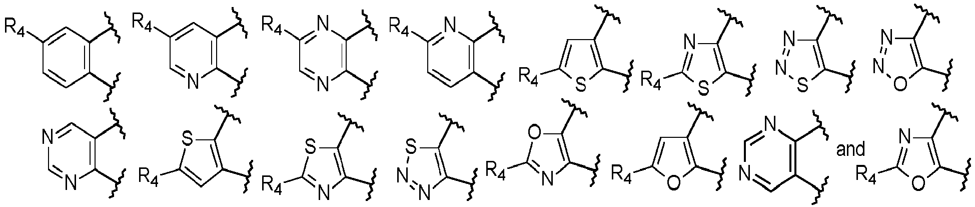
R¹ is selected from the group consisting of H, -(CH₂)ₙ-OR⁶, -(CH₂)ₙ-C(=O)-R⁷, -C(=O)-(CH₂)ₙ-R⁸, -C(=O)-(CH₂)ₙ-OR⁹, -C(=O)-CR¹⁰(=CR¹¹), -C(=O)-(CH₂)ₙ-SR¹², -(CH₂)ₙ-O-C(=O)-R¹³, -(CH₂)ₙ-C(=O)-OR¹⁴, -C(=O)-O-(CH₂)ₙ-R¹⁵, -O-C(=O)-O-(CH₂)ₙ-R¹⁶, -C(=O)-NR¹⁷R¹⁸, -C(=S)-NR¹⁷R¹⁸, -N R¹⁷R¹⁸, -S(=O)ₙ-R¹⁹, -(CH₂)n-Si-(R²⁰)ₙ and -(CH₂)ₙ-O-(CH₂)ₙ-Si-(R²¹)ₙ; whereby R¹ is unsubstituted or further substituted by 1, 2 or 3 identical or different groups R^{12a} which are independently selected from the group consisting of F, Cl, Br, I, OH, SH, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, C(=O)-(OH), - C(=O)-(NR¹⁷R¹⁸), -C(=O)-(H), -O-C(=O)-(C₁-C₈-alkyl), -C(=O)-(C₁-C₈-alkyl) and -C(=O)-O-(C₁-C₈-alkyl);
R² is selected from the group consisting of H; halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R² is unsubstituted or further substituted by 1, 2 or 3 identical or different groups R^{12b} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, - S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, -Si-(C₁-C₈-alkyl)₃, -Si-(C₁-C₈-alkyl)₂-phenyl, -Si-(phenyl)₃, phenyl and -CH₂-phenyl;
R³ is selected from the group consisting of F, Cl, Br, I, SH, CN, NO₂, NR¹⁷R¹⁸, -O-C₁-C₈-alkyl,-O-C₂-C₈-alkenyl, -O-C₂-C₈-alkynyl, C₁-C₈-alkyl, C₂-C₈-alkenyl, C₂-C₈-alkynyl, C(=O)-(OH), C(=O)-(NR¹⁷R¹⁸), C(=O)-(H), O-C(=O)-(C₁-C₈-alkyl), -C(=O)-O-(C₁-C₈-alkyl), -O-C(=O)-(C₂-C₈-alkenyl), -C(=O)-O-(C₂-C₈-alkenyl), -O-C(=O)-(C₅-C₆-aryl), -C(=O)-O-C₅-C₆-aryl, C₃-C₈-cycloalkyl, O-C₃-C₈-cycloalkyl, -C(=O)-(C₁-C₈-alkyl), -C(=O)-(C₅-C₆-aryl), C₅-C₆-aryl, O-C₅-C₆-aryl and C₃-C₈-cycloalkenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(NR⁷R⁸), --C(=O)-(H), -O-C(=O)-(C₁-C₈-alkyl), -C(=O)-(C₁-C₈-alkyl) and -C(=O)-(O)(C₁-C₈-alkyl);
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ and R²¹ are independently of each other, selected from the group consisting of H, F, Cl, Br, I, CN, C₁-C₈-alkyl, C₂-C₈-alkenyl, C₂-C₈-alkynyl, C₃-C₈-cycloalkyl, C₅-C₆-aryl, C₅-C₆-heterocycloalkenyl, C₅-C₆-heterocycloalkyl or C₅-C₆-heteroaryl; wherein heterocycloalkenyl, heterocycloalkyl or heteroaryl contain besides carbon atoms as ring members, 1, 2 or 3 heteroatoms independently selected from O, N or S as ring members; or R¹⁷ together with R¹⁸ form a C₅-C₆-heterocycloalkenyl, C₅-C₆-heterocycloalkyl or C₅-C₆-heteroaryl;
R⁴ is selected from the group consisting of F, Br, I, CN, -C(=O)-OR¹⁴, -O-C₁-C₈-alkyl, CH₂F, C₂-C₈-alkyl, C₂-C₈-alkenyl, C₂-C₈-alkynyl, C₃-C₈-cycloalkyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁-C₆-alkyl) and C₃-C₈-cycloalkenyl whereby R⁴ is unsubstituted or further substituted by 1, 2 or 3 identical or different groups R^{12d} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl;
R⁵ is selected from the group consisting of H; halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R⁵ is unsubstituted or further substituted by 1, 2 or 3 identical or different groups R^{12e} which are independently selected from the group consisting of CI, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, - S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, -Si-(C₁-C₈alkyl)₃, -Si-(C₁-C₈-alkyl)₂-phenyl, -Si-(phenyl)₃, phenyl and -CH₂-phenyl; and
n is selected from 0, 1, 2, 3, 4 and 5;
or in the form of a stereoisomer or an agriculturally acceptable salt or a tautomer or an isotopic form of a N-oxide or a S-oxide or a prodrug thereof, as phytofungicide.

In one aspect, the present invention relates to the method of protecting a crop of useful plants susceptible to and/or under attack by fungi, which method comprises applying to said crop, treating a plant propagation material of said crop with, and/or applying to said fungi, the composition comprising at least one compound of formula (I) or in the form of a stereoisomer or an agriculturally acceptable salt or a tautomer or an isotopic form of a N-oxide or a S-oxide or a prodrug thereof.

In another aspect, the present invention relates to the use of a compound of formula (I), which comprises treating the fungi, the plant, or the plant propagation material selected from the group consisting of seeds, roots, fruits, tubers, bulbs, rhizomes, shoots, sprouts and other parts of plants, including seedlings and young plants to be protected against fungi attack, the stored materials or harvest, or alternately, the locus or soil or soil substituents or surfaces therefrom, with an effective amount of at least one compound of formula (I) or in the form of a stereoisomer or an agriculturally acceptable salt or a tautomer or an isotopic form of a N-oxide or a S-oxide or a prodrug thereof.

In a further aspect, the present invention relates to a composition, comprising at least one compound of formula (I) as defined herein above or in the form of a stereoisomer or an agriculturally acceptable salt or a tautomer or an isotopic form of a N-oxide or a S-oxide or a prodrug thereof, and an auxiliary.

In a further aspect, the present invention relates to an agrochemical mixture comprising at least one fertilizer; and at least one compound of formula (I) as defined herein above; or in the form of a stereoisomer or an agriculturally acceptable salt or a tautomer or an isotopic form or a N-oxide or a S-oxide or a prodrug thereof, and at least one pesticidally active substance selected from the group consisting of herbicides, safeners, fungicides, insecticides and plant growth regulators.

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

It will be understood that "substitution", "substituted" or "substituted with" means that one or more hydrogens of the specified moiety are replaced with a suitable substituent and includes the implicit proviso that such substitutions are in accordance with permitted valence of the substituted atom and the substituent and results in a stable compound.

When any variable (for instance, R¹, R², R³, R⁴, R⁵ etc.) or substituent has more than one occurrence, its definition on each occurrence is independent at every other occurrence. Also, combinations of substituents and/or variables are permissible only if such combinations result in stable compounds.

The term "independently" when used in the context of selection of substituents for a variable, it means that where more than one substituent is selected from a number of possible substituents, those substituents may be the same or different.

Within the context of the present invention, the term "alkyl", as used herein, alone or as part of a substituent group refers to an acylic saturated aliphatic groups, including straight-chain or branched alkyl residues. Furthermore, the alkyl residue can be unsubstituted or substituted with one or more substituents, as in the case of C₁-C₈ alkyl, 1 to 8 carbon atoms. When one or more substituents denote an alkyl residue or comprise an alkyl residue which is mono or polysubstituted, this may be preferably be substituted with 1, 2 or 3 substituents independently selected from the group consisting of F, Cl, Br, I, -NO₂, -CN, -OH, -SH, -NH₂, -NH(C₁-C₆)alkyl, -NH-CH(CH₃)₂, -N(C₁-C₆-alkyl)₂, -N(C₁-C₆-alkyl)-phenyl, -C(=O)-H, -C(=O)-C₁-C₆-alkyl, -C(=O)-OH,-C(=O)-O-C₁-C₆-alkyl, -C(=O)-O-phenyl, -CH₂-C(=O)-OH, -CH₂-C(=O)-C₁-C₆-alkyl, -CH₂-C(=O)-O-C₁-C₆-alkyl, -C(=O)-NH₂, -C(=O)-NH(C₁-C₆)alkyl, -C(=O)-NH-phenyl, -NH-C(=O)C(CH₃)₃,-S(=O)-C₁-C₆-alkyl, -S(=O)-phenyl, -S(=O)₂-C₁-C₆-alkyl, -S(=O)₂-phenyl, -S(=O)₂-NH₂, -NH-SO₂-phenyl, -N(CH₃)-SO₂-phenyl, -C₁-C₆-alkyl, -Si-(CH₃)₃, -Si(C₂H₅)₃, -Si-(CH₃)₂-C₆H₅, -Si-(C₆H₅)₃, Si(C₂H₅)₃, -Si-(CH₃)₂-C₆H₅, -Si-(C₆H₅)₃, ethenyl, propenyl, isopropenyl, isobutenyl, isopentenyl, ethynyl, propynyl, butynyl, cyclopropyl, cyclobutyl, cyclopentyl, cyclohexyl, furyl, oxazolyl, thiazolyl, pyrazolyl, thiophenyl, oxadiazolyl, thiadiazolyl, pyridinyl, pyrimidinyl, piperazinyl, pyrazinyl, phenyl and -CH₂-phenyl;, NH-C(=O)C(CH₃)₃, -Si-(CH₃)₃, -Si(C₂H₅)₃, -Si-(CH₃)₂-C₆H₅, -Si-(C₆H₅)₃, phenyl and -CH₂-phenyl.

Representative examples of alkyl which may be unsubstituted or mono- or polysubstituted include, but are not limited to, methyl, ethyl, n-propyl, n-butyl, n-pentyl, n-hexyl, isopropyl, isobutyl, tertiary butyl, isopentyl, 2-methylbutyl, 3-methylbutyl and the like. Polysubstituted alkyl residues are understood to be those alkyl residues which are polysubstituted, preferably di- or trisubstituted, either on different or on the same carbon atoms, for example trisubstituted on the same C atom as in the case of -CF₃ or at different locations as the case of -(CHCl)-(CH₂)F. Polysubstitution may proceed with identical or different substituents. Representative examples of suitable substituted alkyl residues which may include, but are not limited to, -CF₃, -CF₂H,-CFH₂, -CH₂Cl, -(CH₂)-OH, -(CH₂)-(CF₃), -(CH₂)-(CH₂)-NH₂, -(CH₂)-C(=O)-OH, -CH(CH₃)₂-OH-CH(CF₃)-OH, -(CH₂)-(CH₂)-C(=O)-OH, -(CH₂)-C(=O)-OCH₃, -(CH₂)-N(CH₃)₂, -(CF₂)-(CF₃),-(CH₂)-(CH₂)-(CH₂)-(CH₂)-Cl and -(CH₂)-(CH₂)-NH-C(=O)-O-C(CH₃)₃.

Within the context of the present invention, the term "alkenyl", as used herein, refers to acyclic unsaturated hydrocarbon residues, including straight-chain or branched alkenyl residues, and comprise at least one double bond, preferably 1, 2, or 3 double bonds, with as in the case of C₂-C₈ alkenyl, 2 to 8 carbon atoms. Furthermore, the alkenyl residue can be unsubstituted or substituted with one or more substituents, as in the case of C₂-C₆-alkenyl, 2 to 6 carbon atoms. When one or more substituents denote an alkenyl residue or comprise an alkenyl residue which is mono or polysubstituted, this may be optionally be substituted with 1, 2 or 3 substituents independently selected from the group consisting of F, Cl, Br, I, -NO₂, -CN, -CCl₃, -CF₃, -OH, -SH, -NH₂, -NH(C₁-C₆)alkyl,-NH-CH(CH₃)₂, -N(C₁-C₆-alkyl)₂, -N(C₁-C₆-alkyl)-phenyl, -C(=O)-H, - C(=O)-C₁-C₆-alkyl, -C(=O)-OH, -C(=O)-O-C₁-C₆-alkyl, -C(=O)-O-phenyl, -CH₂-C(=O)-OH, -CH₂-C(=O)-C₁-C₆-alkyl, -CH₂-C(=O)-O-C₁-C₆-alkyl, -C(=O)-NH₂, -C(=O)-NH(C₁-C₆)alkyl, -C(=O)-NH-phenyl, -NH-C(=O)C(CH₃)₃, -S(=O)-C₁-C₆-alkyl, -S(=O)-phenyl, -S(=O)₂-C₁-C₆-alkyl, -S(=O)₂-phenyl, -S(=O)₂-NH₂, -NH-SO₂-phenyl, -N(CH₃)-SO₂-phenyl; -C₁-C₆-alkyl, -Si-(CH₃)₃, -Si(C₂H₅)₃, -Si-(CH₃)₂-C₆H₅, -Si-(C₆H₅)₃, -O(CH₂)₂-Si(CH₃)₃, methyl, ethyl, propyl, butyl, isopropyl, isobutyl, tertiary butyl, pentyl, hexyl, heptyl, ethenyl, propenyl, isopropenyl, isobutenyl, isopentenyl, ethynyl, propynyl, butynyl, cyclopropyl, cyclobutyl, cyclopentyl, cyclohexyl, furyl, oxazolyl, thiazolyl, pyrazolyl, thiophenyl, oxadiazolyl, thiadiazolyl, pyridinyl, pyrimidinyl, piperazinyl, pyrazinyl, phenyl and -CH₂-phenyl.

Representative examples for alkenyl which may be unsubstituted or mono- or polysubstituted include, but are not limited to, ethenyl, 1-propenyl, 1-butenyl, 2-butenyl, 3-butenyl, 1-pentenyl, 4-pentenyl, hexenyl, -CH=C(CH₃)₂ and the like. Polysubstituted alkyl residues are understood to be those alkenyl residues which are polysubstituted, preferably disubstituted, either on different or on the same carbon atoms, for example disubstituted on the same C atom as in the case of - CH=CCl₂, or at different locations as in the case of -CCl=CH-(CH₂)-NH₂. Polysubstitution may proceed with identical or different substituents.

Within the context of the present invention, the term "alkynyl", as used herein, refers to acyclic unsaturated hydrocarbon residues, including straight-chain or branched alkenyl residues, and comprise at least one triple bond, preferably 1 or 2 triple bonds, with as in the case of C₂-C₈ alkynyl, 2 to 8 carbon atoms. Furthermore, the alkynyl residue can be unsubstituted or substituted with one or more substituents, as in the case of C₂-C₆-alkynyl, 2 to 6 carbon atoms. When one or more substituents denote an alkenyl residue or comprise an alkenyl residue which is mono or polysubstituted, this may be optionally be substituted with 1, 2 or 3 substituents independently selected from the group consisting of F, Cl, Br, I, -NO₂, -CN, -OH, -SH, -NH₂, -NH(C₁-C₆)alkyl,-NH-CH(CH₃)₂, -N(C₁-C₆-alkyl)₂, -N(C₁-C₆-alkyl)-phenyl, -C(=O)-H, -C(=O)-C₁-C₆-alkyl,-C(=O)-OH, -C(=O)-O-C₁-C₆-alkyl, -C(=O)-O-phenyl, -CH₂-C(=O)-OH, -CH₂-C(=O)-C₁-C₆-alkyl,-CH₂-C(=O)-O-C₁-C₆-alkyl, -C(=O)-NH₂, -C(=O)-NH(C₁-C₆)alkyl, -C(=O)-NH-phenyl, -NH-C(=O)C(CH₃)₃, -S(=O)-C₁-C₆-alkyl, -S(=O)-phenyl, -S(=O)₂-C₁-C₆-alkyl, -S(=O)₂-phenyl, -S(=O)₂-NH₂, -NH-SO₂-phenyl, -N(CH₃)-SO₂-phenyl, -C₁-C₆-alkyl, -Si-(CH₃)₃, -Si(C₂H₅)₃, -Si-(CH₃)₂-C₆H₅, -Si-(C₆H₅)₃, methyl, ethyl, propyl, butyl, isopropyl, isobutyl, tertiary butyl, pentyl, hexyl, heptyl, cyclopropyl, cyclobutyl, cyclopentyl, cyclohexyl, furyl, oxazolyl, thiazolyl, pyrazolyl, thiophenyl, oxadiazolyl, thiadiazolyl, pyridinyl, pyrimidinyl, piperazinyl, pyrazinyl, phenyl and -CH₂-phenyl.

Representative examples for alkynyl which may be unsubstituted or mono- or polysubstituted include, but are not limited to, ethynyl, 1-propynyl, 1-butynyl, 2-butynyl, 3-butynyl, 1-pentynyl, 2-pentynyl, 3-pentynyl, 4-pentynyl, hexynyl and the like. Polysubstituted alkynyl residues are understood to be those alkynyl residues which are polysubstituted, preferably disubstituted, either on different or on the same carbon atoms. Representative examples of alkynyl residues include, but are not limited to, -C≡C-Si(CH₃)₃, -C≡C-Si(CH₂)₂-CH₃ and -C≡C-Si(CH₂)₂-C₆H₅.

Within the context of the present invention and as used herein, the term "cycloalkyl" refers to a saturated cyclic hydrocarbon residue including preferably 3, 4, 5, 6, 7 or 8 atoms, as ring members; and more preferably refers to a cycloalkyl with 3, 4, 5, 6 or 7 carbon atoms. The cycloalkyl group may be unsubstituted or monosubstituted or identically or differently polysubstituted. Representative examples of cycloalkyl include, but are not limited to, cyclopropyl, cyclobutyl, cyclopentyl, cyclohexyl and cycloheptyl.

Within the context of the present invention and as used herein, the term "cycloalkenyl"refers to a cyclic unsaturated hydrocarbon residue including preferably 3, 4, 5, 6, 7 or 8 carbon atoms, as ring members; and more preferably refers to a cycloalkenyl with 3, 4, 5, 6 or 7 carbon atoms. The cycloalkenyl group may be unsubstituted or monosubstituted or identically or differently polysubstituted. Representative examples of cycloalkenyl include, but are not limited to, cyclopropenyl, cyclobutenyl, cyclopentenyl, cyclohexenyl, cyclohexadienyl, cycloheptenyl and cycloheptadienyl.

Within the context of the present invention and as used herein, the term "heterocycloalkyl" refers to a cyclic saturated hydrocarbon residue with preferably 5 or 6 carbon atoms, wherein one or more C atoms are replaced heteroatoms independently selected from oxygen, nitrogen and sulfur. Heterocycloalkyl residues may preferably comprise 1, 2, or 3 heteroatom(s) mutually independently selected from the group consisting of oxygen, sulfur and nitrogen as ring members. A heterocycloalkyl residue may be unsubstituted or monosubstituted or identically or differently polysubstituted.

Representative examples of heterocycloalkyls include, but are not limited to, tetrahydrofuranyl, tetrahydrothiophenyl, tetrahydropyranyl, piperidinyl, piperazinyl, morpholinyl, thiomorpholinyl, 1,2,4-oxadiazonyl, 1,2,4-thiodiazonyl, 1,3,4-trimethyl piperazinyl, 2-piperidinyl, 3-piperidinyl, 4-piperidinyl, 1,3-dioxan-5-yl, tetrahydropyrimidinyl, tetrahydropyrazinyl and tetrahydropyridazinyl.

Within the context of the present invention and as used herein, the term "heterocycloalkenyl" refers to a cyclic unsaturated hydrocarbon residue with preferably 5 or 6 carbon atoms, which comprises at least one double bond, and wherein, one or more C atoms are replaced by heteroatoms independently selected from oxygen, nitrogen and sulfur. Heterocycloalkenyl residues may preferably comprise 1, 2, or 3 heteroatom(s) mutually independently selected from the group consisting of oxygen, sulfur and nitrogen as ring members. A heterocycloalkenyl residue may be unsubstituted or monosubstituted or identically or differently polysubstituted.

Representative examples of heterocycloalkenyls include, but are not limited to, (2,3)-dihydrofuranyl, (2,3)-dihydrothienyl, (2,3)-dihydropyrrolyl, (2,5)-dihydropyrrolyl, (2,5)-dihydropyrrolyl, (2,3)-dihydroisoxazolyl, (1,4)-dihydropyridin-1-yl, dihydropyranyl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 4,5-dihydropyrazol-2-yl, 4,5-dihydropyrazol-3-yl, 4,5-dihydropyrazol-4-yl, 4,5-dihydropyrazol-5-yl, 2,5-dihydrothienyl and (1,2,3,4)-tetrahydropyridin-1-yl.

The cycloalkyl, heterocycloalkyl, cycloalkenyl or heterocycloalkenyl residues may be fused with an unsubstituted or at least monosubstituted mono- or bicyclic ring system. Within the context of the present invention, a mono- or bicyclic ring system should be understood to mean mono- or bicyclic hydrocarbon residues which may be saturated, unsaturated or aromatic and optionally comprise one or more heteroatoms as ring members.

When one or more substituents comprise a monocyclic or bicyclic ring system, which is mono- or polysubstituted, this may be optionally substituted with 1, 2, or 3 substituents, which may be independently selected from the group consisting of F, CI , Br, I, -CN, -NH(C₁-C₆)alkyl, -NH-CH(CH₃)₂, -C(=O)-H, -C(=O)-C₁-C₆-alkyl, -C(=O)-OH, -C(=O)-O-C₁-C₆-alkyl, -C(=O)-O-phenyl,-C(=O)-NH₂, -C(=O)-NH(C₁-C₆)alkyl, -NH-C(=O)C(CH₃)₃, -S(=O)₂-C₁-C₆-alkyl, -S(=O)₂-phenyl,-S(=O)₂-NH₂, methyl, ethyl, n-propyl, isopropyl, n-butyl, isobutyl, tertiary butyl, ethenyl, ethynyl, propynyl, -CF₃, oxo (=O), cyclopropyl, cyclobutyl, cyclopentyl, cyclohexyl, furyl, oxazolyl, thiazolyl, pyrazolyl, thiophenyl, oxadiazolyl, thiadiazolyl, pyridinyl, pyrimidinyl, piperazinyl, pyrazinyl, phenyl and -CH₂-phenyl.

Within the context of the present invention and as used herein, the term "aryl", refers to a monocyclic hydrocarbon ring system having 5 or 6 ring carbon atoms, wherein at least one carbocyclic ring is having a π-electron system. An aryl residue may be unsubstituted, monosubstituted or identically or differently polysubstituted. Examples of aryl residues include phenyl.

Within the context of the present invention and as used herein, the term "heteroaryl" refers to an aromatic hydrocarbon containing 5 or 6 carbon atoms, in which one to four carbon atoms are replaced by identical or different hetero atoms selected from the group consisting of oxygen, sulfur and nitrogen. Heteroaryl residues may preferably comprise 1, 2, 3 or 4 particularly preferably 1, 2, or 3, heteroatoms independently selected from the group consisting of oxygen, sulfur and nitrogen. A heteroaryl residue may be unsubstituted or monosubstituted or identically or differently polysubstituted.

Representative examples of suitable heteroaryl residues include, but are not limited to, furyl, pyridyl, oxazolyl, thiazolyl, pyrazolyl, pyrimidinyl, pyrrolyl, isooxazolyl, triazolyl, tetrazolyl, pyridazinyl, isothiazolyl, benzothiazolyl, benzooxazolyl, benzimidazolyl, quinolinyl or isoquinolinyl.

Unless stated otherwise, when one or more substituents denote an aryl or heteroaryl residue or comprise an aryl or heteroaryl residue, which are mono substituted or polysubstituted, this may be optionally substituted with 1, 2 or 3 substituents independently selected from the group consisting of F, Cl, Br, I, -NO₂, -CN, -OH, -SH, -O-CH₃, -O-C₂H -NH₂, -NH(C₁-C₆)alkyl, -NH-CH(CH₃)₂, -N(C₁-C₆-alkyl)₂, -N(C₁-C₆-alkyl)-phenyl, -C(=O)-H, -C(=O)-C₁-C₆-alkyl, -C(=O)-OH,-C(=O)-O-C₁-C₆-alkyl, -C(=O)-O-phenyl, -CH₂-C(=O)-OH, -CH₂-C(=O)-C₁-C₆-alkyl, -C(=O)-NH₂,-C(=O)-NH(C₁-C₆)alkyl, -C(=O)-NH-phenyl, -S(=O)-C₁-C₆-alkyl, -S(=O)-phenyl, -S(=O)₂-C₁-C₆-alkyl, -S(=O)₂-phenyl, -S(=O)₂-NH₂, -NH-SO₂-phenyl, -N(CH₃)-SO₂-phenyl, -C₁-C₆-alkyl, -CF₃, cyclopropyl, cyclobutyl, cyclopentyl, cyclohexyl, furyl, oxazolyl, thiazolyl, pyrazolyl, thiophenyl, oxadiazolyl, thiadiazolyl, pyridinyl, pyrimidinyl, piperazinyl, pyrazinyl, phenyl, -CH₂-phenyl, -O-phenyl, -O-CH₂-phenyl and CH₂-pyridinyl.

The term "heteroatom" as used herein, includes nitrogen (N), oxygen (O) and sulfur (S). Any heteroatom with unsatisfied valency is assumed to have a hydrogen atom or a C₁-C₆-alkyl group to satisfy the valency.

Within the context of the present invention and as used herein, the "compounds of the present invention" include all the stereoisomeric and tautomeric forms and mixtures thereof in all ratios, prodrugs, isotopic forms, their agriculturally acceptable salts, N-oxides and S-oxides thereof.

Within the context of the present invention and as used herein, the term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers).

Within the context of the present invention and as used herein, the term "tautomer" refers to the coexistence of two (or more) compounds that differ from each other only in the position of one (or more) mobile atoms and in electron distribution, for example, keto-enol tautomers.

The term "agriculturally acceptable salts" as used herein, includes salts of the active compounds which are prepared with acids or bases, depending on the substituents found on the compounds described herein.

Within the context of this present application and as used herein, the term "isotopic forms" or "isotopically labeled forms" is a general term used for isotopic forms of compounds of formula, wherein one or more atoms of compounds of formula (I); I(a) are replaced by their respective isotopes. All isotopes of any particular atom or element as specified are contemplated within the scope of the compounds of the invention. Examples of isotopes that may be incorporated into the compounds disclosed herein include, but are not limited to, isotopes of hydrogen such as ²H (deuterium or D) and ³H, carbon such as ¹¹C, ¹³C and ¹⁴C, nitrogen such as ¹³N and ¹⁵N, oxygen such as ¹⁵O, ¹⁷O and ¹⁸O, chlorine such as ³⁶Cl, fluorine such as ¹⁸F and sulphur such as ³⁵S.

Within the context of the present invention and as used herein, "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

Within the context of the present invention and as used herein, "S-oxide" refers to the oxide of the sulfur atom (S-oxide) or dioxide of the sulfur atom (S, S-dioxide) of a sulfur-containing heteroaryl or heterocycle. S-oxide and S, S-dioxides can be in the presence of an oxidizing agent for example peroxide such as m-chloro-perbenzoic acid or oxone.

In one aspect, the present invention relates to the use of the compound of formula (I), wherein R¹ is selected from the group consisting of H, -CH₂-OH, -CH₂-OCH₃, -CH₂-O-CH(CH₃)₂, -CH₂-O-(CH₂)₂-Si(CH₃)₃, -C(=O)-CH₃, -C(=O)-CH₂-CH₃, -C(=O)-(CH₂)₂-CH₃, -C(=O)-(CH₂)₃-CH₃, -C(=O)-(CH₂)₄-CH₃, -C(=O)-(CH₂)₅-CH₃, -C(=O)-C(CH₃)₃, -C(=O)-CH₂-OCH₃; -C(=O)-CH=CH-CH₃;-C(=O)-phenyl; -C(=O)-cyclopropyl; -C(=O)-CH₂-cyclopropyl; -C(=O)-CH₂-phenyl; -CH₂-O-C(=O)-CH₃; -CH₂-O-C(=O)-CH₂-CH₃; -CH₂-O-C(=O)-(CH₂)₃-CH₃; -CH₂-O-C(=O)-(CH₂)₄-CH₃; -CH₂-O-C(=O)-(CH₂)₅-CH₃; -CH₂-O-C(=O)-C(CH₃)₃; -CH₂-O-C(=O)-phenyl; -C(=O)-OCH₃; C(=O)-O-(CH₂)₂CH_{3;} -C(=O)-O-(CH₂)₃-CH₃; -C(=O)-O-(CH₂)₄-CH₃; -C(=O)-O-(CH₂)₅-CH₃; -C(=O)-OCH₂-CH(CH₃)₂; -C(=O)-OCH(CH₃)₂; -C(=O)-O-CH₂-CCl₃; -C(=O)-OC(CH₃)₃; -C(=O)-O-(CH₂)₃-CH₃;-C(=O)-O-CH₂-CH=CH; -C(=O)-O-CH₂-C=CH; -C(=O)O-phenyl; -C(=O)-O-CH₂-phenyl; -CH₂-C(=O)-OCH₃; -CH₂-C(=O)-OCH₂-CH₃; -CH₂-C(=O)-O(CH₂)₂-CH₃; -CH₂-C(=O)-O(CH₂)₃-CH₃;-CH₂-C(=O)-O(CH₂)₄-CH₃; -CH₂-C(=O)-O(CH₂)₅-CH₃; -CH₂-C(=O)-OC(CH₃)₃; -CH₂-C(=O)-O-CH₂-CH=CH; -CH₂-C(=O)-O-CH=CH-CH₃; -C(=O)-NH₂; -C(=O)-NH-CH₃; -C(=O)-N-(CH₃)₂;-C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)2-CH₃; -S(=O)₂-CH₂-CH₃; -S(=O)2-(CH₂)₂-CH₃; -S(=O)2-(CH₂)₂-CH₃;-S(=O)₂-tert-butyl and -S(=O)₂-phenyl; whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, -C(=O)-(OH), -C(=O)-(H), -O-C(=O)-(C₁-C₈-alkyl), -C(=O)-(C₁-C₈-alkyl) and -C(=O)-(O)(C₁-C₈-alkyl).

In preferred embodiment, R¹ is selected from the group consisting of H, -CH₂-OH, -CH₂-OCH₃,--C(=O)-phenyl; -C(=O)-cyclopropyl; -C(=O)-OCH₃; -C(=O)O-phenyl; -C(=O)-NH₂; -C(=O)-NH-CH₃; -C(=O)-N-(CH₃)₂; -C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)₂-CH₃; -S(=O)₂-CH₂-CH₃; -S(=O)₂-(CH₂)₂-CH₃; -S(=O)2-(CH₂)₂-CH₃; -S(=O)₂-tert-butyl and -S(=O)₂-phenyl, whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl and -C(=O)-(H).

In more preferred embodiment, R¹ is H.

In another aspect, the present invention relates to the use of the compound of formula (I), wherein R² is selected from the group consisting of H, Cl, Br, F, I, CN, -C(=O)-O-CH₃, -C(=O)-O-CH₂-CH₃, -C(=O)-O-(CH₂)₃-CH₃ -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl, ethynyl, propynyl; whereby R² is unsubstituted or further substituted by 1, 2 or 3 identical or different groups R^{12b} which are independently selected from the group consisting of CI, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)₂-phenyl, -N(CH₃)-S(=O)2-phenyl, phenyl and -CH₂-phenyl.

In preferred embodiment, R² is selected from the group consisting of H, Cl, F, CN, -C(=O)-O-CH₃, methyl, ethyl, propyl, cyclopropyl, cyclobutyl, cyclopentyl, cyclohexyl, cyclopropenyl, cyclobutenyl, cyclopentenyl and cyclohexenyl, ethynyl and propynyl, whereby R² is unsubstituted or further substituted by 1, 2 or 3 identical or different groups R^{12b} which are independently selected from the group consisting of Cl, Br, F, I, CN, -NH₂ and -C(=O)-H.

In more preferred embodiment, R² is selected from the group consisting of H, F and Cl.

In another aspect, the present invention relates to the use of the compound of formula (I), wherein R³ is selected from the group consisting of Cl, F, Br, I, CN, -O-CH₃, -O-CH₂-CH₃,-C(=O)-O-CH₃; -C(=O)-O-CH₂-CH₃; -C(=O)-O-(CH₂)₂-CH₃; -C(=O)-O-(CH₂)₃-CH₃; -C(=O)-O-(CH₂)₄-CH₃; -C(=O)-O-(CH₂)5-CH₃; -C(=O)-O-CH(CH₃)₂; -C(=O)-O-C(CH₃)₃; methyl, ethyl, propyl, butyl, isopropyl, isobutyl, tert butyl, ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(H), -C(=O)-(C₁-C₈-alkyl) and -C(=O)-(O)(C₁-C₈-alkyl).

In preferred embodiment, R³ is selected from the group consisting of CI, F, Br, I, CN, -O-CH₃, C₁-C₈-alkyl, -C(=O)-(H), ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl, whereby R³ is unsubstituted or further substituted by 1, 2 or 3 identical or different groups R^{12c} which are independently selected from the group consisting of F, Cl, Br and I.

In more preferred embodiment, R³ is selected from the group consisting of CI, F, Br, I, CN, -O-CH₃, C₁-C₈-alkyl, -C(=O)-(H), whereby R³ is unsubstituted or further substituted by 1, 2 or 3 identical or different groups R^{12c} which are independently selected from the group consisting of F, Cl, Br and I.

In yet another aspect, the present invention relates to the use of the compound of formula (I), wherein R⁴ is selected from the group consisting of F, Br, I, CN, -C(=O)-OR¹⁴, -O-C₁-C₈-alkyl, CH₂F, C₂-C₈-alkenyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl), S(O₂)(C₁-C₆-alkyl), C3-C8-cycloalkenyl and C2-C8-alkynyl, whereby R⁴ is unsubstituted or further substituted by 1, 2 or 3 identical or different groups R^{12d} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl.

In preferred embodiment, R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I.

In more preferred embodiment, R⁴ is selected from the group consisting of F, CN and -C=CH.

In yet another aspect, the present invention relates to the use of the compound of formula (I), wherein R⁵ is selected from the group consisting of H, Cl, Br, F, I, CN, -C(=O)-O-CH₃, -C(=O)-O-CH₂-CH₃, -C(=O)-O-(CH₂)₃-CH₃ -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl; whereby R⁵ is unsubstituted or further substituted by 1, 2 or 3 identical or different groups R^{12e} which are independently selected from the group consisting of Cl, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, - C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl.

In preferred embodiment, R⁵ is selected from the group consisting of H, Cl, Br, F, I, CN, -C(=O)-O-CH₃, methyl, ethyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl, whereby R⁵ is unsubstituted or further substituted by 1, 2 or 3 identical or different groups R^{12e} which are independently selected from the group consisting of Cl, Br, F, I, CN, -NH₂, -C(=O)-H.

In more preferred embodiment, R⁵ is selected from the group consisting of H, Cl and F.

In preferred embodiment, the present invention provides the use of the compound of formula (I), wherein,
X denotes CH or N; Y denotes O or S;
A, together with two carbon atoms of the pyrrole ring, is selected from the group consisting of 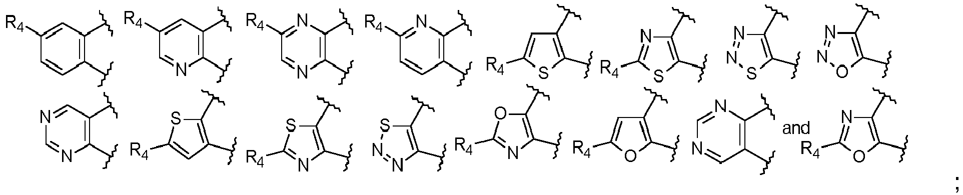 R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are independently selected from the group consisting of F, Cl, Br and I; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I; and
R⁵ is selected from the group consisting of H, F and Cl.

In more preferred embodiment, the present invention provides the use of the compound of formula (I), wherein,
X denotes N and Y denotes O;
A, together with two carbon atoms of the pyrrole ring, is selected from the group consisting of; 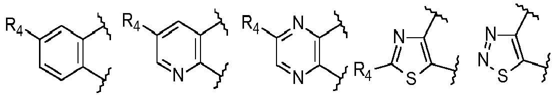 R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and R⁵ is selected from the group consisting of H, F and Cl.

In preferred embodiment, the present invention provides the use of the compound of formula (I), wherein the compound of formula (I) is a compound of formula (IA), formula (IB), formula (IC) or formula (ID) 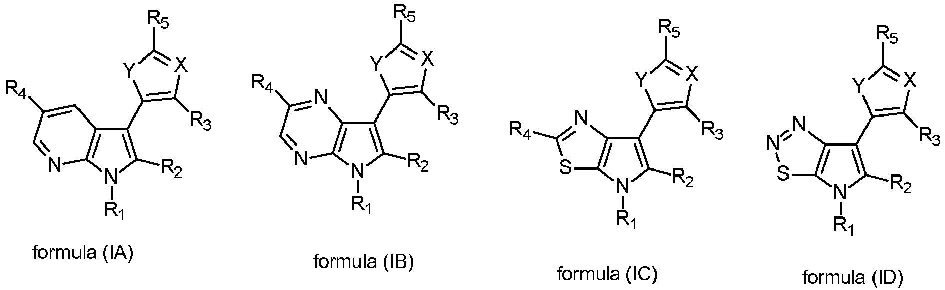 wherein;
X denotes CH or N; Y denotes NH, O or S;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and R⁵ is selected from the group consisting of H, F and Cl.

In more preferred embodiment, the present invention provides the use of the compound of formula (I), wherein the compound of formula (I) is a compound of formula (IA) or formula (IB), 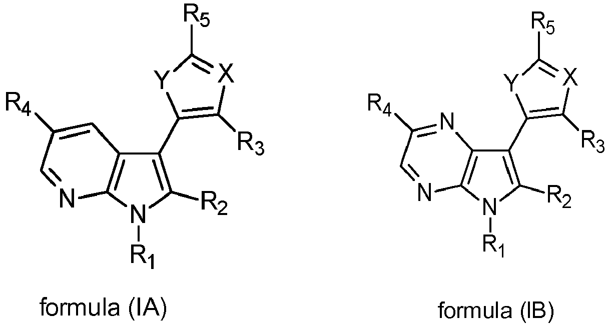 wherein;
X denotes N; Y denotes O;
R¹ is H; R² is selected from the group consisting of H, F and Cl;
R³ is selected from the group consisting of Cl, CF₃, CHF₂ and CH₂F; and
R⁴ is selected from the group consisting of F, CN and -C=CH; and
R⁵ is selected from the group consisting of H, F and Cl.

In preferred embodiment, the present invention provides the use of the compound of formula (I), wherein the compound of formula (I) is the compound of formula (IE) 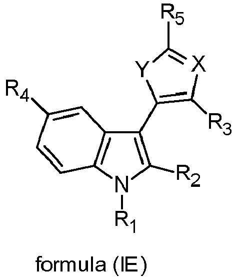 wherein;
X denotes CH or N; Y denotes O or S;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and R⁵ is selected from the group consisting of H, F and Cl.

In one aspect, the present invention provides a compound of the formula (I') 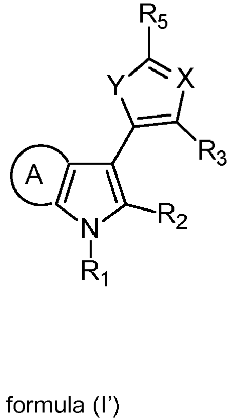 wherein,
X denotes CH or N;
Y denotes NH, O or S;
A, together with two carbon atoms of the pyrrole ring, is selected from the group consisting of; 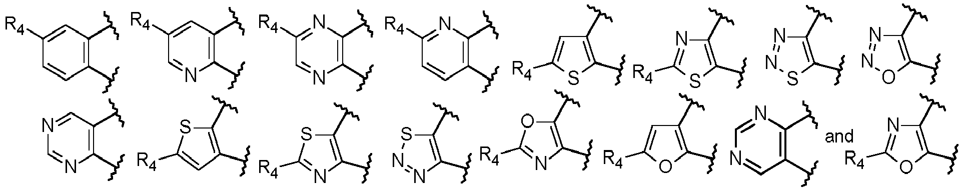
R¹ is selected from the group consisting of H, -(CH₂)ₙOR⁶, -(CH₂)ₙ-C(=O)-R⁷, -C(=O)-(CH₂)ₙ-R⁸, -C(=O)-(CH₂)ₙ-OR⁹, -C(=O)-CR¹⁰(=CR¹¹), -C(=O)-(CH₂)ₙ-SR¹², -(CH₂)ₙ-O-C(=O)-R¹³, -(CH₂)ₙ-C(=O)-OR¹⁴, -C(=O)-O-(CH₂)ₙ-R¹⁵, -O-C(=O)-O-(CH₂)ₙ-R¹⁶, -C(=O)-NR¹⁷R¹⁸, -C(=S)-NR¹⁷R¹⁸, -N R¹⁷R¹⁸, -S(=O)ₙ-R¹⁹, -(CH₂)ₙ-Si-(R²⁰)ₙ and -(CH₂)ₙ-O-(CH₂)ₙ-Si-(R²¹)ₙ; whereby R¹ is unsubstituted or further substituted by 1, 2 or 3 identical or different groups R^{12a} which are independently selected from the group consisting of F, Cl, Br, I, OH, SH, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, -C(=O)-(OH),-C(=O)-(NR¹⁷R¹⁸), -C(=O)-(H), -O-C(=O)-(C₁-C₈-alkyl), -C(=O)-(C₁-C₈-alkyl) and -C(=O)-(O)-(C₁-C₈-alkyl);
R² is selected from the group consisting of H, halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R² is unsubstituted or further substituted by 1, 2 or 3 identical or different groups R^{12b} which are independently selected from the group consisting of CI, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, - S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, -Si-(C₁-C₈alkyl)₃, -Si-(C₁-C₈-alkyl)₂-phenyl, -Si-(phenyl)₃, phenyl and -CH₂-phenyl;
R³ is selected from the group consisting of F, Cl, Br, I, SH, CN, NO₂, NR¹⁷R¹⁸, -O-C₁-C₈-alkyl, - O-C₂-C₈-alkenyl, -O-C₂-C₈-alkynyl, C₁-C₈-alkyl, C₂-C₈-alkenyl, C₂-C₈-alkynyl, C=O(OH), C=O(NR¹⁷R¹⁸), C=O(H), OC=O(C₁-C₈-alkyl), C=O(O)(C₁-C₈-alkyl), OC=O(C₂-C₈-alkenyl), C=O(O)(C₂-C₈-alkenyl), OC=O(C₅-C₆-aryl), C=O(O)C₅-C₆-aryl, C₃-C₈-cycloalkyl, O-C₃-C₈-cycloalkyl, C=O(C₁-C₈-alkyl), C=O(C₅-C₆-aryl), C₅-C₆-aryl, O-C₅-C₆-aryl and C₃-C₈-cycloalkenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, C=O(OH), C=O(NR¹⁷R¹⁸), C=O(H), OC=O(C₁-C₈-alkyl), C=O(C₁-C₈-alkyl) and C=O(O)(C₁-C₈-alkyl);
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ and R²¹ are independently of each other, selected from the group consisting of H, F, Cl, Br, I, CN, C₁-C₈-alkyl, C₂-C₈-alkenyl, C₂-C₈-alkynyl, C₃-C₈-cycloalkyl, C₅-C₆-aryl, C₅-C₆-heterocycloalkenyl, C₅-C₆-heterocycloalkyl or C₅-C₆-heteroaryl; wherein heterocycloalkenyl, heterocycloalkyl or heteroaryl contain besides carbon atoms as ring members, 1, 2 or 3 heteroatoms independently selected from O, N or S as ring members; or R¹⁷ together with R¹⁸ form a C₅-C₆-heterocycloalkenyl, C₅-C₆-heterocycloalkyl or C₅-C₆-heteroaryl;
R⁴ is selected from the group consisting of F, Br, I, CN, -C(=O)-OR¹⁴, -O-C₁-C₈-alkyl, CH₂F, C₂-C₈-alkyl, C₂-C₈-alkenyl, C₂-C₈-alkynyl, C₃-C₈-cycloalkyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁-C₆-alkyl) and C₃-C₈-cycloalkenyl and C₃-C₈-cycloalkenyl whereby R⁴ is unsubstituted or further substituted by 1, 2 or 3 identical or different groups R^{12d} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl;
R⁵ is selected from the group consisting of H, halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R⁵ is unsubstituted or further substituted by 1, 2 or 3 identical or different groups R^{12e} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, - S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, -Si-(C₁-C₈alkyl)₃, --Si-(C₁-C₈-alkyl)₂-phenyl, -Si-(phenyl)₃, phenyl and -CH₂-phenyl; and
n is selected from 0, 1, 2, 3, 4 and 5;
or in the form of a stereoisomer or an agriculturally acceptable salt or a tautomer or an isotopic form of a N-oxide or a S-oxide or a prodrug thereof.

In preferred embodiment, the present invention provides the compound of formula (I'), wherein R¹ is selected from the group consisting H, -CH₂-OH, -CH₂-OCH₃, -CH₂-O-CH(CH₃)₂, -CH₂-O-(CH₂)₂-Si(CH₃)₃, -C(=O)-CH₃, -C(=O)-CH₂-CH₃, -C(=O)-(CH₂)₂-CH₃, -C(=O)-(CH₂)₃-CH₃, -C(=O)-(CH₂)₄-CH₃, -C(=O)-(CH₂)₅-CH₃, -C(=O)-C(CH₃)₃, -C(=O)-CH₂-OCH₃; -C(=O)-CH=CH-CH₃;-C(=O)-phenyl; -C(=O)-cyclopropyl; -C(=O)-CH₂-cyclopropyl; -C(=O)-CH₂-phenyl; -CH₂-O-C(=O)-CH₃; -CH₂-O-C(=O)-CH₂-CH₃; -CH₂-O-C(=O)-(CH₂)₃-CH₃; -CH₂-O-C(=O)-(CH₂)₄-CH₃; -CH₂-O-C(=O)-(CH₂)5-CH₃; -CH₂-O-C(=O)-C(CH₃)₃; -CH₂-O-C(=O)-phenyl; -C(=O)-OCH₃; C(=O)-O-(CH₂)₂CH₃; -C(=O)-O-(CH₂)₃-CH₃; -C(=O)-O-(CH₂)₄-CH₃; -C(=O)-O-(CH₂)₅-CH₃;-C(=O)-OCH₂-CH(CH₃)₂; -C(=O)-OCH(CH₃)₂; -C(=O)-O-CH₂-CCl₃; -C(=O)-OC(CH₃)₃; -C(=O)-O-(CH₂)₆-CH₃;-C(=O)-O-CH₂-CH=CH; -C(=O)-O-CH₂-C=CH; -C(=O)O-phenyl; -C(=O)-O-CH₂-phenyl; -CH₂-C(=O)-OCH₃; -CH₂-C(=O)-OCH₂-CH₃; -CH₂-C(=O)-O(CH₂)₂-CH₃; -CH₂-C(=O)-O(CH₂)₃-CH₃;-CH₂-C(=O)-O(CH₂)₄-CH₃; -CH₂-C(=O)-O(CH₂)₅-CH₃; -CH₂-C(=O)-OC(CH₃)₃; -CH₂-C(=O)-O-CH₂-CH=CH; -CH₂-C(=O)-O-CH=CH-CH₃; -C(=O)-NH₂; -C(=O)-NH-CH₃; -C(=O)-N-(CH₃)₂;-C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)2-CH₃; -S(=O)2-CH₂-CH₃; -S(=O)2-(CH₂)₂-CH₃; -S(=O)2-(CH₂)₂-CH₃;-S(=O)₂-tert-butyl and -S(=O)₂-phenyl; whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(H), -OC(=O)-(C₁-C₈-alkyl), -C(=O)-(C₁-C₈-alkyl) and -C(=O)-(O)(C₁-C₈-alkyl);
R² is selected from the group consisting H, Cl, Br, F, I, CN, -C(=O)-O-CH₃, -C(=O)-O-CH₂-CH₃,-C(=O)-O-(CH₂)₃-CH₃ -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, ethynyl, propynyl, cyclopropyl, cyclobutyl, cyclopentyl, cyclohexyl, cyclopropenyl, cyclobutenyl, cyclopentenyl and cyclohexenyl; whereby R² is unsubstituted or further substituted by 1, 2 or 3 identical or different groups R^{12b} which are independently selected from the group consisting of Cl, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂,-C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl;
R³ is selected from the group consisting of Cl, F, Br, I, CN, -O-CH₃, -O-CH₂-CH₃, -C(=O)-O-CH₃; -C(=O)-O-CH₂-CH₃; -C(=O)-O-(CH₂)₂-CH_{3;} -C(=O)-O-(CH₂)₃-CH₃; -C(=O)-O-(CH₂)₄-CH₃; -C(=O)-O-(CH₂)₅-CH₃; -C(=O)-O-CH(CH₃)₂; -C(=O)-O-C(CH₃)₃; methyl, ethyl, propyl, butyl, isopropyl, isobutyl, tert butyl, ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl; whereby R³ is unsubstituted or further substituted by 1, 2 or 3 identical or different groups R^{12c} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(H), -C(=O)-(C₁-C₈-alkyl) and -C(=O)-(O)(C₁-C₈-alkyl);
R⁴ is selected from the group consisting of H, F, Cl, Br, I, CN, -C(=O)-OR¹⁴, CH₂F, -O-C₁-C₈-alkyl, C₁-C₈-alkenyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁-C₆-alkyl) and C₃-C₈-cycloalkenyl and C₂-C₆-alkynyl, whereby R⁴ is unsubstituted or further substituted by 1, 2 or 3 identical or different groups R^{12d} which are independently selected from the group consisting of F, Cl, Br, I, CN, NO₂, NR¹⁷R¹⁸, - O-C₁-C₈-alkyl, -O-C₂-C₈-alkenyl, -O-C₂-C₈-alkynyl, C₁-C₈-alkyl, C₂-C₈-alkenyl and C₂-C₈-alkynyl; and
R⁵ is selected from the group consisting H, Cl, Br, F, I, CN, -C(=O)-O-CH₃, -C(=O)-O-CH₂-CH₃,-C(=O)-O-(CH₂)₃-CH₃ -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl; whereby R⁵ is unsubstituted or further substituted by 1, 2 or 3 identical or different groups R^{12e} which are independently selected from the group consisting of Cl, Br, F, I, CN,-NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl.

In more preferred embodiment, the present invention provides the compound of formula (I'), wherein
R¹ is selected from the group consisting H, -CH₂-OH, -CH₂-OCH₃, -C(=O)-phenyl; -C(=O)-cyclopropyl; -C(=O)-CH₂-cyclopropyl; -C(=O)-CH₂-phenyl; -CH₂-O-C(=O)-CH₃; -C(=O)-OCH₃;-C(=O)-O-CH₂-C≡CH; -C(=O)O-phenyl; -C(=O)-O-CH₂-phenyl; -CH₂-C(=O)-OCH₃; -C(=O)-NH₂;-C(=O)-NH-CH₃; -C(=O)-N-(CH₃)₂; -C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)₂-CH₃; -S(=O)₂-CH₂-CH₃;-S(=O)₂-(CH₂)₂-CH₃; -S(=O)₂-(CH₂)₂-CH₃; -S(=O)₂-tert-butyl and -S(=O)₂-phenyl; whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl, - O-C₂-C₈-alkenyl, -O-C₂-C₈-alkynyl, C₁-C₈-alkyl, C₂-C₈-alkenyl, C₂-C₈-alkynyl, -C(=O)-(OH), - C(=O)-(H);
R² is selected from the group consisting H, Cl, Br, F, I, CN, -C(=O)-O-CH₃, - methyl, ethyl, propyl, butyl, hexyl, heptyl, isopropyl, isobutyl, isopentyl, tert butyl, ethynyl, propynyl, cyclopropyl, cyclobutyl, cyclopentyl, cyclohexyl, cyclopropenyl, cyclobutenyl, cyclopentenyl and cyclohexenyl; whereby R² is unsubstituted or further substituted by 1, 2 or 3 identical or different groups R^{12b} which are independently selected from the group consisting of Cl, Br, F, I, CN, -NH₂,-C(=O)-H, -C(=O)-C₁-C₈-alkyl, -C(=O)-OH, -C(=O)-O-C₁-C₈-alkyl, -C(=O)-NH₂, -S(=O)-C₁-C₈-alkyl, -S(=O)-phenyl, -S(=O)₂-C₁-C₈-alkyl, -S(=O)₂-phenyl, -S(=O)₂-NH₂,, phenyl and -CH₂-phenyl;
R³ is selected from the group consisting of Cl, F, Br, I, CN, -O-CH₃, -C(=O)-O-CH₃; methyl, ethyl, propyl, butyl, isopropyl, isobutyl, tert butyl, ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl; whereby R³ is unsubstituted or further substituted by 1, 2 or 3 identical or different groups R^{12c} which are independently selected from the group consisting of F, Cl, Br, I, CN, NO₂, NR¹⁷R¹⁸, C₁-C₈-alkyl, C₂-C₈-alkenyl, C₂-C₈-alkynyl, -C(=O)-(OH), -C(=O)-(H);
R⁴ is selected from the group consisting of H, F, Cl, Br, I, CN, -C(=O)-OR¹⁴, CH₂F, -O-C₁-C₈-alkyl, C₁-C₈-alkenyl, -O-CN, -N-CN, -S-CN, C=O(NR¹⁷R¹⁸), C=O(H), NR¹⁷R¹⁸, S-(C₁-C₆-alkyl), C₃-C₈-cycloalkenyl and C₂-C₆-alkynyl, whereby R⁴ is unsubstituted or further substituted by 1, 2 or 3 identical or different groups R^{12d} which are independently selected from the group consisting of F, Cl, Br, I, CN, NO₂, NR¹⁷R¹⁸, -O-C₁-C₈-alkyl, C₁-C₈-alkyl, C₂-C₈-alkenyl and C₂-C₈-alkynyl; and
R⁵ is selected from the group consisting H, Cl, Br, F, I, CN, -C(=O)-O-CH₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl; whereby R⁵ is unsubstituted or further substituted by 1, 2 or 3 identical or different groups R^{12e} which are independently selected from the group consisting of CI, Br, F, I, CN, -NH₂, -C(=O)-H, -C(=O)-OH,-C(=O)-O-phenyl, -CH₂-C(=O)-OH, -C(=O)-NH-phenyl, -S(=O)₂-phenyl, phenyl and -CH₂-phenyl.

In yet more preferred embodiment, the present invention relates to compound of formula (I'), wherein,
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are independently selected from the group consisting of F, Cl, Br and I; and
R⁴ is selected from the group consisting of F, Br, I, CN, -O-C₁-C₈-alkyl, CHF₂, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I; and
R⁵ is selected from the group consisting of H, F and Cl.

In preferred embodiment, the present invention provides a compound of formula (I'), wherein A, together with two carbon atoms of the pyrrole ring, is selected from the group consisting of

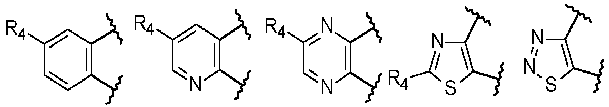

In preferred embodiment, the present invention provides a compound of formula (I'), wherein,
X denotes CH or N; Y denotes O or S;
A, together with two carbon atoms of the pyrrole ring, is selected from the group consisting of 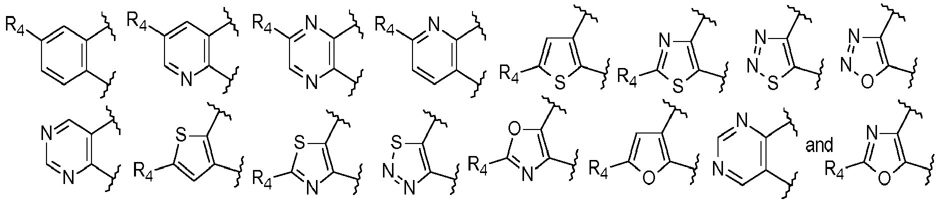 ;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are independently selected from the group consisting of F, Cl, Br and I; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I; and
R⁵ is selected from the group consisting of H, F and Cl.

In more preferred embodiment, the present invention provides a compound of formula (I'), wherein,
X denotes N and Y denotes O;
A, together with two carbon atoms of the pyrrole ring, is selected from the group consisting of 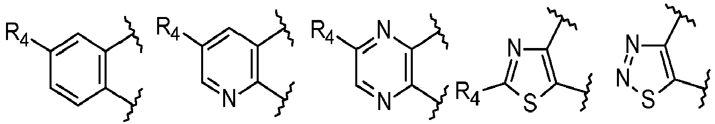 ;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and
R⁵ is selected from the group consisting of H, F and Cl.

In yet more preferred embodiment, the present invention provides a compound of formula (I'), wherein the compound of formula (I') is a compound of formula (I'A), formula (I'B), formula (I'C) or formula (I'D) 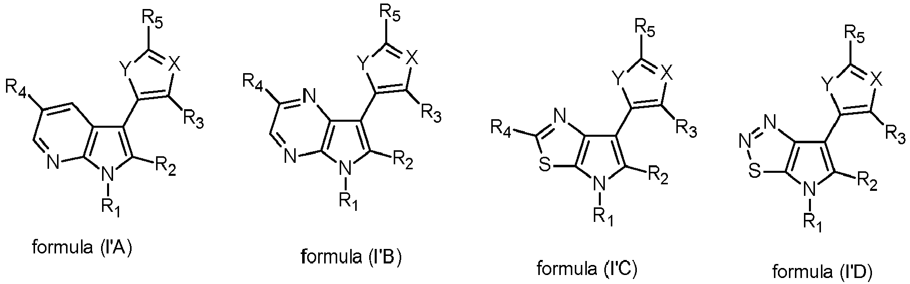 wherein;
X denotes CH or N; Y denotes O or S;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and
R⁵ is selected from the group consisting of H, F and Cl.

In yet more preferred embodiment, the present invention provides a compound of formula (I'), wherein the compound of formula (I') is a compound of formula (I'A), formula (I'B), formula (I'C) or formula (I'D), 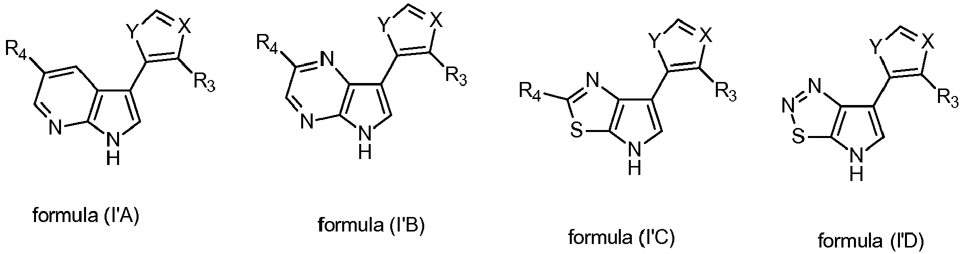 wherein;
X denotes N; Y denotes O;
R³ is selected from the group consisting of F, Cl, CF₃, CHF₂ and CH₂F; and
R⁴ is selected from the group consisting of F, CN and -C=CH.

In most preferred embodiment, the present invention provides a compound of formula (I'), wherein the compound of formula (I') is a compound of formula (I'A) or formula (I'B), 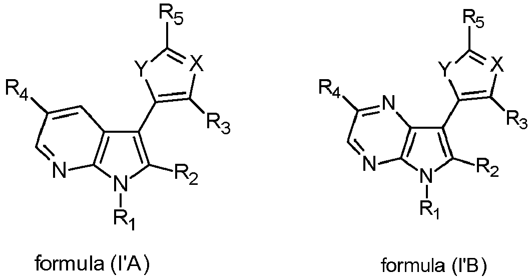 wherein;
X denotes N; Y denotes O;
R¹ is H; R² is selected from the group consisting of H, F and Cl;
R³ is selected from the group consisting of F, Cl, CF₃, CHF₂ and CH₂F; and
R⁴ is selected from the group consisting of F, CN and -C=CH; and
R⁵ is selected from the group consisting of H, F and Cl.

In preferred embodiment, the present invention provides a compound of formula (I'), wherein the compound of formula (I') is a compound of formula (I'A) or formula (I'B), 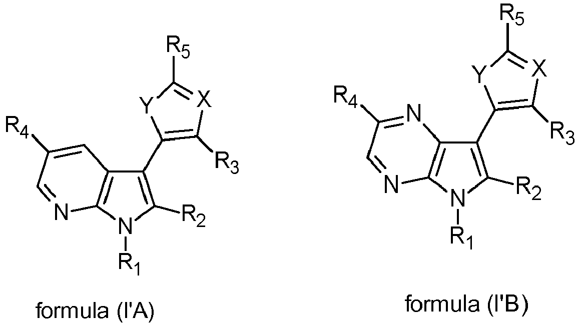 wherein;
X denotes N; Y denotes S;
R¹ is H; R² is selected from the group consisting of H, F and Cl;
R³ is selected from the group consisting of F, Cl, CF₃, CHF₂ and CH₂F; and
R⁴ is selected from the group consisting of F, CN and -C=CH; and
R⁵ is selected from the group consisting of H, F and Cl.

In preferred embodiment, the present invention provides the compound of formula (I'), wherein the compound of formula (I') is the compound of formula (I'E) 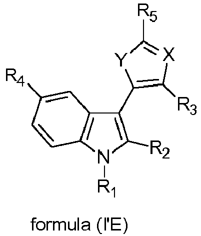 wherein;
X denotes CH or N; Y denotes O or S;
R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and
R⁵ is selected from the group consisting of H, F and Cl.

In preferred embodiment, the present invention provides an agrochemical mixture comprising at least one compound of formula (I'), or in the form of a stereoisomer or an agriculturally acceptable salt or a tautomer or an isotopic form or a N-oxide or a S-oxide or a prodrug thereof.

In preferred embodiment, the present invention provides a composition comprising at least one compound of formula (I'), or in the form of a stereoisomer or an agriculturally acceptable salt or a tautomer or an isotopic form of a N-oxide or a S-oxide or a prodrug thereof, and an auxiliary.

In preferred embodiment, the present invention provides the use of the compound of formula (I) in a method of protecting a crop of useful plants susceptible to and/or under attack by fungi, which method comprises applying to said crop, treating a plant propagation material of said crop with, and/or applying to said fungi, the composition comprising at least one compound of formula (I).

In preferred embodiment, the present invention provides the use of the compound of formula (I), which comprises treating the fungi, the plant, or the plant propagation material selected from the group consisting of seeds, roots, fruits, tubers, bulbs, rhizomes, shoots, sprouts and other parts of plants, including seedlings and young plants to be protected against fungi attack, the stored materials or harvest, or alternately, the locus or soil or soil substituents or surfaces therefrom, with an effective amount of at least one compound of formula (I).

The compounds of formula (I) and/or (I') can be prepared by standard processes of organic chemistry.

The compounds of formula (I) and/or (I') can be prepared according to methods or in analogy to standard techniques that are described in the state of art. The synthetic procedure utilises the starting materials that are either commercially available or that may be prepared according to conventional procedures starting from readily available compounds.

### General Procedures:

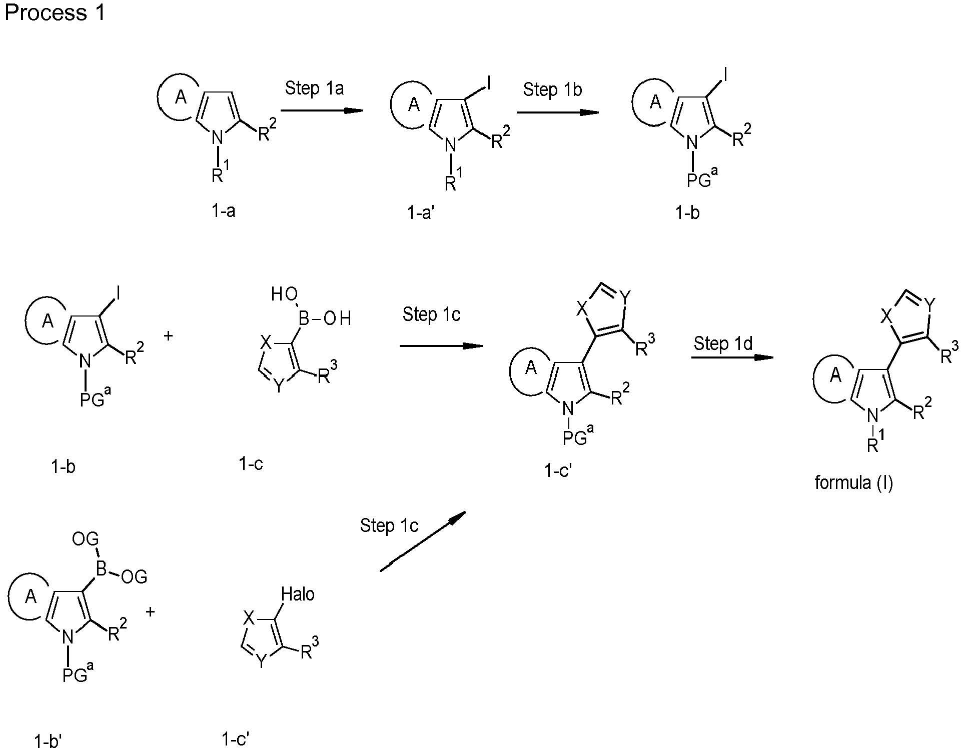

### Reaction conditions:

Step 1a: Potassium iodide (KI), iodine (I) and N-iodosuccinimide; dimethyl formamide (DMF), methanol, ethanol and water; 2- 16 h; RT (room temperature);
Step 1b: Tosyl chloride and tert-butoxycarbonyl anhydride; 2-16 h; 0°C; or Me₂NSO₂Cl and NaH in THF or DMF
Step 1c: Tetrahydrofuran (THF) or DMF; Potassium carbonate (K₂CO₃) or Na₂CO₃) or their mixture; 2-14 h; 110°C
Step 1d: Sodium hydride (NaH), Sodium methoxide (MeONa) and Sodium ethoxide (EtONa); 1 to 24 h; RT or 0°C to 90°C;

### Step 1a:

This process step comprises reacting compound of formula 1-a and R¹ is hydrogen; with a halogenating agent selected from the group consisting of KI, I and N-iodosuccinimide, or KBr, N-bromosuccinimide or Br₂ to obtain the corresponding Bromo derivative in the presence of solvent selected from the group consisting of dimethylformamide, dichloromethane, dichloroethane, methanol, ethanol and water to obtain a compound of formula 1-a'.

### Step 1b:

This process step comprises reacting the compound of formula 1-a' with a reagent selected from the group consisting of a sulfonyl chloride (e.g.: tosyl chloride) and tert-butoxycarbonyl anhydride or a base and sulfonyl chloride in a non-protic solvent such as tetrahydrofuran or dimethylformamide to obtain a compound of formula 1-b.

### Step 1c:

This process step comprises reacting the compound of formula 1-a with compound of formula 1-b in the presence of a polar solvent such as tetrahydrofuran or dimethylformamide and a base selected from the group consisting of K₂CO₃ and Na₂CO₃ to form a compound of formula (1-c').

### Step 1d:

This process step comprises reacting the compound of formula 1-c' in the presence of a base selected from the group consisting of NaH, MeONa and EtONa or TBAF in tetrahydrofuran or dimethylformamide to obtain a compound of formula (I); wherein R¹ is H;

### Step 1e:

This process step comprises replacing the R³ group in compound of formula (I) obtained in step (1d) with any other required group with a suitable reagent.

### Step 1f:

This process step comprises reacting the compound of formula (I) obtained in step (1d) with an appropriate R1 in the presence of a suitable base; to obtain a compound of formula (I) or if desired, the compound of formula (I) obtained in step (1d) or step (1e) is converted to its agriculturally acceptable salts.

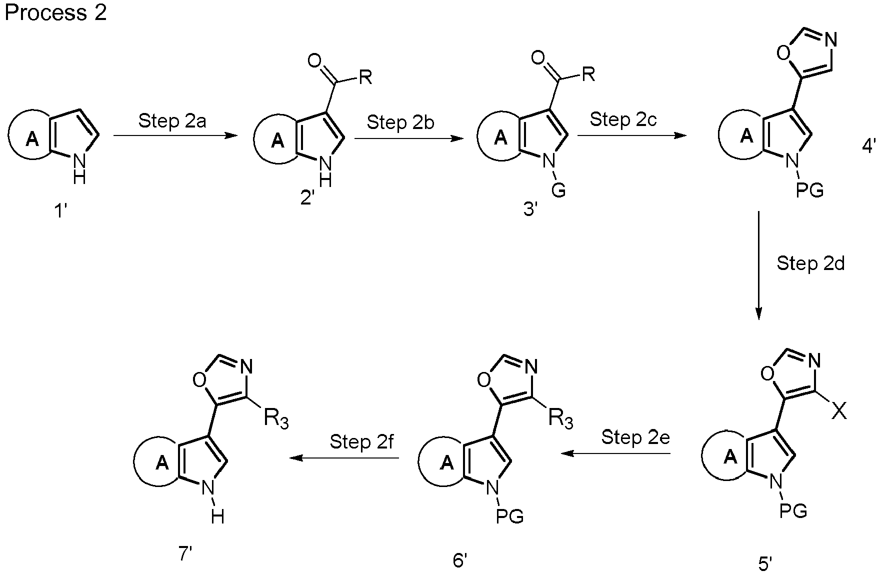

### Reaction conditions:

Step 2a: When R=H, Phosphoryl chloride (POCl₃) and dimethyl formamide (DMF), 0°C, 15-60 minutes and then for 1- 8 hours at room temperature (RT); followed by work-up with sodium hydroxide (NaOH) and water; or AcOH/H₂O, hexamethylenetetramine, reflux; 2-16 h.
   When R=CH₃, AlCl₃, Acetyl chloride in DCM 0°C - RT; 2-16 hours
Step 2b: When R=H, tosyl chloride and tert-butoxycarbonyl anhydride, 2-16 h, 0°C to room temperature; or Me₂NSO₂Cl and NaH in THF or DMF at 0°C - 5°C.
   When R=CH₃, Step 2b will be performed after 2c with the above-mentioned reagents.
Step 2c: When R=H, TosMIC; Methanol (MeOH), Potassium carbonate (K₂CO₃) or Na₂CO₃ or their mixture, 2-16 h at room temperature to 85 °C, or TosMIC, resin in DME/MeOH or mixture at RT-100 °C for 2-14 h.
Step 2d: for iodo derivative - LiHMDS or NaHMDS and iodine (I₂) or N-iodosuccinimide in THF or NIS, THF/CCl₄, 2- 16 h at room temperature;
   for Bromo-derivative: KBr, Bromine (Br₂), N-Bromosuccinimide, dimethyl formamide (DMF)/THF/CCl₄, 2- 16 h, room temperature or LiHMDS and Br₂ in THF/Diethylether.
Step 2e: when R₃=CF₃ - FSO₂CF₂CO₂Me, CuI, DMF at 60-130 °C, 2-16 h.
Step 2f: Deprotection of protecting group using standard reaction conditions such as sodium hydride (NaH), sodium methoxide (MeONa) or sodium ethoxide (EtONa) for 1 to 24 h at room temperature or 0°C to 90°C; or TBAF in DMSO.

The compounds of formula (I) and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective, and they can be used in crop protection as foliar fungicides, fungicides for seed dressing and soil fungicides. Moreover, they are suitable for controlling harmful fungi, which inter alia occur in wood or roots of plants.

The compounds of formula (I) and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds of formula (I) and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds of formula (I) and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified using recombinant DNA techniques that under natural circumstances cannot readily be obtained by cross breeding, mutations or natural recombination. Typically, one or more genes have been integrated into the genetic material of a genetically modified plant to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibitors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and an herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady® (glyphosate-tolerant, Monsanto, U.S.A.), Cultivance® (imidazolinone tolerant, BASF SE, Germany) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CrylA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme phosphinothricin-N-acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are using recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the Mexican wild potato *Solanum bulbocastanum*) or T4-lysozym (e. g. potato cultivars capable of synthesizing these proteins with increased resistance against bacteria such as *Erwinia amylovora*). The methods for producing such genetically modified plants are generally known to the person skilled in the art and are described, e. g. in the publications mentioned above.

Furthermore, plants are also covered that are using recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain using recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain using recombinant DNA techniques a modified amount of substances of content or new substances of content, specifically to improve raw material production, e. g. potatoes that produce increased amounts of amylopectin (e. g. Amflora® potato, BASF SE, Germany).

The compounds of formula (I) and compositions thereof, respectively, are particularly suitable for controlling the following plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solani* or *A. alternata*), tomatoes (e. g. *A. solani* or *A. alternata*) and wheat; *Aphanomyces* spp. on sugar beets and vegetables; *Ascochyta* spp*.* on cereals and vegetables, e. g. *A. tritici* (anthracnose) on wheat and *A. hordei* on barley; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeiiana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (*Dutch* elm disease) on elms; *Cercospora* spp. (Cercospora leaf spots) on corn (e. g. Gray leaf spot: *C. zeae-maydis*), rice, sugar beets (e. g. *C*. *beticola*)*,* sugar cane, vegetables, coffee, soybeans (e. g. *C. sojina* or *C. kikuchii*) and rice; *Cladosporium* spp*.* on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (C. *carbonum*), cereals (e. g. *C. sativus,* anamorph: *B. sorokiniana*) and rice (e. g. *C. miyabeanus,* anamorph: *H. oryzae*)*; Colletotrichum* (teleomorph: *Glomerella*) spp. (anthracnose) on cotton (e. g. *C. gossypii*)*,* corn (e. g. *C. graminicola:* Anthracnose stalk rot), soft fruits, potatoes (e. g. *C. coccodes*: black dot), beans (e. g. *C*. *lindemuthianum*) and soybeans (e. g. *C. truncatum* or *C. gloeosporioides*)*; Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D. tritici-repentis*: tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa, Elsinoe* spp. on pome fruits (*E. pyri*), soft fruits (*E. veneta:* anthracnose) and vines (*E. ampelina:* anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumani-ae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M. fijiensis* (black Sigatoka disease) on bananas; *Peronospora* spp. (downy mildew) on cabbage (e. g. *P. brassicae*), rape (e. g. *P. parasitica*), onions (e. g. *P. destructor*)*,* tobacco (*P. tabacina*) and soybeans (e. g. *P. manshurica*); *Phakopsora pachyrhizi* and *P. meibomiae* (soybean rust) on soybeans; *Phialophora* spp*.* e. g. on vines (e. g. *P. tracheiphila* and *P. tetraspora*) and soybeans (e. g. *P. gregata:* stem rot); *Phoma lingam* (root and stem rot) on rape and cabbage and *P. betae* (root rot, leaf spot and damping-off) on sugar beets; *Phomopsis* spp*.* on sunflowers, vines (e. g. *P. viticola*: can and leaf spot) and soybeans (e. g. stem rot: *P. phaseoli,* teleomorph: *Diaporthe phaseolorum*)*; Physoderma maydis* (brown spots) on corn; *Phytophthora* spp. (wilt, root, leaf, fruit and stem root) on various plants, such as paprika and cucurbits (e. g. *P. capsici*)*,* soybeans (e. g. *P. megasperma,* syn. *P. sojae*), potatoes and tomatoes (e. g. *P. infestans.* late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii* (*orange* rust) on sugar cane and *P. asparagi* on asparagus; *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stoionifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*)*; Septoria* spp. on various plants, e. g. *S. glycines* (brown spot) on soybeans, *S. tritici* (Septoria blotch) on wheat and *S.* (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum,* syn. *Helminthosporium turcicum*) and turf; *Sphacelotheca* spp. (smut) on corn, (e. g. *S. reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp*.* (black root rot) on tobacco, pome fruits, vegetables, soybeans and cotton, e. g. *T. basicola* (syn. *Chalara elegans*)*; Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda and U. avaenae*), corn (e. g. *U. maydis*: corn smut) and sugar cane; *Venturia* spp. (scab) on apples (e. g. *V*. *inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds of formula (I) and compositions thereof, respectively, are also suitable for controlling harmful fungi in the protection of stored products or harvest and in the protection of materials.

The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula spp.* and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds of formula (I) and compositions thereof, respectively, may be used for improving the health of a plant. The invention also relates to a method for improving plant health by treating a plant, its propagation material and/or the locus where the plant is growing or is to grow with an effective amount of compounds of formula (I) and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress. The above identified indicators for the health condition of a plant may be interdependent or may result from each other.

The compounds of formula I can be present in different crystal modifications whose biological activity may differ. They are likewise subject matter of the present invention.

The compounds of formula (I) are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds of formula (I) as such or a composition comprising at least one compound of formula (I) prophylactically either at or before planting or transplanting.

The invention also relates to agrochemical compositions comprising an auxiliary and at least one compound of formula (I) according to the invention.

An agrochemical composition comprises a fungicidally effective amount of a compound of formula (I). The term "effective amount" denotes an amount of the composition or of the compounds of formula (I), which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound of formula (I) used.

The compounds of formula (I), their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005. Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates. Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids. Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:
i) Water-soluble concentrates (SL, LS)
   10-60 wt% of a compound of formula (I) and 5-15 wt% wetting agent (e. g. alcohol alkoxylates) are dissolved in water and/or in a water-soluble solvent (e. g. alcohols) ad 100 wt%. The active substance dissolves upon dilution with water.
ii) Dispersible concentrates (DC)
   5-25 wt% of a compound of formula (I) and 1-10 wt% dispersant (e. g. polyvinyl pyrrolidone) are dissolved in organic solvent (e. g. cyclohexanone) ad 100 wt%. Dilution with water gives a dispersion.
iii) Emulsifiable concentrates (EC)
   15-70 wt% of a compound of formula (I) and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.
iv) Emulsions (EW, EO, ES)
   5-40 wt% of a compound of formula (I) and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound of formula (I) are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.
vi) Water-dispersible granules and water-soluble granules (WG, SG)
   50-80 wt% of a compound of formula (I) are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.
viii) Gel (GW, GF)
   In an agitated ball mill, 5-25 wt% of a compound of formula (I) are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.
ix) Microemulsion (ME)
   5-20 wt% of a compound of formula (I) are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound of formula (I), 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound of formula (I) according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.
xi) Dustable powders (DP, DS)
   1-10 wt% of a compound of formula (I) are ground finely and mixed intimately with solid carrier (e. g. finely divided kaolin) ad 100 wt%.
xii) Granules (GR, FG)
   0.5-30 wt% of a compound of formula (I) is ground finely and associated with solid carrier (e. g. silicate) ad 100 wt%. Granulation is achieved by extrusion, spray-drying or fluidized bed.
xiii) Ultra-low volume liquids (UL)
   1-50 wt% of a compound of formula (I) are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, more preferably between 1 and 70%, and in particular between 10 and 60%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound of formula (I) and compositions thereof, respectively, onto plant propagation material, especially seeds, include dressing, coating, pelleting, dusting, and soaking as well as in-furrow application methods. Preferably, compound of formula (I) or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on microorganisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
Microbial pesticides consist of bacteria, fungi or viruses (and often include the metabolites that bacteria and fungi produce). Entomopathogenic nematodes are also classified as microbial pesticides, even though they are multi-cellular.

Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

When living microorganisms, such as microbial pesticides from groups L1), L3) and L5), form part of such kit, it must be taken care that choice and amounts of the components (e. g. chemical pesticides) and of the further auxiliaries should not influence the viability of the microbial pesticides in the composition mixed by the user. Especially for bactericides and solvents, compatibility with the respective microbial pesticide has to be taken into account.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds of formula (I) or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds of formula (I) can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22),), 1-[2-[[1-(4-chlorophenyl)pyrazol-3-yl]oxy¬methyl]-3-methyl-phenyl]-4-methyl-tetra¬zol-5-one (A.1.25), (Z,2E) 5 [1-(2,4-dichloro¬phenyl)pyrazol-3-yl]-oxy-2-methoxyimino-N,3-dimethyl-pent-3-en¬amide (A.1.34), (Z,2E) 5 [1(4-chlorophenyl)pyrazol-3-yl]oxy-2-methoxyimino-N,3-dimethyl-pent-3-enamide (A.1.35), pyriminostrobin (A.1.36), bifujunzhi (A.1.37), 2-(ortho-((2,5-dimeth¬ylphenyl-oxy¬methylen)phenyl)-3-methoxy-acrylic acid methylester (A.1.38);
   - inhibitors of complex III at Qi site: cyazofamid (A.2.1), amisulbrom (A.2.2), [(6S,7R,8R) 8 benzyl-3-[(3-hydroxy-4-methoxy-pyridine-2-carbonyl)amino]-6-methyl-4,9-di¬oxo-1,5-dioxonan-7-yl] 2-methylpropanoate (A.2.3), fenpicoxamid (A.2.4);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam N-[(2Z)-2-[3-chloro-5-(2-cyclopropylethynyl)-2-pyridyl]-2-isopropoxyimino-ethyl]-3-(difluoromethyl)-1-methyl-pyrazole-4-carboxamide (A.3.23), fluindapyr (A.3.28), methyl (E)-2-[2-[(5-cyano-2-methyl-phenoxy)methyl]phenyl]-3-methoxy-prop-2 enoate (A.3.30), isoflucypram (A.3.31), 2-(difluoromethyl)-N-(1,1,3-trimethyl-indan-4-yl)pyridine-3-carboxamide (A.3.32), 2-(difluoromethyl)-N-[(3R)-1,1,3-trimethylindan-4-yl]pyridine-3-carboxamide (A.3.33), 2-(difluoromethyl)-N-(3-ethyl-1,1-dimethyl-indan-4-yl)-pyridine-3-carboxamide (A.3.34), 2-(difluoromethyl)-N-[(3R)-3-ethyl-1,1-dimethyl-indan-4-yl]-pyridine-3-carboxamide (A.3.35), 2-(difluoromethyl)-N-(1,1-dimethyl-3-propyl-indan-4-yl)¬py-ridine-3-carboxamide (A.3.36), 2-(difluoromethyl)-N-[(3R)-1,1-dimethyl-3-propyl-indan-4-yl]-pyridine-3-carboxamide (A.3.37), 2-(difluoromethyl)-N-(3-isobutyl-1,1-dimethyl-indan-4-yl)-pyridine-3-carboxamide (A.3.38), 2-(difluoromethyl)-N-[(3R)-3-isobutyl-1,1-dimethyl-indan-4 yl]pyridine-3-carboxamide (A.3.39) ;
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromu¬conazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothio¬conazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), ipfentrifluconazole , (B.1.37), mefentrifluconazole (B.1.38), 2-(chloromethyl)-2-methyl-5-(p-tolylmethyl)-1 (1,2,4-triazol-1 ylmethyl)cyclopentanol (B.1.43); imidazoles: imazalil (B.1.44), pefurazoate (B.1.45), prochloraz (B.1.46), triflumizol (B.1.47); pyrimidines, pyridines and piperazines: fe¬narimol (B.1.49), pyrifenox (B.1.50), triforine (B.1.51), [3-(4-chloro-2-fluorophenyl)-5-(2,4-di¬fluoro-phenyl)isoxazol-4-yl]-(3-pyridyl)methanol (B.1.52);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8); - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4 amine (C.2.6), 5-fluoro-2-(4-fluorophenylmethoxy)pyrimidin-4 amine (C.2.7), 5-fluoro-2 (4 chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), 3-chloro-4-(2,6-difluorophenyl)-6-methyl-5-phenyl-pyri¬da-zine (D.1.6), 3-chloro-6-methyl-5-phenyl-4-(2,4,6-trifluorophenyl)pyridazine (D.1.7), N eth¬yl-2-[(3-ethynyl-8-methyl-6-quinolyl)oxy]butanamide (D.1.8), N-ethyl-2-[(3-ethynyl-8 methyl-6 quinolyl)oxy]-2-methylsulfanyl-acetamide (D.1.9), 2-[(3-ethynyl-8-methyl-6-quinol-yl)oxy]-N (2-fluoroethyl)butanamide (D.1.10), 2-[(3-ethynyl-8-methyl-6-quinolyl)oxy]-N-(2-flu¬oroeth¬yl)-2-methoxy-acetamide (D.1.11), 2-[(3-ethynyl-8-methyl-6-quinolyl)oxy]-N-propyl-butanam¬ide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2 [(3 ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3 amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7);
E) Inhibitors of amino acid and protein synthesis
   - methionine synthesis inhibitors: cyprodinil (E.1.1), mepani¬pyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), 2-{3-[2-(1-{[3,5-bis(di¬fluoromethyl-1H-pyrazol-1-yl]acetyl}piperidin-4-yl)-1,3-thiazol-4-yl]-4,5-dihydro-1,2 oxazol-5-yl}-phenyl methanesulfonate (G.5.2), 2-{3-[2-(1-{[3,5-bis(difluoro¬methyl)-1H-pyrazol-1-yl]-acetyl}piperidin-4-yl) 1,3-thiazol-4-yl]-4,5-dihydro-1,2-oxazol-5 yl}-3-chlorophenyl methanesulfonate (G.5.3), 4-[1-[2-[3-(difluoromethyl)-5-methyl-pyrazol-1-yl]acetyl]-4-piperidyl]-N-te¬tralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyridine-2-carboxamide (G.5.7), 4-[1-[2-[5-methyl-3-(trifluoromethyl)pyrazol-1-yl]acetyl]-4-pi¬peridyl]-N-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-N-tetralin-1-yl-pyridine-2-carboxamide (G.5.9), 4 [1 [2-[3,5-bis(trifluoromethyl)pyrazol-1-yl]acetyl]-4-piperidyl]-N-tetralin-1-yl-pyridine-2-car¬boxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11) ;
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachloro¬benzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-di¬methyl-1H,5H-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (I.2.2), carpropamid (1.2.3), dicyclomet (I.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4 cyclopropyl-N-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclo¬mezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), N'-(4-(4-chloro-3-trifluoro¬methyl-phen¬oxy)-2,5-dimethyl-phenyl)-N-ethyl-N methyl formamidine (K.1.27), N' (4-(4-fluoro-3-trifluoro¬methyl-phenoxy)-2,5-dimethyl-phenyl)-N-eth¬yl-N-methyl formamidine (K.1.28), N'-[4-[[3-[(4-chlorophenyl)methyl]-1,2,4-thiadiazol-5-yl]¬oxy]-2,5-dimethyl-phenyl]-N-ethyl-N-methyl-formamidine (K.1.29), N'-(5-bromo-6-indan-2-yl¬oxy-2-methyl-3-pyridyl)-N-ethyl-N-methyl-formamidine (K.1.30), N'-[5-bromo-6-[1-(3,5-difluorophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N' [5 bromo-2-methyl-6-(1-phenylethoxy)-3-pyridyl]-N-ethyl-N-methyl-formamidine (K.1.33), N'-(2-methyl-5-trifluoromethyl-4-(3-trimethyl¬silanyl-prop¬oxy)-phenyl)-N-ethyl-N-methyl forma-midine (K.1.34), N'-(5-difluoromethyl-2 methyl-4-(3-tri¬methylsilanyl-propoxy)-phenyl)-N-ethyl-N-methyl formamidine (K.1.35), 2-(4-chloro-phenyl)-N-[4-(3,4-dimeth¬oxy-phenyl)-isoxazol-5 yl]-2-prop-2-ynyloxy-acetamide (K.1.36), 3 [5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1 (4,6-di¬methoxy-pyrimidin-2-yl)-2-methyl-1H-benzoimidazole (K.1.39), ethyl (Z) 3 amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxy¬methyl]-2-pyridyl]carba-mate (K.1.42), but-3-ynyl N-[6-[[(Z)-[(1-methyltetrazol-5-yl)-phenyl-methylene]amino]oxy-methyl]-2-pyridyl]carbamate (K.1.43), 2-[2-[(7,8-difluoro-2-methyl-3-quinolyl)oxy]-6-fluoro-phenyl]propan-2-ol (K.1.44), 2-[2-fluoro-6-[(8-fluoro-2-methyl-3-quinolyl)oxy]¬phen-yl]propan-2-ol (K.1.45), quinofumelin (K.1.47), 9-fluoro-2,2-dimethyl-5-(3-quinol¬yl)-3H 1,4 benzoxazepine (K.1.49), 2-(6-benzyl-2-pyridyl)quinazoline (K.1.50), 2-[6-(3-fluoro-4 methoxy-phenyl)-5-methyl-2-pyridyl]quinazoline (K.1.51), dichlobentiazox (K.1.52), N'-(2,5-dimethyl-4-phenoxy-phenyl)-N-ethyl-N-methyl-formamidine (K.1.53), py rifenamine (K.1.54);
L) Biopesticides
   L1) Microbial pesticides with fungicidal, bactericidal, viricidal and/or plant defense activator activity: Ampelomyces quisqualis, Aspergillus flavus, Aureobasidium pullulans, Bacillus altitudinis, B. amyloliquefaciens, B. megaterium, B. mojavensis, B. mycoides, B. pumilus, B. simplex, B. solisalsi, B. subtilis, B. subtilis var. amyloliquefaciens, Candida oleophila, C. saitoana, Clavibacter michiga¬nensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, Paenibacillus polymyxa, Pantoea vagans, Penicillium bilaiae, Phlebiopsis gigantea, Pseudomonas sp., Pseudomonas chloraphis, Pseudozyma flocculosa, Pichia anomala, Pythium oligandrum, Sphaerodes myco¬parasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia, zucchini yellow mosaic virus (avirulent strain);
   L2) Biochemical pesticides with fungicidal, bactericidal, viricidal and/or plant defense activator activity: harpin protein, Reynoutria sachalinensis extract;
   L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tenebrionis, Beauveria bassiana, B. brongniartii, Burkholderia spp., Chromobacterium subtsugae, Cydia pomonella granulovirus (CpGV), Cryptophlebia leucotreta granulovirus (CrleGV), Flavobacterium spp., Helicoverpa armigera nucleopolyhedrovirus (HearNPV), Helicoverpa zea nucleopolyhedrovirus (HzNPV), Helicoverpa zea single capsid nucleopolyhedrovirus (HzSNPV), Heterorhabditis bacteriophora, Isaria fumosorosea, Lecanicillium longispo¬rum, L. muscarium, Metarhizium anisopliae, Metarhizium anisopliae var. anisopliae, M. anisopliae var. acridum, Nomuraea rileyi, Paecilomyces fumosoroseus, P. lilacinus, Paenibacillus popilliae, Pasteuria spp., P. nishizawae, P. penetrans, P. ramosa, P. thornea, P. usgae, Pseudomonas fluorescens, Spodoptera littoralis nucleopoly¬hedrovirus (SpliNPV), Steinernema carpocapsae, S. feltiae, S. kraussei, Streptomyces galbus, S. microflavus;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadeca-dien-1-ol acetate, (E,Z)-3,13-octadecadien-1-ol, R-1-octen-3-ol, pentatermanone, (E,Z,Z)-3,8,11-tetradecatrienyl acetate, (Z,E) 9,12-tetradecadien-1-yl acetate, Z-7-tetradecen-2-one, Z-9-tetradecen-1-yl acetate, Z-11-tetradecenal, Z-11-tetradecen-1-ol, extract of Chenopodium ambrosiodes, Neem oil, Quillay extract;
   L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium spp., B. elkanii, B. japo¬ni¬cum, B. liaoningense, B. lupini, Delftia acidovorans, Glomus intraradices, Mesorhizo¬bium spp., Rhizobium leguminosarum bv. pha¬seoli, R. I. bv. trifolii, R. I. bv. viciae, R. tropici, Sinorhizobium meliloti;
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dike¬gulac, dimethipin, 2,6-dimethylpuridine, ethephon, flumetralin, flurprimidol, fluthi¬acet, forchlorfenuron, gibberellic acid, inabenfide, indole-3-acetic acid, maleic hydrazide, mefluidide, mepiquat, mepiquat chloride, naphthaleneacetic acid, N 6 benzyladenine, paclobutrazol, prohexadione, prohexadione-calcium, prohydrojasmon, thidiazuron, triapenthenol, tributyl phosphoro¬trithioate, 2,3,5 tri iodobenzoic acid, trinexapac-ethyl and uniconazole;
N) Herbicides from classes N.1 to N.15
   N.1 Lipid biosynthesis inhibitors: alloxydim, alloxydim-sodium, butroxydim, clethodim, clodinafop, clodinafop-propargyl, cycloxydim, cyhalofop, cyhalofop-butyl, diclofop, diclofopmethyl, fenoxaprop, fenoxaprop-ethyl, fenoxaprop-P, fenoxaprop-P-ethyl, fluazifop, fluazifopbutyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-chloro-4-cyclo¬propyl-2'-fluoro[1,1'-biphenyl]-3-yl)-5-hydroxy-2,2,6,6-tetramethyl-2H-pyran-3(6H)-one (CAS 1312337-72-6); 4-(2',4'-dichloro-4-cyclopropyl[1,1'-biphenyl]-3-yl)-5-hydroxy-2,2,6,6-tetramethyl-2H-pyran-3(6H)-one (CAS 1312337-45-3); 4 (4' chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetra¬methyl-2H-pyran-3,5(4H,6H)-dione (CAS 1312340-84-3); 5-(acetyloxy)-4-(4'-chloro-4-cyclo¬propyl-2'-fluoro[1,1'-biphenyl]-3-yl)-3,6-dihydro-2,2,6,6-tetramethyl-2H-pyran-3-one (CAS 1312337-48-6); 5-(acetyloxy)-4-(2',4'-dichloro-4-cyclopropyl-[1,1'-biphenyl]-3-yl)-3,6-dihy¬dro-2,2,6,6-tetramethyl-2H-pyran-3-one; 5-(acetyloxy)-4-(4'-chloro-4-ethyl-2'-fluoro[1,1'-bi¬phenyl]-3-yl)-3,6-dihydro-2,2,6,6-tetramethyl-2H-pyran-3-one (CAS 1312340-82-1); 5-(acet¬yloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-chloro-4-cyclopropyl-2'-fluoro[1,1'-biphenyl]-3-yl)-5,6-di¬hydro-2,2,6,6-tetramethyl-5-oxo-2H-pyran-3-yl carbonic acid methyl ester (CAS 1312337-51-1); 4-(2',4'-dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-chloro-4-ethyl-2'-fluoro[1,1'-biphenyl]-3 yl)-5,6-dihydro-2,2,6,6-tetramethyl-5-oxo-2H-pyran-3-yl carbonic acid methyl ester (CAS 1312340-83-2); 4-(2',4'-dichloro-4-ethyl¬[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
   N.2 ALS inhibitors: amidosulfuron, azimsulfuron, bensulfuron, bensul¬furon-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensul¬furon-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosul¬furon, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl, tritosulfuron, imazametha¬benz, imazamethabenz-methyl, imazamox, imazapic, imazapyr, imazaquin, imazethapyr; cloransulam, cloransulam-methyl, diclosulam, flumetsulam, florasulam, metosulam, penoxsulam, pyrimisulfan and pyroxsulam; bispyribac, bispyribac-sodium, pyribenzoxim, pyriftalid, pyriminobac, pyriminobac-methyl, pyrithiobac, pyrithiobac-sodium, 4-[[[2-[(4,6-di-methoxy-2-pyrimidinyl)oxy]phenyl]methyl]amino]-benzoic acid-1-methyl¬ethyl ester (CAS 420138-41-6), 4-[[[2-[(4,6-dimethoxy-2-pyrimidinyl)oxy]phenyl]¬methyl]amino]-benzoic acid propyl ester (CAS 420138-40-5), N-(4-bromophenyl)-2-[(4,6-dimethoxy-2-pyrimidinyl)oxy]-benzenemethanamine (CAS 420138-01-8); flucarbazone, flucarbazone-sodium, propoxy-carbazone, propoxycarbazone-sodium, thiencarbazone, thiencarbazone-methyl; triafamone;
   N.3 Photosynthesis inhibitors: amicarbazone; chlorotriazine; ametryn, atrazine, chloridazone, cyanazine, desmetryn, dimethametryn,hexazinone, metribuzin, prometon, prometryn, propazine, simazine, simetryn, terbumeton, terbuthylazin, terbutryn, trietazin; chlorobrom¬uron, chlorotoluron, chloroxuron, dimefuron, diuron, fluometuron, isoproturon, isouron, linuron, metamitron, methabenzthiazuron, metobenzuron, metoxuron, monolinuron, neburon, sidu¬ron, tebuthiuron, thiadiazuron, desmedipham, karbutilat, phenmedipham, phenmedipham-ethyl, bromofenoxim, bromoxynil and its salts and esters, ioxynil and its salts and esters, bromacil, lenacil, terbacil, bentazon, bentazon-sodium, pyridate, pyridafol, pentanochlor, propanil; diquat, diquat-dibromide, paraquat, paraquat-dichloride, paraquat-dimetilsulfate;
   N.4 protoporphyrinogen-IX oxidase inhibitors: acifluorfen, acifluorfen-sodium, azafenidin, bencarbazone, benzfendizone, bifenox, butafenacil, carfentrazone, carfentrazone-ethyl, chlormethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fome¬safen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chioro-4-fluoro-5-(1-methyl-6-trifluoromethyl-2,4-dioxo-1,2,3,4-te¬trahydropyrimidin 3-yl)phenoxy]-2-pyridyloxy]acetate (CAS 353292-31-6), N-ethyl-3-(2,6-di¬chloro-4-trifluoromethylphenoxy)-5-methyl-1H-pyrazole-1-carboxamide (CAS 452098-92-9), N tetrahydrofurfuryl-3-(2,6-dichloro-4-trifluoromethylphenoxy)-5-methyl-1H-pyrazole-1-car-boxamide (CAS 915396-43-9), N-ethyl-3-(2-chloro-6-fluoro-4-trifluoromethyl¬phenoxy)-5 methyl-1H-pyrazole-1-carboxamide (CAS 452099-05-7), N tetrahydro¬furfuryl-3-(2-chloro-6-fluoro-4-trifluoro¬methylphenoxy)-5-methyl-1H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6 thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoro¬methyl-3-(2,2,7-tri-fluoro-3-oxo-4-prop-2-ynyl-3,4-dihydro-2H-benzo[1,4]oxazin-6-yl)-1H-pyrimidine-2,4-dione (CAS 1304113-05-0), methyl (E)-4-[2-chloro-5 [4-chloro-5-(difluoromethoxy)-1H-methyl-pyrazol-3-yl]-4-fluoro-phenoxy]-3-methoxy-but-2 enoate (CAS 948893-00-3), 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);
   N.5 Bleacher herbicides: beflubutamid, diflufenican, fluridone, flurochloridone, flurtamone, norflurazon, picolinafen, 4-(3-trifluoromethyl¬phenoxy)-2-(4-trifluoromethylphen¬yl)¬pyrimidine (CAS 180608-33-7); benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone; aclonifen, amitrole, flumeturon;
   N.6 EPSP synthase inhibitors: glyphosate, glyphosate-isopropylammonium, glyposate-potassium, glyphosate-trimesium (sulfosate);
   N.7 Glutamine synthase inhibitors: bilanaphos (bialaphos), bilanaphos-sodium, glufosinate, glufosinate-P, glufosinate-ammonium;
   N.8 DHP synthase inhibitors: asulam;
   N.9 Mitosis inhibitors: benfluralin, butralin, dinitramine, ethalfluralin, fluchloralin, oryzalin, pendimethalin, prodiamine, trifluralin; amiprophos, amiprophos-methyl, butamiphos; chlorthal, chlorthal-dimethyl, dithiopyr, thiazopyr, propyzamide, tebutam; carbetamide, chlorpropham, flamprop, flamprop-isopropyl, flamprop-methyl, flamprop-M-isopropyl, flamprop-M-methyl, propham;
   N.10 VLCFA inhibitors: acetochlor, alachlor, butachlor, dimethachlor, dimethenamid, dimethenamid-P, metazachlor, metolachlor, metolachlor-S, pethoxamid, pretilachlor, propachlor, propisochlor, thenylchlor, flufenacet, mefenacet, diphenamid, naproanilide, napropamide, napropamide-M, fentrazamide, anilofos, cafenstrole, fenoxasulfone, ipfencarbazone, piperophos, pyroxasulfone, isoxazoline compounds of the formulae II.1, II.2, II.3, II.4, II.5, II.6, II.7, II.8 and II.9 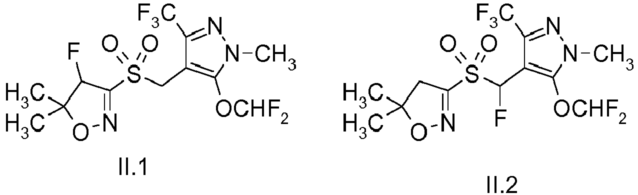 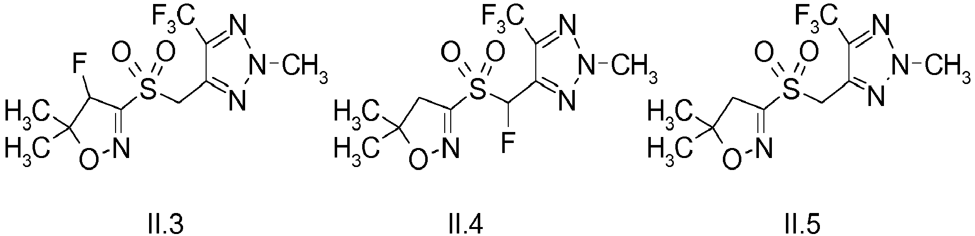 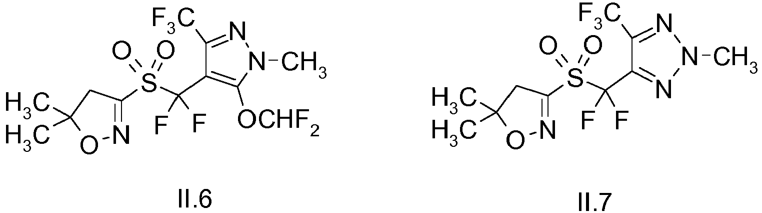 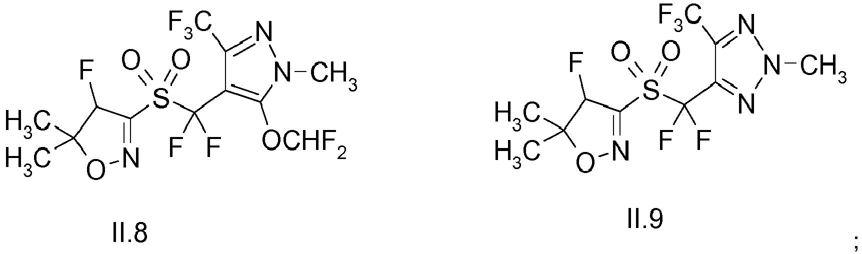
   N.11 Cellulose biosynthesis inhibitors: chlorthiamid, dichlobenil, flupoxam, indaziflam, isoxaben, triaziflam, 1-cyclohexyl-5-pentafluorphenyloxy-14-[1,2,4,6]thiatriazin-3-ylamine (CAS 175899-01-1);
   N.12 Decoupler herbicides: dinoseb, dinoterb, DNOC and its salts;
   N.13 Auxinic herbicides: 2,4-D and its salts and esters, clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxy¬pyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quin¬merac, TBA (2,3,6) and its salts and esters, triclopyr and its salts and esters, 4-amino-3 chloro-6-(4-chloro-2-fluoro-3-methoxyphenyl)-5-fluoropyridine-2-carboxylic acid, benzyl 4 amino-3-chloro-6-(4-chloro-2-fluoro-3-methoxyphenyl)-5-fluoropyridine-2-carboxylate (CAS 1390661-72-9);
   N.14 Auxin transport inhibitors: diflufenzopyr, diflufenzopyr-sodium, naptalam and naptalam-sodium;
   N.15 Other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, tridiphane;
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxyde-meton-methyl, parathion, parathion-methyl, phenthoate, phorate, phosa¬lone, phosmet, phosphamidon, phoxim, pirimiphos- methyl, profenofos, propetamphos, pro¬thiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alphacypermethrin, beta-cypermethrin, theta-cypermethrin, zeta-cypermethrin, cyphenothrin, deltamethrin, empenthrin, esfenvalerate, etofenprox, fenpropathrin, fenvalerate, flucy¬thrinate, flumethrin, tau-fluvalinate, halfenprox, heptafluthrin, imiprothrin, meperfluthrin, metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin and transfluthrin; DDT, methoxychlor;
   O.4 Nicotinic acetylcholine receptor agonists (nAChR): acetamiprid, clothianidin, cycloxaprid, dinotefuran, imidacloprid, nitenpyram, thiacloprid, thiamethoxam; 4,5-dihydro-N-nitro-1 (2 oxiranylmethyl)-1 H-imidazol-2-amine, (2E)-1-[(6-chloropyridin-3-yl)methyl]-N'-nitro-2-pentylidene¬hydrazinecarboximidamide; 1-[(6-chloropyridin-3-yl)methyl]-7-methyl-8-nitro-5-propoxy-1,2,3,5,6,7-hexahydroimidazo[1,2-a]pyridine ; nicotine; sulfoxaflor, flupyradifurone, triflumezopyrim;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen; O.8 miscellaneous non-specific (multi-site) inhibitors: methyl bromide and other alkyl halides; chloropicrin, sulfuryl fluoride, borax, tartar emetic;
   O.9 Chordotonal organ TRPV channel modulators: pymetrozine, pyrifluquinazon; flonicamid;
   O.10 Mite growth inhibitors: clofentezine, hexythiazox, diflovidazin; etoxazole;
   O.11 Microbial disruptors of insect midgut membranes: Bacillus thuringiensis, Bacillus sphaericus and the insecticdal proteins they produce: Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis, the Bt crop proteins: Cry1Ab, Cry1Ac, Cry1Fa, Cry2Ab, mCry3A, Cry3Ab, Cry3Bb, Cry34/35Ab1;
   0.12 Inhibitors of mitochondrial ATP synthase: diafenthiuron; azocyclotin, cyhexatin, fenbutatin oxide, propargite, tetradifon;
   0.13 Uncouplers of oxidative phosphorylation via disruption of the proton gradient: chlorfenapyr, DNOC, sulfluramid;
   0.14 Nicotinic acetylcholine receptor (nAChR) channel blockers: bensultap, cartap hydrochloride, thiocyclam, thiosultap sodium;
   0.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
   0.16 Inhibitors of the chitin biosynthesis type 1: buprofezin;
   0.17 Moulting disruptors: cyromazine;
   0.18 Ecdyson receptor agonists: methoxyfenozide, tebufenozide, halofenozide, fufenozide, chromafenozide;
   0.19 Octopamin receptor agonists: amitraz;
   O.20 Mitochondrial complex III electron transport inhibitors: hydramethylnon, acequinocyl, fluacrypyrim, bifenazate;
   0.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cy¬anophenyl)-1-[3-(trifluoromethyl)phenyl]¬ethylidene]-N-[4-(difluoromethoxy)phenyl]-hydra¬zine-carboxamide, N-(3-chloro-2-methylphenyl)-2-[(4-chlorophenyl)-[4[meth¬yl(methylsulfo¬nyl)-amino]phenyl]¬methylene]-hydrazinecarboxamide;
   O.23 Inhibitors of the of acetyl CoA carboxylase: spirodiclofen, spiromesifen, spirotetramat, spiropidion;
   O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen;
   O.26 Ryanodine receptor-modulators: flubendiamide, chlorantraniliprole, cyantraniliprole, cyclaniliprole, tetraniliprole; (R)-3-chloro-N1-{2-methyl-4-[1,2,2,2-tetrafluoro-1-(trifluoromethyl)¬ethyl]phenyl}-N2-(1-methyl-2-methylsulfonylethyl)phthalamide, (S)-3-chloro-N1-{2-methyl-4-[1,2,2,2-te¬trafluoro-1-(trifluoromethyl)ethyl]phenyl}-N2-(1-methyl-2-methylsulfonylethyl)-phthalamide, methyl-2-[3,5-dibromo-2-({[3-bromo-1-(3-chloropyridin-2-yl)-1H-pyrazol-5-yl]-carbonyl}¬amino)benzoyl]-1,2-dimethylhydrazinecarboxylate; N-[4,6-dichloro-2-[(diethyl-lambda-4-sulfanylidene)¬carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide ; N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; N-[4,6-dichloro-2-[(di-2-propyl-lambda-4-sulfanylidene)¬carba¬moyl]-phenyl]-2-(3-chloro-2-pyridyl)-5-(trifluoromethyl)pyrazole-3-carboxamide; N-[4,6-di¬bromo-2-[(diethyl-lambda-4-sulfanylidene)carbamoyl]-phenyl]-2-(3-chloro-2-pyridyl)-5-(tri¬fluoromethyl)pyrazole-3-carboxamide; N-[2-(5-amino-1,3,4-thiadiazol-2-yl)-4-chloro-6-meth¬ylphenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; 3-bromo-N-[2,4-dichloro-6-(methylcarbamoyl)phenyl]-1-(3,5-dichloro-2-pyri¬dyl)-1H-pyrazole-5-carboxamide; N-[4-chloro-2-[[(1,1-dimethylethyl)amino]carbonyl]-6-meth¬ylphenyl]-1-(3-chloro-2-pyridinyl)-3-(fluoromethoxy)-1H-pyrazole-5-carboxamide; cyhalodi¬amide ;
   O.27: Chordotonal organ Modulators - undefined target site: flonicamid;
   O.28. insecticidal active compounds of unknown or uncertain mode of action: afidopyropen , afoxolaner, azadirachtin, amidoflumet, benzoximate, broflanilide, bromopropy¬late, chino¬methionat, cryolite, dicloromezotiaz , dicofol, flufenerim, flometoquin, fluensulfone, fluhexafon, fluopyram, fluralaner, metoxadiazone, piperonyl butoxide, pyflu¬bumide, pyridalyl, tioxazafen, 11 (4-chloro-2,6 dimethylphenyl)-12-hydroxy-1,4-dioxa-9-azadispiro[4.2.4.2]-tetradec-11-en-10 one, 3(4'-fluoro-2,4-dimethylbiphenyl-3-yl)-4-hydroxy-8-oxa-1-azaspiro[4.5]dec-3-en-2-one , 1[2 fluoro-4-methyl-5-[(2,2,2-trifluoroethyl)sulfinyl]phenyl]-3-(trifluoromethyl)-1H-1,2,4-tri¬azole-5-amine, Bacillus firmus I-1582; flupyrimin; fluazaindolizine ; 4 [5-(3,5-dichlorophenyl)-5-(tri-fluoromethyl)-4H-isoxazol-3-yl]-2-methyl-N-(1-oxothietan-3-yl)benzamide ; fluxamet-amide ; 5 [3 [2,6-dichloro-4-(3,3-dichloroallyloxy)phenoxy]propoxy]-1H-pyrazole; 3-(benzoyl¬methyl¬amino)-N-[2-bromo-4-[1,2,2,3,3,3-hexafluoro-1-(trifluoromethyl)propyl]-6-(trifluoro-methyl)phe¬nyl]-2-fluoro-benzamide; 3-(benzoylmethylamino)-2-fluoro-N-[2-iodo-4 [1,2,2,2 te-trafluoro-1 (trifluoromethyl)ethyl]-6-(trifluoromethyl)phenyl]-benzamide; N[3[[[2 iodo-4[1,2,2,2-tetra¬fluoro-1-(trifluoromethyl)ethyl]-6-(trifluoromethyl)phenyl]amino]¬carbonyl]¬phenyl]-N-methyl-benzamide; N-[3-[[[2-bromo-4-[1,2,2,2-tetrafluoro-1-(trifluoro¬methyl)ethyl]-6-(trifluoromethyl)-phenyl]amino]carbonyl]-2-fluorophenyl]-4-fluoro-N-methyl-benzamide; 4 fluoro-N-[2-fluoro-3[[[2 iodo-4-[1,2,2,2-tetrafluoro-1-(trifluoromethyl)¬ethyl]-6(trifluoro¬methyl)phenyl]amino]carbo¬nyl]-phenyl]-N-methyl-benzamide; 3-fluoro-N-[2-fluoro-3-[[[2-iodo-4-[1,2,2,2-tetrafluoro-1-(tri-fluoromethyl)¬ethyl]-6¬(trifluoromethyl)phenyl]amino]¬carbonyl]phen¬yl]-N-methyl-benzamide; 2 chloro-N-[3-[[[2-iodo-4-[1,2,2,2-tetra¬fluoro-1-(tri¬fluoromethyl)¬ethyl]-6-(trifluoromethyl)phenyl]-amino]carbonyl]phenyl]-3-pyridine¬carboxamide; 4-cyano-N[2-cyano-5-[[2,6-dibromo-4 [1,2,2,3,3,3-hexafluoro-1-(trifluorometh¬yl)propyl]phen¬yl]¬carba¬moyl]phenyl]-2-methyl-benzamide; 4-cyano-3-[(4-cyano-2-methyl-benzoyl)amino]-N[2,6-di¬chloro-4-[1,2,2,3,3,3-he¬xafluoro-1-(trifluoromethyl)¬propyl]phenyl]-2-fluoro-benzamide; N[5[[2-chloro-6-cyano-4 [1,2,2,3,3,3-hexafluoro-1-(trifluoromethyl)propyl]¬phenyl]carba¬moyl]-2-cyano-phenyl]-4-cyano-2-methyl-benzamide; N-[5-[[2-bromo-6-chloro-4-[2,2,2-tri¬fluoro-1-hydroxy-1-(trifluoromethyl)ethyl]phenyl]carbamoyl]-2-cyano-phenyl]-4-cy¬ano-2 methyl-benzamide; N-[5-[[2-bromo-6-chloro-4-[1,2,2,3,3,3-hexafluoro-1-(trifluoro¬methyl)¬propyl]phenyl]carbamoyl]-2-cyanophenyl]-4-cyano-2-methyl-benzamide; 4-cyano-N[2-cy¬ano-5-[[2,6-dichloro-4-[1,2,2,3,3,3-hexafluoro-1-(trifluoromethyl)¬propyl]phenyl]¬carbamoyl]¬phenyl]-2-methyl-benzamide; 4-cyano-N-[2-cyano-5-[[2,6-dichloro-4-[1,2,2,2-te¬trafluoro-1(trifluoromethyl)ethyl]phenyl]carbamoyl]¬phenyl]-2-methyl-benzamide; N [5 [[2 bromo-6 chloro-4-[1,2,2,2-tetrafluoro-1-(trifluoromethyl)ethyl]phenyl]carbamoyl]-2 cyano-phenyl]-4 cyano-2-methyl-benzamide; 2-(1,3-dioxan-2-yl)-6-[2-(3-pyridinyl)-5 thiazolyl]-pyridine; 2 [6 [2-(5-fluoro-3-pyridinyl)-5-thiazolyl]-2-pyridinyl]-pyrimidine; 2[6[2(3-pyridinyl)-5-thi¬azolyl]-2-pyridinyl]-pyrimidine; N-methylsulfonyl-6-[2-(3-pyridyl)thiazol-5-yl]pyri¬dine-2-car¬boxamide; N-methylsulfonyl-6-[2-(3-pyridyl)thiazol-5-yl]pyridine-2-carboxamide ; N-ethyl-N[4methyl-2-(3-pyridyl)thiazol-5-yl]-3-methylthio-propanamide; N-methyl-N-[4-methyl-2 (3 pyridyl)thiazol-5-yl]-3-methylthio-propanamide; N,2-dimethyl-N-[4-methyl-2-(3-pyridyl)¬thiazol-5-yl]-3-methylthio-propanamide; N-ethyl-2-methyl-N-[4-methyl-2-(3-pyridyl)thiazol-5yl]-3-methylthio-propanamide; N-[4-chloro-2-(3-pyridyl)thiazol-5-yl]-N-ethyl-2-methyl-3 methylthio-propanamide; N-[4-chloro-2-(3-pyridyl)thiazol-5-yl]-N,2-dimethyl-3-methylthio-propanamide; N-[4-chloro-2-(3-pyridyl)thiazol-5-yl]-N-methyl-3-methylthio-propanamide; N[4-chloro-2-(3-pyridyl)thiazol-5-yl]-N-ethyl-3-methylthio-propanamide ; 1-[(6-chloro-3-py¬ri¬dinyl)methyl]-1,2,3,5,6,7-hexahydro-5-methoxy-7-methyl-8-nitro-imidazo[1,2-a]pyridine; 1 [(6-chloropyridin-3-yl)methyl]-7-methyl-8-nitro-1,2,3,5,6,7-hexahydroimidazo[1,2-a]pyridin-5-ol; 1-isopropyl-N,5-dimethyl-N-pyridazin-4-yl-pyrazole-4-carboxamide; 1-(1,2-dimeth¬yl¬propyl)-N-ethyl-5-methyl-N-pyridazin-4-yl-pyrazole-4-carboxamide; N,5-dimethyl-N-pyridazin-4-yl-1-(2,2,2-trifluoro-1-methyl-ethyl)pyrazole-4-carboxamide; 1-[1-(1-cyano¬cyclopropyl)¬eth¬yl]-N-ethyl-5-methyl-N-pyridazin-4-yl-pyrazole-4-carboxamide; N-ethyl-1(2 fluoro-1-methyl-propyl)-5-methyl-N-pyridazin-4-yl-pyrazole-4-carboxamide; 1-(1,2-dimethylpropyl)-N,5-di¬methyl-N-pyridazin-4-yl-pyrazole-4-carboxamide; 1-[1-(1-cyanocyclopropyl)¬ethyl]-N,5-di¬methyl-N-pyridazin-4-yl-pyrazole-4-carboxamide; N-methyl-1-(2-flu¬oro-1-methyl-propyl]-5methyl-N-pyridazin-4-yl-pyrazole-4-carboxamide; 1-(4,4-difluorocyclohexyl)-N ethyl-5 methyl-N-pyridazin-4-yl-pyrazole-4-carboxamide; 1-(4,4-difluorocyclohexyl)-N,5-di¬methyl-N-pyridazin-4-yl-pyrazole-4-carboxamide, N-(1-methylethyl)-2-(3-pyridinyl)-2H-inda¬zole-4 carboxamide; N-cyclopropyl-2-(3-pyridinyl)-2H-indazole-4-carboxamide; N-cyclohexyl-2 (3 pyridinyl)-2H-indazole-4-carboxamide; 2-(3-pyridinyl)-N-(2,2,2-trifluoroethyl)-2H-inda¬zole-4-carboxamide; 2-(3-pyridinyl)-N-[(tetrahydro-2-furanyl)methyl]-2H-indazole-5-carbox¬amide; methyl 2-[[2-(3-pyridinyl)-2H-indazol-5-yl]carbonyl]hydrazinecarboxylate; N[(2,2-di¬fluorocyclopropyl)methyl]-2-(3-pyridinyl)-2H-indazole-5-carboxamide; N-(2,2-di¬flu¬oropropyl)-2-(3-pyridinyl)-2H-indazole-5-carboxamide; 2-(3-pyridinyl)-N-(2-pyrimi¬dinylmethyl)-2H-in¬dazole-5-carboxamide; N-[(5-methyl-2-pyrazinyl)methyl]-2(3pyridinyl)-2H-indazole-5-car¬boxamide, tyclopyrazoflor; N-[3-chloro-1-(3-pyridyl)pyrazol-4-yl]-N-ethyl-3(3,3,3-trifluoropropylsulfinyl)¬propanamide; N-[3-chloro-1-(3-pyridyl)pyrazol-4-yl]-3-[(2,2-di¬fluorocyclopropyl)methy¬sulfanyl]-N-ethyl-propanamide; N-[3-chloro-1-(3-pyridyl)pyrazol-4yl]-3-[(2,2-difluorocyclo¬propyl)methylsulfinyl]-N-ethyl-propanamide; sarolaner, lotilaner, N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide ; M.UN.22a 2-(3-ethylsulfonyl-2pyridyl)-3-methyl-6-(trifluoromethyl)imidazo[4,5-b]pyridine, 2-[3-ethylsulfonyl-5-(trifluorometh¬yl)-2-pyridyl]-3-methyl-6-(trifluoromethyl)imidazo[4,5-b]pyridine ,4-[5-(3,5-dichlorophenyl)-5-(tri¬fluoromethyl)-4H-isoxazol-3-yl]-N-[(4R)-2-ethyl-3-oxo-isoxazolidin-4-yl]-2-methyl-benzamide, 4 [5-(3,5-dichloro-4-fluoro-phenyl)-5-(trifluoromethyl)-4H-isoxazol-3-yl]-N-[(4R)-2-ethyl-3-oxo-isoxazolidin-4-yl]-2-methyl-benzamide ; N-[4-chloro-3-(cyclopropylcarbamoyl)phenyl]-2-methyl-5 (1,1,2,2,2-pentafluoroethyl)-4-(trifluoromethyl)pyrazole-3-carboxamide, N-[4-chloro-3-[(1-cy-anocyclopropyl)carbamoyl]phenyl]-2-methyl-5-(1,1,2,2,2-pentafluoroethyl)-4-(trifluoro¬methyl)-pyrazole-3-carboxamide ; acynonapyr ; benzpyrimoxan ; chloro-N-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide .

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP A 141 317; EP-A 152 031; EP-A 226 917; EP A 243 970; EP A 256 503; EP-A 428 941; EP A 532 022; EP-A 1 028 125; EP-A 1 035 122; EP A 1 201 648; EP A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441).

The present invention furthermore relates to agrochemical compositions comprising a mixture of at least one compound of formula I (compound) and at least one further active substance useful for plant protection, e. g. selected from the groups A) to O) (com¬ponent 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds of formula (I) and at least one fungicide from groups A) to K), as described above, is more efficient than combating those fungi with individual compounds of formula (I) or individual fungicides from groups A) to K).

By applying compounds of formula (I) together with at least one active substance from groups A) to O) a synergistic effect can be obtained, i.e. more then simple addition of the individual effects is obtained (synergistic mixtures).

This can be obtained by applying the compounds of formula (I) and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound of formula (I) and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day. In case of a mixture comprising a pesticide II selected from group L), it is preferred that the pesticide II is applied as last treatment.

According to the invention, the solid material (dry matter) of the biopesticides (with the exception of oils such as Neem oil) are considered as active components (e. g. to be obtained after drying or evaporation of the extraction or suspension medium in case of liquid formulations of the microbial pesticides).

In accordance with the present invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 x 1010 CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as Steinernema feltiae.

In the binary mixtures and compositions according to the invention the weight ratio of the component 1) and the component 2) generally depends from the properties of the active components used, usually it is in the range of from 1:10,000 to 10,000:1, often it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to further embodiments of the mixtures and compositions, the weight ratio of the component 1) and the component 2) usually is in the range of from 20,000:1 to 1:10, often in the range of from 10,000:1 to 1:1, regularly in the range of from 5,000:1 to 5:1, preferably in the range of from 5,000:1 to 10:1, more preferably in the range of from 2,000:1 to 30:1, even more preferably in the range of from 2,000:1 to 100:1 and in particular in the range of from 1,000:1 to 100:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

According to further embodiments of the mixtures and compositions, the weight ratio of the component 1) and the component 2) usually is in the range of from 10:1 to 1:20,000, often in the range of from 1:1 to 1:10,000, regularly in the range of from 1:5 to 1:5,000, preferably in the range of from 1:10 to 1:5,000, more preferably in the range of from 1:30 to 1:2,000, even more preferably in the range of from 1:100 to 1:2,000 to and in particular in the range of from 1:100 to 1:1,000.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

When mixtures comprising microbial pesticides are employed in crop protection, the application rates preferably range from about 1 x 106 to 5 x 1016 (or more) CFU/ha, preferably from about 1 x 108 to about 1 x 1013 CFU/ha, and even more preferably from about 1 x 109 to 5 x 1015 CFU/ha and particularly preferred even more preferably from 1 x 1012 to 5 x 1014 CFU/ha. In the case of (entomopathogenic) nematodes as microbial pesticides (e. g. Steinernema feltiae), the application rates preferably range inform about 1 x 105 to 1 x 1012 (or more), more preferably from 1 x 108 to 1 x 1011, even more preferably from 5 x 108 to 1 x 1010 individuals (e. g. in the form of eggs, juvenile or any other live stages, preferably in an infective juvenile stage) per ha.

When mixtures comprising, microbial pesticides are employed in seed treatment, the application rates with respect to plant propagation material preferably range from about 1 x 106 to 1 x 1012 (or more) CFU/seed. Preferably, the concentration is about 1 x 106 to about 1 x 109 CFU/seed. In the case of the microbial pesticides II, the application rates with respect to plant propagation material also preferably range from about 1 x 107 to 1 x 1014 (or more) CFU per 100 kg of seed, preferably from 1 x 109 to about 1 x 1012 CFU per 100 kg of seed.

Preference is also given to mixtures comprising as component 2) at least one active substance selected from inhibitors of complex III at Qo site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qi site in group A), more preferably selected from compounds (A.2.1), (A.2.3) and (A.2.4); particularly selected from (A.2.3) and (A.2.4).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.34), (B.1.37), (B.1.38), (B.1.43) and (B.1.46); particularly selected from (B.1.5), (B.1.8), (B.1.10), (B.1.17), (B.1.22), (B.1.23), (B.1.25), (B.1.33), (B.1.34), (B.1.37), (B.138), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5). Preference is also given to mixtures comprising as component 2) at least one active substance selected from group G), more preferably selected from compounds (G.3.1), (G.3.3), (G.3.6), (G.5.1), (G.5.2), (G.5.3), (G.5.4), (G.5.5), G.5.6), G.5.7), (G.5.8), (G.5.9), (G.5.10) and (G.5.11); particularly selected from (G.3.1), (G.5.1), (G.5.2) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (1.2.2) and (1.2.5). Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.45), (K.1.47) and (K.1.49); particularly selected from (K.1.41), (K.1.44), (K.1.45), (K.1.47) and (K.1.49).

The biopesticides from group L1) and/or L2) may also have insecticidal, acaricidal, molluscidal, pheromone, nematocidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematocidal activity.
1) Many of these biopesticides have been deposited under deposition numbers mentioned herein (the prefixes such as ATCC or DSM refer to the acronym of the respective culture collection, for details see e. g. here: http://www.wfcc.info/ccinfo/collection/by_acronym/), are referred to in literature, registered and/or are commercially available: mixtures of Aureobasidium pullulans DSM 14940 and DSM 14941 isolated in 1989 in Konstanz, Germany (e. g. blastospores in BlossomProtect® from bio-ferm GmbH, Austria), Azospirillum brasilense Sp245 originally isolated in wheat reagion of South Brazil (Passo Fundo) at least prior to 1980 (BR 11005; e. g. GELFIX® Gramíneas from BASF Agricultural Specialties Ltd., Brazil), A. brasilense strains Ab-V5 and Ab-V6 (e. g. in AzoMax from Novozymes BioAg Produtos papra Agricultura Ltda., Quattro Barras, Brazil or Simbiose-Maíz® from Simbiose-Agro, Brazil; Plant Soil 331, 413-425, 2010), Bacillus amyloliquefaciens strain AP-188 (NRRL B-50615 and B-50331; US 8,445,255); B. amyloliquefaciens spp. plantarum D747 isolated from air in Kikugawa-shi, Japan (US 20130236522 A1; FERM BP 8234; e. g. Double Nickel™ 55 WDG from Certis LLC, USA), B. amyloliquefaciens spp. plantarum FZB24 isolated from soil in Brandenburg, Germany (also called SB3615; DSM 96-2; J. Plant Dis. Prot. 105, 181-197, 1998; e. g. Taegro®from Novozyme Biologicals, Inc., USA), B. amyloliquefaciens ssp. plantarum FZB42 isolated from soil in Brandenburg, Germany (DSM 23117; J. Plant Dis. Prot. 105, 181-197, 1998; e. g. RhizoVital® 42 from AbiTEP GmbH, Germany), B. amyloliquefaciens ssp. plantarum MBI600 isolated from faba bean in Sutton Bonington, Nottinghamshire, U.K. at least before 1988 (also called 1430; NRRL B 50595; US 2012/0149571 A1; e. g. Integral® from BASF Corp., USA), B. amyloliquefaciens spp. plantarum QST-713 isolated from peach orchard in 1995 in California, U.S.A. (NRRL B 21661; e. g. Serenade® MAX from Bayer Crop Science LP, USA), B. amyloliquefaciens spp. plantarum TJ1000 isolated in 1992 in South Dakoda, U.S.A. (also called 1BE; ATCC BAA-390; CA 2471555 A1; e. g. QuickRoots™ from TJ Technologies, Watertown, SD, USA), B. firmus CNCM I-1582, a variant of parental strain EIP-N1 (CNCM I-1556) isolated from soil of central plain area of Israel (WO 2009/126473, US 6,406,690; e. g. Votivo® from Bayer CropScience LP, USA), B. pumilus GHA 180 isolated from apple tree rhizosphere in Mexico (IDAC 260707-01; e. g. PRO-MIX® BX from Premier Horticulture, Quebec, Canada), B. pumilus INR-7 otherwise referred to as BU F22 and BU-F33 isolated at least before 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e. g. Sonata® or Ballad® Plus from Bayer Crop Science LP, USA), B. simplex ABU 288 (NRRL B-50304; US 8,445,255), B. subtilis FB17 also called UD 1022 or UD10-22 isolated from red beet roots in North America (ATCC PTA-11857; System. Appl. Microbiol. 27, 372-379, 2004; US 2010/0260735; WO 2011/109395); B. thurin¬giensis ssp. aizawai ABTS-1857 isolated from soil taken from a lawn in Ephraim, Wisconsin, U.S.A., in 1987 (also called ABG 6346; ATCC SD-1372; e. g. XenTari® from BioFa AG, Münsingen, Germany), B. t. ssp. kurstaki ABTS-351 identical to HD-1 isolated in 1967 from diseased Pink Bollworm black larvae in Brownsville, Texas, U.S.A. (ATCC SD-1275; e. g. Dipel® DF from Valent BioSciences, IL, USA), B. t. ssp. kurstaki SB4 isolated from E. saccharina larval cadavers (NRRL B-50753; e. g. Beta Pro® from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e. g. Novodor® from Valent BioSciences, Switzerland), Beauveria bassiana GHA (ATCC 74250; e. g. BotaniGard® 22WGP from Laverlam Int. Corp., USA), B. bassiana JW-1 (ATCC 74040; e. g. Naturalis® from CBC (Europe) S.r.l., Italy), B. bassiana PPRI 5339 isolated from the larva of the tortoise beetle Conchyloctenia punctata (NRRL 50757; e. g. BroadBand® from BASF Agricultural Specialities (Pty) Ltd., South Africa), Brady-rhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e. g. in Rhizoflo®, Histick®, Hicoat® Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e. g. Contans® WG, Intercept® WG from Bayer CropScience AG, Germany), harpin (alpha-beta) protein (Science 257, 85-88, 1992; e. g. Messenger™ or HARP-N Tek from Plant Health Care plc, U.K.), Helicoverpa armigera nucleopolyhedrovirus (HearNPV) (J. Invertebrate Pathol. 107, 112-126, 2011; e. g. Helicovex® from Adermatt Biocontrol, Swit¬zerland; Diplomata® from Koppert, Brazil; Vivus® Max from AgBiTech Pty Ltd., Queensland, Australia), Helicoverpa zea single capsid nucleopolyhedrovirus (HzSNPV) (e. g. Gemstar® from Certis LLC, USA), Helicoverpa zea nucleopolyhedrovirus ABA-NPV-U (e. g. Heligen® from AgBiTech Pty Ltd., Queensland, Australia), Heterorhabditis bacteriophora (e. g. Nemasys® G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e. g. PFR-97™ or PreFeRal® from Certis LLC, USA), Metarhizium anisopliae var. anisopliae F52 also called 275 or V275 isolated from codling moth in Austria (DSM 3884, ATCC 90448; e. g. Met52® Novozymes Biologicals BioAg Group, Canada), Metschnikowia fructicola 277 isolated from grapes in the central part of Israel (US 6,994,849; NRRL Y-30752; e. g. formerly Shemer® from Agrogreen, Israel), Paecilomyces ilacinus 251 isolated from infected nematode eggs in the Philippines (AGAL 89/030550; WO1991/02051; Crop Protection 27, 352-361, 2008; e. g. BioAct® from Bayer CropScience AG, Germany and MeloCon® from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva™ PN from Syngenta Crop Protection, LLC, USA), Penicillium bilaiae (also called P. bilaii) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), Reynoutria sachalinensis extract (EP 0307510 B1; e. g. Regalia® SC from Marrone Biolnnovations, Davis, CA, USA or Milsana® from BioFa AG, Germany), Steinernema carpocapsae (e. g. Millenium® from BASF Agricultural Specialities Limited, UK), S. feltiae (e. g. Nemashield® from BioWorks, Inc., USA; Nemasys® from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e. g. Trichoplus® from BASF Agricultural Specialities (Pty) Ltd., South Africa), T. harzianum T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA).

According to one embodiment of the inventive mixtures, the at least one pesticide II is selected from the groups L1) to L5):
L1) Microbial pesticides with fungicidal, bactericidal, viricidal and/or plant defense activator activity: Aureobasidium pullulans DSM 14940 and DSM 14941 (L1.1), Bacillus amyloliquefaciens AP-188 (L.1.2), B. amyloliquefaciens ssp. plantarum D747 (L.1.3), B. amylo¬lique-faciens ssp. plantarum FZB24 (L.1.4), B. amyloliquefaciens ssp. plantarum FZB42 (L.1.5), B. amyloliquefaciens ssp. plantarum MBI600 (L.1.6), B. amyloliquefaciens ssp. plantarum QST-713 (L.1.7), B. amyloliquefaciens ssp. plantarum TJ1000 (L.1.8), B. pumilus GB34 (L.1.9), B. pumilus GHA 180 (L.1.10), B. pumilus INR-7 (L.1.11), B. pumilus KFP9F (L.1.12), B. pumilus QST 2808 (L.1.13), B. simplex ABU 288 (L.1.14), B. subtilis FB17 (L.1.15), Coniothyrium minitans CON/M/91-08 (L.1.16), Metschnikowia fructicola NRRL Y 30752 (L.1.17), Paenibacillus alvei NAS6G6 (L.1.18), P. epiphyticus Lu17015 (L.1.25), P. polymyxa ssp. plantarum Lu16774 (L.1.26), P. p. ssp. plantarum strain Lu17007 (L.1.27), Penicillium bilaiae ATCC 22348 (L.1.19), P. bilaiae ATCC 20851 (L.1.20), Penicillium bilaiae ATCC 18309 (L.1.21), Streptomyces microflavus NRRL B-50550 (L.1.22), Trichoderma asperelloides JM41R (L.1.23), T. harzianum T-22 (L.1.24);
L2) Biochemical pesticides with fungicidal, bactericidal, viricidal and/or plant defense activator activity: harpin protein (L.2.1), Reynoutria sachalinensis extract (L.2.2);
L3) Microbial pesticides with insecticidal, acaricidal, molluscidal and/or nematicidal activity: Bacillus firmus I 1582 (L.3.1); B. thuringiensis ssp. aizawai ABTS-1857 (L.3.2), B. t. ssp. kurstaki ABTS-351 (L.3.3), B. t. ssp. kurstaki SB4 (L.3.4), B. t. ssp. tenebrionis NB-176-1 (L.3.5), Beauveria bassiana GHA (L.3.6), B. bassiana JW-1 (L.3.7), B. bassiana PPRI 5339 (L.3.8), Burkholderia sp. A396 (L.3.9), Helicoverpa armigera nucleopolyhedrovirus (HearNPV) (L.3.10), Helicoverpa zea nucleopolyhedrovirus (HzNPV) ABA-NPV-U (L.3.11), Helicoverpa zea single capsid nucleopolyhedrovirus (HzSNPV) (L.3.12), Heterohabditis bacteriophora (L.3.13), Isaria fumosorosea Apopka-97 (L.3.14), Metarhizium anisopliae var. anisopliae F52 (L.3.15), Paecilomyces lilacinus 251 (L.3.16), Pasteuria nishizawae Pn1 (L.3.17), Steinernema carpocapsae (L.3.18), S. feltiae (L.3.19);
L4) Biochemical pesticides with insecticidal, acaricidal, molluscidal, pheromone and/or nematicidal activity: cis-jasmone (L.4.1), methyl jasmonate (L.4.2), Quillay extract (L.4.3);
L5) Microbial pesticides with plant stress reducing, plant growth regulator, plant growth promoting and/or yield enhancing activity: Azospirillum brasilense Ab-V5 and Ab-V6 (L.5.1), A. brasilense Sp245 (L.5.2), Bradyrhizobium elkanii SEMIA 587 (L.5.3), B. elkanii SEMIA 5019 (L.5.4), B. japonicum 532c (L.5.5), B. japonicum E-109 (L.5.6), B. japonicum SEMIA 5079 (L.5.7), B. japonicum SEMIA 5080 (L.5.8).

The present invention furthermore relates to agrochemical compositions comprising a mixture of compound of formula (I) (component 1) and at least one biopesticide selected from the group L) (component 2), in particular at least one biopesticide selected from the groups L1) and L2), as described above, and if desired at least one suitable auxiliary.

The present invention furthermore relates to agrochemical compositions comprising a mixture of compound of formula (I) (component 1) and at least one biopesticide selected from the group L) (component 2), in particular at least one biopesticide selected from the groups L3) and L4), as described above, and if desired at least one suitable auxiliary.

Preference is also given to mixtures comprising as pesticide II (component 2) a biopesticide selected from the groups L1), L3) and L5), preferably selected from strains denoted above as (L.1.2), (L.1.3), (L.1.4), (L.1.5), (L.1.6), (L.1.7), (L.1.8), (L.1.10), (L.1.11), (L.1.12), (L.1.13), (L.1.14), (L.1.15), (L.1.17), (L.1.18), (L.1.19), (L.1.20), (L.1.21), (L.1.25), (L.1.26), (L.1.27), (L.3.1); (L.3.9), (L.3.16), (L.3.17), (L.5.1), (L.5.2), (L.5.3), (L.5.4), (L.5.5), (L.5.6), (L.5.7), (L.5.8); (L.4.2), and (L.4.1); even more preferably selected from (L.1.2), (L.1.6), (L.1.7), (L.1.8), (L.1.11), (L.1.12), (L.1.13), (L.1.14), (L.1.15), (L.1.18), (L.1.19), (L.1.20), (L.1.21), (L.3.1); (L.3.9), (L.3.16), (L.3.17), (L.5.1), (L.5.2), (L.5.5), (L.5.6); (L.4.2), and (L.4.1). These mixtures are particularly suitable for treatment of propagation materials, i. e. seed treatment purposes and likewise for soil treatment. These seed treatment mixtures are particularly suitable for crops such as cereals, corn and leguminous plants such as soybean.

Preference is also given to mixtures comprising as pesticide II (component 2) a biopesticide selected from the groups L1), L3) and L5), preferably selected from strains denoted above as (L1.1), (L.1.2), (L.1.3), (L.1.6), (L.1.7), (L.1.9), (L.1.11), (L.1.12), (L.1.13), (L.1.14), (L.1.15), (L.1.17), (L.1.18), (L.1.22), (L.1.23), (L.1.24), (L.1.25), (L.1.26), (L.1.27), (L.2.2); (L.3.2), (L.3.3), (L.3.4), (L.3.5), (L.3.6), (L.3.7), (L.3.8), (L.3.10), (L.3.11), (L.3.12), (L.3.13), (L.3.14), (L.3.15), (L.3.18), (L.3.19); (L.4.2), even more preferably selected from (L.1.2), (L.1.7), (L.1.11), (L.1.13), (L.1.14), (L.1.15), (L.1.18), (L.1.23), (L.3.3), (L.3.4), (L.3.6), (L.3.7), (L.3.8), (L.3.10), (L.3.11), (L.3.12), (L.3.15), and (L.4.2). These mixtures are particularly suitable for foliar treatment. These mixtures for foliar treatment are particularly suitable for vegetables, fruits, vines, cereals, corn, leguminous crops such as soybeans.

The mixtures of active substances can be prepared as compositions comprising besides the active ingredients at least one inert ingredient (auxiliary) by usual means, e. g. by the means given for the compositions of compounds of formula (I). Concerning usual ingredients of such compositions reference is made to the explanations given for the compositions containing compounds of formula (I).

According to one embodiment, the microbial pesticides selected from groups L1), L3) and L5) embrace not only the isolated, pure cultures of the respective microorganism as defined herein, but also its cell-free extract, its suspensions in a whole broth culture or as a metabolite-containing culture medium or a purified metabolite obtained from a whole broth culture of the microorganism.

When living microorganisms, such as pesticides II from groups L1), L3) and L5), form part of the compositions, such compositions can be prepared as compositions comprising besides the active ingredients at least one auxiliary by usual means (e. g. H.D. Burges: Formulation of Micobial Biopesticides, Springer, 1998). Suitable customary types of such compositions are suspensions, dusts, powders, pastes, granules, pressings, capsules, and mixtures thereof. Examples for composition types are suspensions, capsules, pastes, pastilles, wettable powders or dusts, pressings, granules, insecticidal articles, as well as gel formulations. Herein, it has to be taken into account that each formulation type or choice of auxiliary should not influence the viability of the microorganism during storage of the composition and when finally applied to the soil, plant or plant propagation material. Suitable formulations are e. g. mentioned in WO 2008/002371, US 6955,912, US 5,422,107.

Compounds can be characterized e.g. by Liquid Chromatography Mass spectroscopy (LCMS), NMR and/or by their melting points.

| LCMS Method | LCMS method description | Device description |
|---|---|---|
| 1 | Mobile Phase: A: water + 0,1% TFA; B:ACN | MSD4/5: |
| | Temperature: 60°C | Shimadzu Nexera UHPLC + Shimadzu LCMS 20-20,ESI |
| | Gradient:5% B to 100% B in 1,50min; 100% B 0,25min | Column: |
| | Flow: 0,8ml/min to 1,0ml/min in 1,51 min | Phenomenex Kinetex 1,7µm XB-C18 100A, 50 x 2,1mm |
| | MS method: ESI positive | |
| | Mass range (m/z): 100-700 | |
| 2a | Column: YMC ODS A | LCMS2020 (Shimadzu) |
| | (50 mm×3.0 mm× 3µ) | Ionization source: ESI |
| | Gradient | Mass range: 100 - 800 amu |
| | A: 10Mm Ammonium formate in water. B: 0.1 % Formic acid in acetonitrile | Polarity: Dual (Positive and negative simultaneous scan) |
| | | Mode: Scan |
| | Flow: 1.2 ml/min | LC System: Nexera |
| | Column oven: 40° C | High pressure gradient system. |
| | Gradient program: | Pump: Binary pump |
| | 10 % B to 100 % B in 1.5 min. | Detector: PDA |
| | Hold for 1 min 100 % B. | Scanning wavelength: 220 nm / max plots |
| | 1 min 10% B. Run time: 3.75 min | |
| 2b | Column: Agilent Eclipse Plus C18 (50 mm×4.6 mm× 3µ) | LCMS2020 (Shimadzu) Ionization source: ESI |
| | Gradient | Mass range: 100 - 800 amu |
| | A: 10Mm Ammonium formate in water. | Polarity: Dual (Positive and negative simultaneous scan) |
| | B: 0.1 % Formic acid in acetonitrile | Mode: Scan |
| | Flow: 1.2 ml/min | LC System: Nexera |
| | Column oven: 40° C | High pressure gradient system. |
| | Gradient program: | Pump: Binary pump |
| | 10 % B to 100 % B in 1.5 min. | Detector: PDA |
| | Hold for 1 min 100 % B. | Scanning wavelength: 220 nm / max plots |
| | 1 min 10 % B. Run time: 3.75 or 3.50 min. | |
| 3a | Mobile Phase: A: water + 0,1% TFA; B:ACN | |
| | Temperature: 60°C | |
| | Gradient:5% B to 100% B in 1,50min; | Shimadzu Nexera LCMS 2020 |
| | 100% B 0,25min | Phenomenex Kinetex 1,7µm XB-C18 |
| | Flow: 0,8ml/min to 1,0ml/min in 1,51 min | 100A, 50 x 2,1mm |
| | MS method: ESI positive | |
| | Mass range (m/z): 160-700 | |

### Synthesis examples

### Example 1: Preparation of 3-[3-(trifluoromethyl)-2-thienyl]-1H-indole-5-carbonitrile

### Step 1:

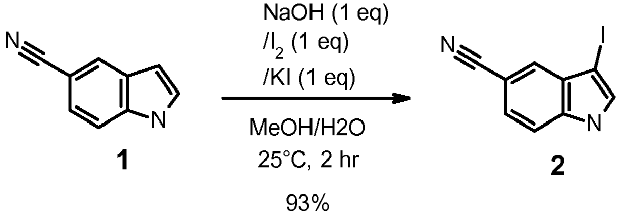

Compound 1 (15 g) was taken in methanol (300 ml) and water (30 ml) mixture and NaOH (4.6 g) and KI (20.89 g) were added. Iodine (30.51 g) was added in portions at RT. Reaction mixture was stirred at RT for 6 hr. Reaction was monitored using LCMS & TLC. After reaction was complete, the reaction mixture was diluted with water (1000 ml) and the precipitated solid was filtered-off and dried under vacuum. Yellow solid obtained was dissolved in DCM and dried over sodium sulphate and concentrated under vacuum. 25 g of pure solid compound 2 was obtained. 1H NMR (300 MHz, DMSO-d6): δ 12.09 (s, 1H), 7.82 - 7.73 (m, 2H), 7.64 - 7.47 (m, 2H).

### Step 2:

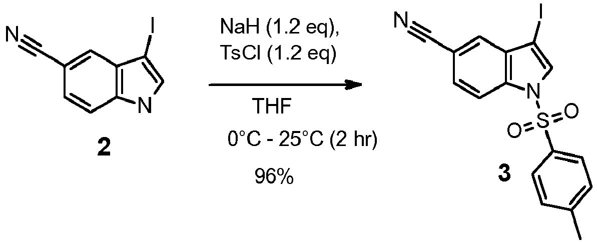

Compound 2 (22 g) was taken in dry THF (200 ml) and cooled to 0°C and added NaH in portions under stirring. After 30 minutes, tosyl chloride was added in portions and the reaction mixture was stirred at RT for 3 hr and reaction was monitored by TLC. After completion, reaction was quenched with saturated ammonium chloride solution and diluted with ethyl acetate. Aqueous layer was extracted with ethyl acetate and the combined organic layer was again washed with water and brine. Organic layer was dried over sodium sulphate and concentrated under vacuum. Ethyl acetate was evaporated to minimum quantity and was diluted with heptane and stirred for 1hr. A solid was obtained which was filtered and dried under vacuum. 33 g of the pure solid compound 3 was obtained.
1H NMR (300 MHz, DMSO-d6): δ 8.31 (s, 1H), 8.13 (d, *J* = 8.6 Hz, 1H), 7.99 (d, *J* = 8.4 Hz, 2H), 7.91 - 7.78 (m, 2H), 7.44 (d, *J* = 8.2 Hz, 2H), 2.34 (s, 3H).

### Step 3:

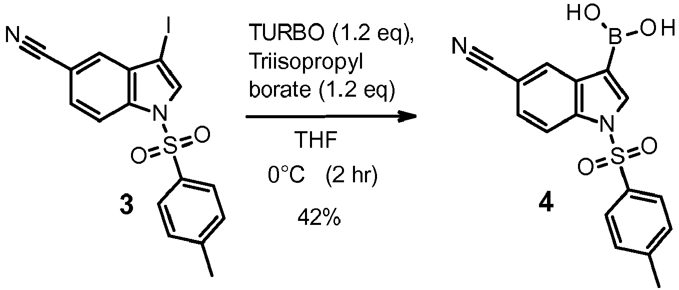

Dissolved compound 3 (13 g) in THF (30 ml). Allowed it to cool to 0°C. Added TURBO (Isopropyl magnesium chloride lithium chloride complex) solution (1.3M in THF) (5.3 g) to the reaction mixture dropwise at 0°C. Allowed it to stir at RT for 20 min. Then added Tri-isopropyl borate (5.35 g) to the reaction mixture at 0°C. Allowed it to stir at RT for 2 hrs. Reaction progress was monitored by TLC and LCMS. Reaction was quenched with 1N HCL Solution and diluted with ethyl acetate. Aqueous layer was extracted with ethyl acetate and the combined organic layer was again washed with brine. Organic layer was dried over sodium sulphate and concentrated under vacuum. The solid was triturated with ethyl acetate/heptane to afford the product as a light brown solid. 7 g of the desired solid compound 4 was obtained.

### Step 4:

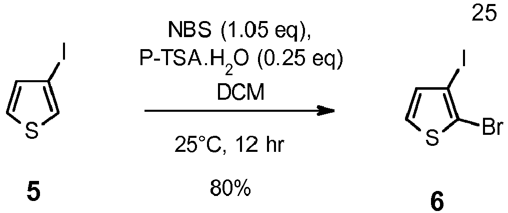

To a stirred solution of compound 5 (1 g) in dry DCM (10 ml) was added p-TSA (0.222 g). Cooled the reaction mixture to 0 °C and to this solution, N-Bromo succinimide (0.889 g) was added in portions. Allowed the reaction mixture to reach 25°C and stirred for 12 h. Reaction was monitored by LCMS. After completion, added Na₂S₂O₃ solution and DCM. Stirred and separated the organic layer and washed with water and brine. Organic layer was dried over Na₂SO₄. Evaporated the solvent completely. Crude compound was purified by flash column chromatography. Pure compound was eluted with only Heptane. Evaporation of solvent afforded 1.1 g of compound 6.
1H NMR (300 MHz, DMSO-d6): δ 7.64(d, J = 5.4 Hz, 1H), 7.09 (d, J =5.4 Hz, 1H).

### Step 5:

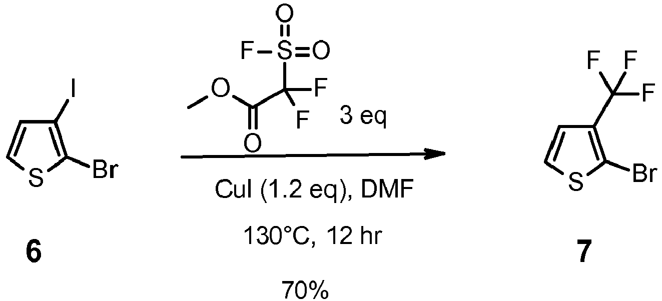

Compound 6 (0.5 g) was taken in DMF (5 ml) and fluorinating agent (0.997 g) followed by Cul (0.494 g) addition under nitrogen atmosphere. Resulting reaction mixture was heated and stirred at 90°C for 12 hrs. Reaction was monitored by TLC and LCMS. After completion of the reaction, cooled it to RT and added ice water to it. The solid precipitated was filtered through celite. The aqueous layer was extracted with pentane. Combined organic layer was washed with water and brine. Organic layer was dried over Na₂SO₄ and evaporated completely to afford the product 0.380 g as compound 7.

### Step 6:

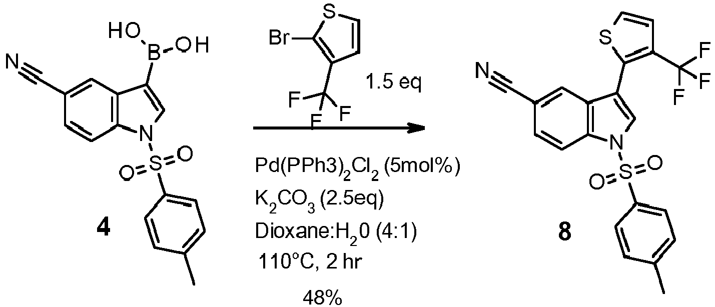

Compound 4 (0.6 g), bromo-thiophene (0.60 g) and K₂CO₃ (0.72 g) were taken in a mixture of dioxane (2.4ml) and water (0.6ml) in a RB flask. Resulting mixture was degassed for 10 min. To this was added Pd(pph3)₂Cl₂ (0.061 g) and again degassed for 5 min. Resulting reaction was stirred and heated at 110°C for 2 hrs under nitrogen atmosphere. Reaction was monitored by TLC. After completion of the reaction, cooled it to RT. Filtered the reaction through celite and washed with ethyl acetate. The aqueous layer was extracted with ethyl acetate. Combined organic layer was washed with brine. Organic layer was dried over Na₂SO₄ and evaporated completely. Crude material was purified by flash chromatography. Pure compound was eluted using 15-20% ethyl acetate in Heptane. Evaporation of solvent afforded 0.38 g of compound 8.

### Step 7:

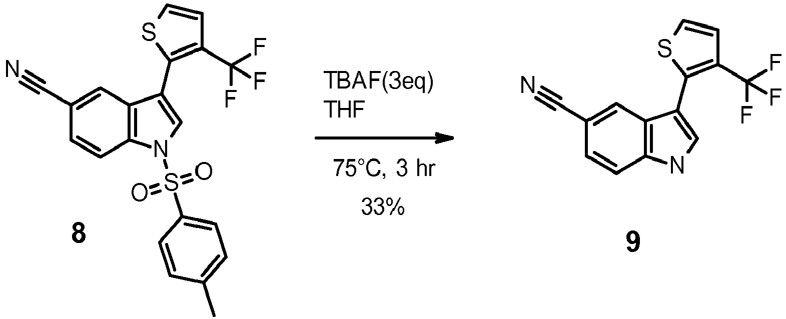

To a solution of compound 8 (0.380 g) in THF (4 ml) was added a solution of TBAF (0.668 g) (1M solution in THF). The resulting reaction mixture was refluxed for 2 hours. Reaction progress was monitored by TLC and LCMS. After completion of the reaction, it was cooled to RT and quenched with water. Aqueous layer was extracted with ethyl acetate. The combined organic layer was washed with brine and dried over Na₂SO₄ and the solvent was evaporated. The crude product was purified by flash column chromatography. Pure compound was eluted with 20-25% ethyl acetate in Heptane. Evaporation of solvent afforded 0.083 g of white solid compound 9.

### Example 2: Preparation of 3-[4-(trifluoromethyl)oxazol-5-yl]-1H-indole-5-carbonitrile

### Step 1:

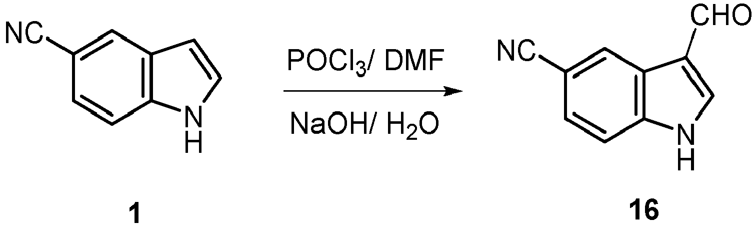

POCl₃ (15 mL) was added drop-wise to DMF (100 mL) at 0°C, and then stirred for 30 min at 0°C, 1 (21.3 g, 150 mmol) was dissolved in DMF (50 mL) and added drop-wise, the resulting mixture was stirred for 1h at 35°C. 1 was consumed on TLC (PE: EtOAc=1:1), quenched with ice (-200 g), and basified pH to 8 with 2M NaOH solution, with solid was formed, filtered, the filter cake was dried under vacuum to get 16 (25.1 g, yield: 98.4%) as a yellow solid.
¹H NMR (DMSO Varian_D_400MHz): δ: 7.58 - 7.63 (m, 1 H) 7.65 - 7.74 (m, 1 H) 8.46 (bd, *J*=13.89 Hz, 2 H) 9.98 (s, 1 H).

### Step 2:

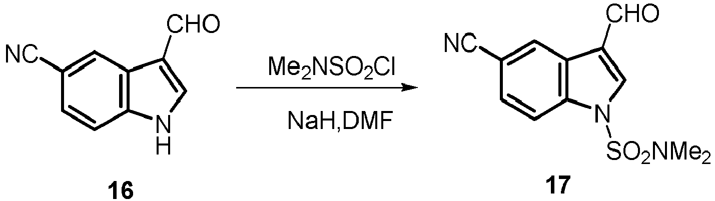

To a solution of 16 (5.1 g, 30 mmol) in DMF (50 mL) was added NaH (1.78 g, 45 mmol) in portions at 0°C, then stirred for 15 min at 0°C, with the sulfonyl chloride compound (4.72 g, 33 mmol) added drop-wise, and the resulting mixture was stirred for 2 h at 5°C. TLC (PE: EtOAc=1:1) showed 16 was consumed, the reaction solution was quenched with ice (-200 g), the solid was formed, filtered, the filter cake was dried under vacuum to obtain 17 (21 g, yield: 81.1%) as a yellow solid.
¹H NMR (DMSO Bruker_B_400MHz): δ: 2.92 (s, 6 H) 7.87 (dd, *J*=8.72, 1.57 Hz, 1 H) 8.11 (d, *J*=8.78 Hz, 1 H) 8.56 (d, *J*=1.00 Hz, 1 H) 8.89 (s, 1 H) 10.10 (s, 1H).

### Step 3:

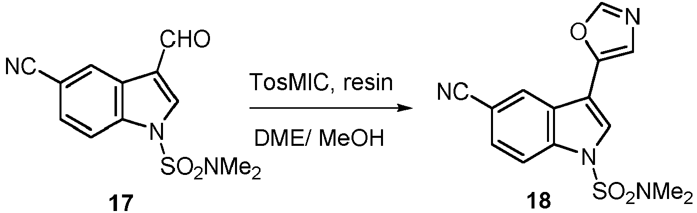

To a solution of 17 (18 g, 62.5 mmol) in DME\MeOH (200 mL\200 mL) was added TosMIC reagent (13.4 g, 68.7 mmol), resin (120 g), and then stirred for 16 h at 95°C. TLC (PE: EtOAc=1:1) showed 17 was consumed, filtered, the filter cake was washed with THF (500 mL), organic phase was combined, concentrated, purified by silica gel chromatography (gradient EtOAc: PE = from 1:3 to 2: 1) to obtain 18 (5 g, yield: 21.74%) as a yellow solid.
¹H NMR (DMSO Varian_D_400MHz): δ: 2.89 (s, 6 H) 7.85 (dd, *J*=8.80, 1.56 Hz, 1 H) 7.93 (s, 1 H) 8.12 (d, *J*=8.80 Hz, 1 H) 8.26 (s, 1 H) 8.52 (s, 1 H) 8.58 (d, *J*=1.17 Hz, 1 H).

### Step 4:

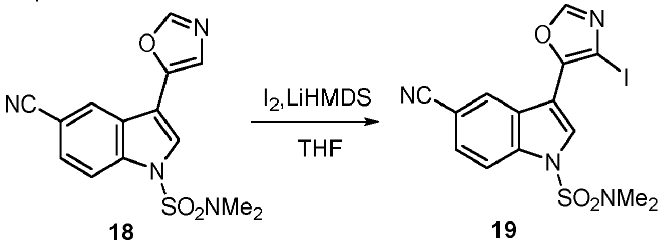

To a solution of 18 (634 mg, 2 mmol) in THF (18 mL) was added LiHMDS (6 mL, 6 mmol) drop-wise at -65°C, then stirred for 30 min at -65°C, followed by I₂ (610 mg, 2.4 mmol) in THF (2 mL) added drop-wise at -65°C, the resulting mixture was stirred for 1 h at -65°C. Most of 18 was consumed on TLC (PE: EtOAc=2:1), quenched with NH₄Cl solution (15 mL), extracted with EtOAc (20 mL x 2), organic phase was washed with brine (15 mL), dried Na₂SO₄, concentrated, purified by silica gel chromatography (gradient EtOAc: PE = from 1:5 to 1: 2) to obtain 19 (600 mg, yield: 67.8%) as a yellow solid.
¹H NMR (DMSO Bruker_B_400MHz): δ: 2.91 (s, 6 H) 7.87 (dd, *J*=8.72, 1.32 Hz, 1 H) 8.14 (d, *J*=8.78 Hz, 1 H) 8.39 (s, 1 H) 8.46 (s, 1 H) 8.62 (s, 1 H).

### Step 5:

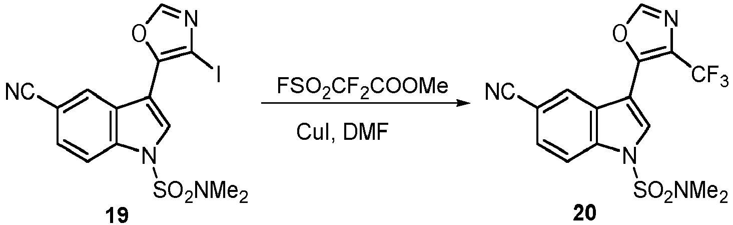

To a solution of 19 (560 mg, 1.266 mmol) in DMF (6 mL) was added Trifluoromethylation agent (1.21 g, 6.33 mmol) and Cul (1.21 g, 6.33 mmol) and stirred for 2h at 85°C. 19 was consumed on TLC (PE: EtOAc=1:1), cooled to RT, diluted with EtOAc (25 mL), filtered, the filtrated was concentrated, purified by silica gel chromatography (gradient EtOAc: PE = from 1:10 to 1: 3) to get 20 (490 mg, yield: 72.3%) as a yellow solid.
¹H NMR (CDCl3 Bruker_B_400MHz): δ: 2.94 (s, 6 H) 7.67 - 7.71 (m, 1 H) 7.97 (s, 1 H) 8.10 - 8.16 (m, 2 H) 8.34 (d, *J*=0.88 Hz, 1 H).

### Step 6:

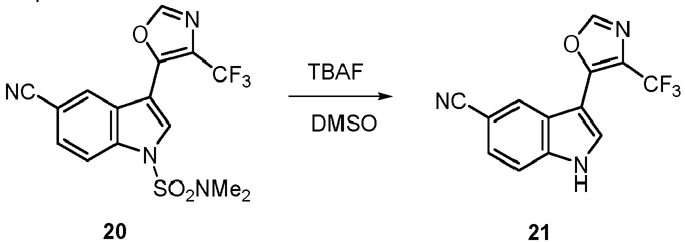

To a solution of 20 (450 mg, 1.17 mmol) in DMSO (2.5 mL) was added TBAF (1M in THF, 4.68 mL) and stirred for 5h at 45°C, TLC (PE: EtOAc=1:1) showed that 20 was consumed. It was then quenched with water (10 mL), extracted with EtOAc (15 mL x 3), organic phase was washed with brine (15 mL), dried Na₂SO₄, concentrated, was purified by Prep-TLC (DCM : MeOH = 20:1) to get 21 (0.23 g, yield: 71.0%) as a white solid.
¹H NMR (MeOD) Bruker_B_400MHz: δ: 7.55 (dd, *J*=8.53, 1.25 Hz, 1 H) 7.65 (d, *J*=8.53 Hz, 1 H) 7.89 (s, 1 H) 8.34 (s, 1 H) 8.38 (s, 1 H).

### Example 3: Preparation of 3-(4-iodooxazol-5-yl)-1H-indole-5-carbonitrile

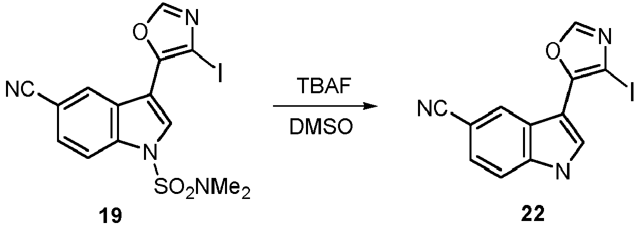

To a solution of 19 (442 mg, 1 mmol) in DMSO (1.5 mL) was added TBAF (1M in THF, 3 mL) and stirred for 2h at 50°C, TLC (PE: EtOAc=2:1) showed 19 was consumed. Then quenched with water (20 mL), extracted with EtOAc (25 mL x 2), organic phase was dried Na₂SO₄, concentrated, was purified by silica gel chromatography (gradient EtOAc: PE = from 1:10 to 1: 3) to obtain 22 (100 mg, yield: 29.8%) as a white solid.
¹H NMR (MeOD) Varian_D_400MHz: δ: 7.51 (dd, *J*=8.41, 1.37 Hz, 1 H) 7.62 (d, *J*=8.61 Hz, 1 H) 8.25 (s, 1 H) 8.30 (s, 1 H) 8.40 (s, 1 H).

### Example 4: Preparation of 3-(4-bromooxazol-5-yl)-1H-indole-5-carbonitrile

### Step 1:

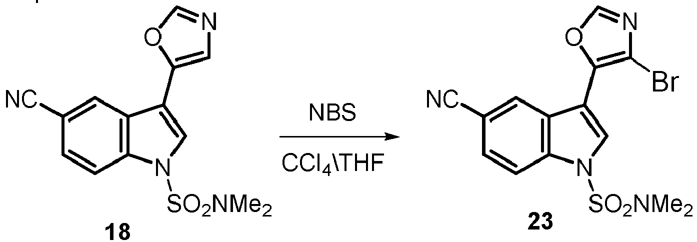

To a mixture of 18 (634 mg, 2 mmol) in THF/CCl₄ (4 mL/4 mL) was added NBS (4274 mg, 2.4 mmol) and stirred for 2h at 50°C, TLC (PE: EtOAc=1:1) showed that 18 was consumed, concentrated to get 23 (1.1 g crude, yellow solid) and was used directly.
¹H NMR (DMSO) Bruker_B_400MHz: δ 2.90 (s, 6 H) 7.88 (dd, *J*=8.78, 1.51 Hz, 1 H) 8.14 (d, *J*=8.78 Hz, 1 H) 8.27 (s, 1 H) 8.48 (s, 1 H) 8.67 (s, 1 H).

### Step 2:

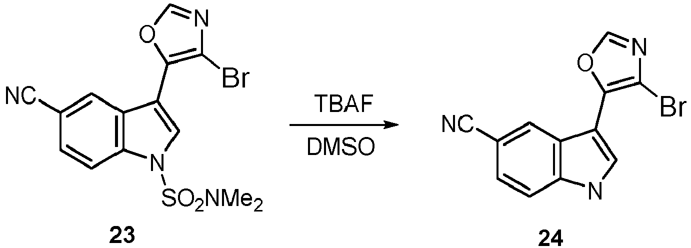

To a solution of 23 (396 mg crude, -0.6 mmol) in DMSO (3 mL) was added TBAF (1M in THF, 1.2 mL) and stirred for 16h at 70°C, TLC (PE: EtOAc=1:1) showed that 23 was consumed. It was then quenched with water (10 mL), extracted with EtOAc (20 mL x 3), organic phase was washed with brine (20 mL), dried Na₂SO₄, concentrated, was purified by silica gel chromatography (gradient EtOAc: PE = from 1:5 to 1: 1) to get 24 (200 mg, yield: 57.8%) as a brown solid. ¹H NMR (MeOD) Bruker_B_400MHz: δ 7.52 (dd, *J*=8.53, 1.51 Hz, 1 H) 7.62 (d, *J*=8.53 Hz, 1 H) 8.13 (s, 1 H) 8.28 (s, 1 H) 8.42 (d, *J*=0.75 Hz, 1 H).

### Example 5: Preparation of 3-(4-(difluoromethyl)oxazol-5-yl)-1H-indole-5-carbonitrile

### Step 1:

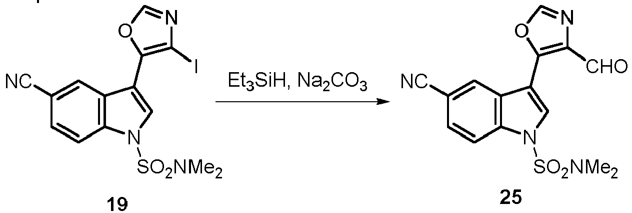

To a solution of 19 (1.4 g, 3.17 mmol) in DMF (15 mL) was added Et₃SiH (1.47 g 12.68 mmol), Na₂CO₃ (672 mg, 6.34 mmol), Pd (dppf) Cl₂ (200 mg), the mixture was stirred for 3 h at 70°C under CO at 50Psi. TLC (PE: EtOAc=1:1) showed that 19 was consumed, then quenched with water (100 mL), extracted with EtOAc (100 mL x 2), organic phase was washed with brine (60 mL), dried Na₂SO₄, concentrated, was purified by silica gel chromatography (gradient EtOAc: PE = from 1:3 to 2: 1) to get 25 (4 g crude) as a dark solid and used directly.

### Step 2:

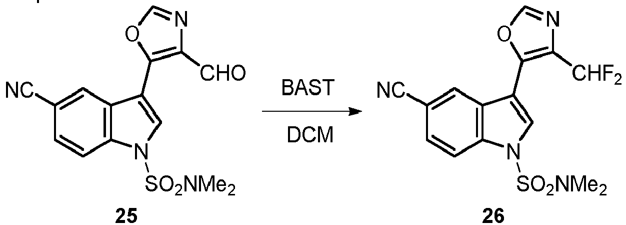

To a solution of 25 (1.72 g crude, ∼2 mmol) in DCM (15 mL) was added bis(2-methoxyethyl)aminosulfur trifluoride (BAST) (4.42 g 20 mmol) and stirred for 2 h at 55 °C. Quenched with ice (-20 g), extracted with DCM (25 mL x 2), organic phase was washed with NaHCO₃ solution (20 mL), brine (20 mL), dried Na₂SO₄, concentrated, was purified by silica gel chromatography (gradient EtOAc: PE = from 1:10 to 1: 3) to get 26 (950 mg, yield: 64.9%) as a yellow solid.
¹H NMR (CDCl₃) Varian_D_400MHz: δ 2.93 (s, 6 H) 6.70 - 6.97 (m, 1 H) 7.68 (dd, *J*=8.80, 1.37 Hz, 1 H) 8.03 (s, 1 H) 8.07 (s, 1 H) 8.14 (d, *J*=8.80 Hz, 1 H) 8.39 (d, *J*=0.98 Hz, 1 H).

### Step 3:

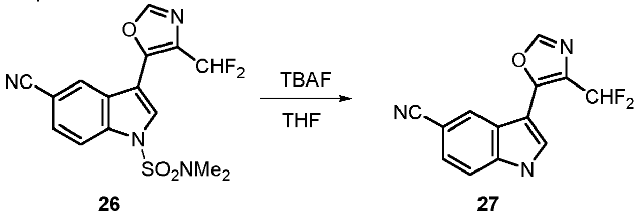

To a solution of 26 (900 mg, 2.46 mmol) in THF (3 mL) was added TBAF (1M in THF, 9.8 mL) and stirred for 16 h at 20°C, TLC (PE: EtOAc=2:1) showed that 26 was consumed. Quenched with water (10 mL), extracted with EtOAc (15 mL x 2), organic phase was washed with brine (10 mL), dried Na₂SO₄, concentrated, was purified by silica gel chromatography (gradient EtOAc: PE = from 1:5 to 1: 2) to obtain 27 (180 mg, yield: 28.4%) as a yellow solid.
¹H NMR (MeOD) Varian_D_400MHz: δ 6.82 - 7.09 (m, 1 H) 7.42 - 7.58 (m, 1 H) 7.64 (d, *J*=8.61 Hz, 1 H) 7.90 (s, 1 H) 8.17 - 8.52 (m, 2 H).

### Example 6: Preparation of 5-(5-ethynyl-1H-pyrrolo[2,3-b]pyridin-3-yl)-4-(trifluoromethyl)oxazole

### Step 1:

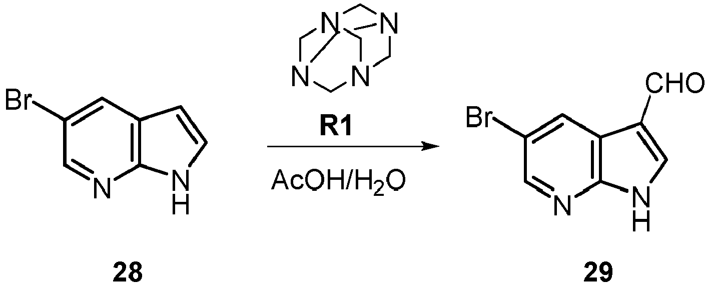

To a mixture of 28 (8.7 g, 44 mmol) in AcOH\H₂O (20 mL\ 30 mL) was added R1 (8.7 g, 61.6 mmol) and stirred for 16 h at 120°C in a sealed tube, TLC (PE: EtOAc=3:1) showed that 28 was consumed. Quenched with water (200 mL), with solid formed, filtered, the filter cake was washed with water (50 mL), dried under vacuum to get 29 (18 g, yield: 90.59%) as a yellow solid.
¹H NMR (DMSO) Varian_D_400MHz: δ 8.45 (d, 2 H) 8.52-8.53(d, 1 H) 9.92 (s, 1 H)

### Step 2:

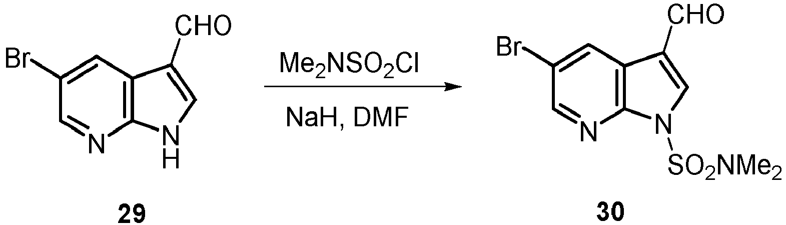

To a solution of 29 (38.5 g, 170 mmol) in DMF (300 mL) was added NaH (8.16 g, 204 mmol) in portions at 0°C, then stirred for 30 min at 0°C, with sulfonyl chloride (26.93 g, 187 mmol) added drop-wise, and the resulting mixture was stirred for 16 h at 20°C. TLC (PE: EtOAc=1:1) showed 29 was consumed, the reaction solution was quenched with water (500 mL), with solid was formed, filtered, the filter cake was washed with water (150 mL), dried under vacuum to get 30 (48 g, yield: 84.2%) as a yellow solid.
¹H NMR (DMSO) Varian_D_400MHz: δ 3.00 (s, 6 H) 8.57 - 8.67 (m, 2 H) 8.81 (s, 1 H) 10.02 (s, 1 H).

### Step 3:

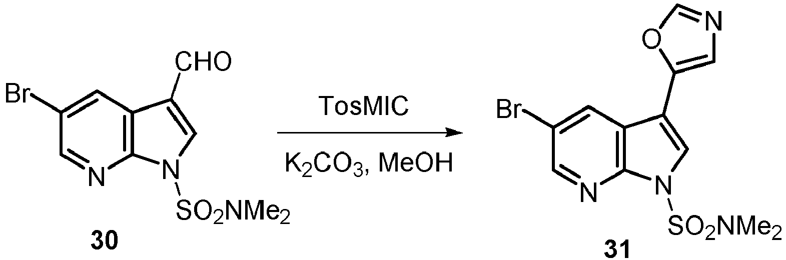

To a solution of 30 (41 g, 123.1 mmol) in MeOH (1200 mL) was added TosMIC (26.4 g, 135.4 mmol), K₂CO₃ (18.7 g, 135.4 mmol), and then stirred for 6 h at 85°C. TLC (PE: EtOAc=2:1) showed 30was consumed, concentrated, purified by silica gel chromatography (gradient EtOAc: PE = from 1:5 to 1: 2) to get 31 (20 g, yield: 43.8%) as a yellow solid.
¹H NMR (DMSO) Bruker_B_400MHz: δ 2.97 (s, 6 H) 7.88 (s, 1 H) 8.17 (s, 1 H) 8.49 (s, 1 H) 8.61 (d, *J*=1.88 Hz, 1 H) 8.69 (d, *J*=2.01 Hz, 1 H).

### Step 4:

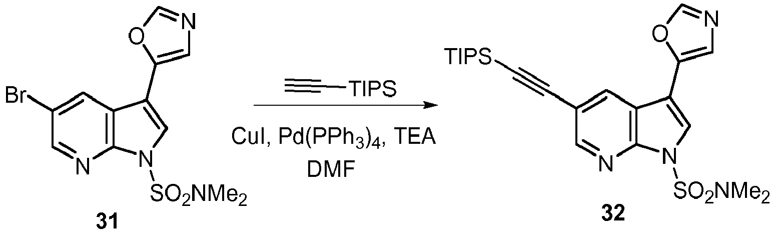

To a solution of 31 (12 g, 32.4 mmol) in DMF (120 mL) was added TIPS-acetylene (17.7g, 97.3 mmol), Pd (PPh₃)₄ (1.82 g, 1.62 mmol), TEA (9.8 g, 97.3 mmol), CuI (1.24 g, 6.48 mmol), and the resulting mixture was stirred for 2 hrs at 100°C. TLC (PE: EtOAc=2:1) showed that 31 was consumed. Quenched with water (200 mL), extracted with EtOAc (200 mL x 3), organic phase was washed with brine (150 mL), dried Na₂SO₄, concentrated, purified by silica gel chromatography (gradient EtOAc : PE = from 1:20 to 1:5) to get 32 (9.5g, yield: 62.1%) as a yellow solid.
¹H NMR (CDCl₃) Varian_D_400MHz: δ 1.15 - 1.19 (m, 21 H) 3.08 (s, 6 H) 7.37 (s, 1 H) 7.96 (s, 1 H) 7.98 (s, 1 H) 8.23 (d, *J*=1.96 Hz, 1 H) 8.59 (d, *J*=1.56 Hz, 1 H).

### Step 5:

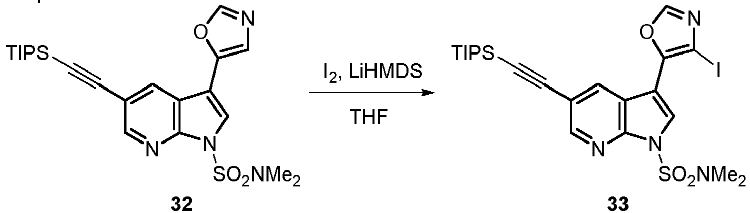

To a solution of 32 (3.4 g, 7.2 mmol) in THF (150 mL) was added LiHMDS (7.2 mL, 18 mmol) drop-wise at -65°C, then stirred for 20 min, followed by I₂ (2.38 g, 9.36 mmol) in THF (20 mL) added drop-wise, the resulting mixture was stirred for 0.5 h at -65°C. 32 was consumed on TLC (PE: EtOAc=5:1), quenched with NH₄Cl solution (60 mL), extracted with EtOAc (80 mL x 2), organic phase was washed with Na₂SO₃ solution (50 mL), brine (50 mL), dried Na₂SO₄, concentrated, purified by silica gel chromatography (gradient EtOAc: PE = from 1:50 to 1: 10) to obtain 33 (1.4 g, yield: 32.5%) as a yellow solid.
¹H NMR (CDCl₃) Bruker_B_400MHz: δ 1.14 - 1.19 (m, 21 H) 3.09 (s, 6 H) 8.01 (s, 1 H) 8.41 (d, *J*=1.88 Hz, 1 H) 8.45 (s, 1 H) 8.60 (d, *J*=1.76 Hz, 1H).

### Step 6:

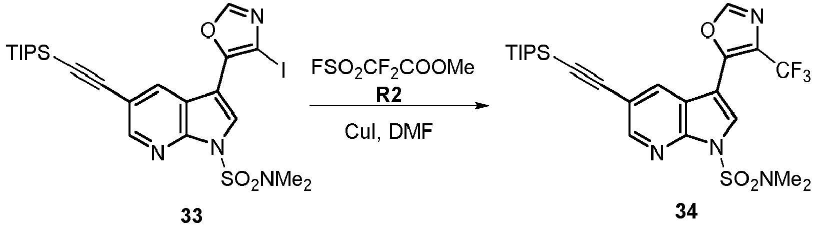

To a solution of 33 (1.4 g, 2.3 mmol) in DMF (15 mL) was added R2 (1.32 g, 6.9 mmol) and CuI (1.32 1 g, 6.9 mmol) and stirred for 1.5 h at 90°C. 33 was consumed on TLC (PE: EtOAc = 5:1), quenched with water (25 mL), extracted with EtOAc (25 mL x 3), organic phase was washed with brine (25 mL), dried Na₂SO₄, concentrated , purified by silica gel chromatography (gradient EtOAc : PE = from 1:50 to 1:10) to get 34 (250 mg, yield: 20.1%) as a yellow solid.
¹H NMR (CDCl₃) Bruker_B_400MHz: δ 1.15 - 1.18 (m, 21 H) 3.09 (s, 6 H) 8.04 (s, 1 H) 8.08 (s, 1 H) 8.31 (d, *J*=1.76 Hz, 1 H) 8.61 (d, *J*=1.76 Hz, 1 H).

### Step 7:

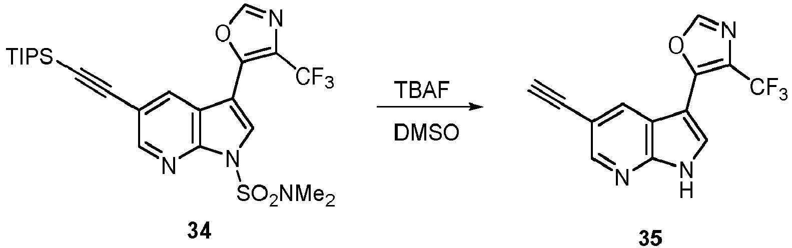

To a solution of 34 (500 mg, 0.926 mmol) in DMSO (2 mL) was added TBAF (1M in THF, 4.63 mL) and stirred for 1.5h at 40°C, TLC (PE: EtOAc=3:1) showed that 34 was consumed. Quenched with water (10 mL), extracted with EtOAc (15 mL x 3), organic phase was washed with brine (10 mL), dried Na₂SO₄, concentrated, was purified by Prep-TLC (PE : EtOAc = 2:1) to get 35 (0.15 g, yield: 58.6%) as a yellow solid.
¹H NMR (MeOD) Varian_D_400MHz: δ 3.60 (s, 1 H) 7.88 (s, 1 H) 8.36 (s, 1 H) 8.41 (d, *J*=1.96 Hz, 1 H) 8.43 (d, *J*=1.76 Hz, 1 H).

### Example 7: Preparation of 5-(5-ethynyl-1H-pyrrolo[2,3-b]pyridin-3-yl)-4-iodooxazole

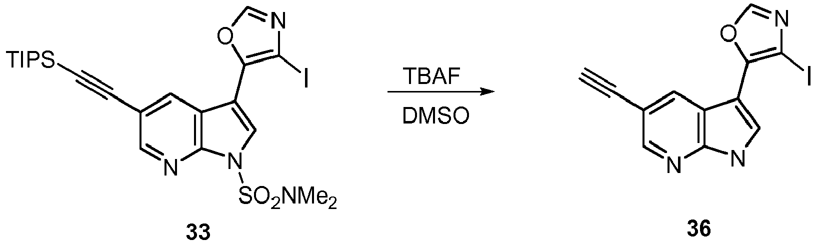

To a solution of 33 (600 mg crude, ∼1 mmol) in DMSO (4 mL) was added TBAF (1M in THF, 4 mL) and stirred for 16h at 20 °C, then heated to 40°C and stirred for another 16 h, TLC (PE: EtOAc=4:1) showed that 33 was consumed. Quenched with water (15 mL), extracted with EtOAc (15 mL x 3), organic phase was dried Na₂SO₄, concentrated, purified by silica gel chromatography (gradient EtOAc: PE = from 1:5 to 1:3) to get 36 (100 mg, yield: 30%) as a white solid.
¹H NMR (MeOD) Bruker_B_400MHz: δ 3.60 (s, 1 H) 8.25 (s, 1 H) 8.29 (s, 1 H) 8.41 (d, *J*=1.88 Hz, 1 H) 8.49 (d, *J*=1.88 Hz, 1 H).

### Example 8: Preparation of 5-(5-fluoro-1H-pyrrolo[2,3-b]pyridin-3-yl)-4-(trifluoromethyl)oxazole

### Step 1:

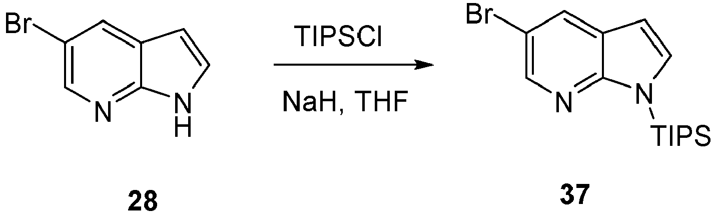

To a suspension of 28 (37 g, 180 mmol) in THF (500 mL) was added NaH (60%, 9.06 g, 220 mmol) in portions at 0°C under N₂ and stirred for 30 min. Then TIPSCI (43.6 g, 220 mmol) was added drop wise and the mixture was stirred at 0∼20°C for 3 h. TLC (PE: EtOAc=5:1) showed the reaction was completed. The mixture was quenched with 100 mL H₂O, extracted with 500 mL EtOAc, washed with brine (100 mL), dried over Na₂SO₄, filtered, concentrated and purified by silica gel column (PE: EtOAc=20:1∼5:1) to get 37 (60 g, 94.5%) as a white solid.
¹H NMR (CDCl₃) Bruker_B_400MHz: δ ppm 1.12 (d, *J*=7.53 Hz, 18 H) 1.84 (spt, *J*=7.55 Hz, 3 H) 6.50 (d, *J*=3.51 Hz, 1 H) 7.31 (d, *J*=3.51 Hz, 1 H) 7.98 (d, *J*=2.13 Hz, 1 H) 8.28 (d, *J*=2.26 Hz, 1 H).

### Step 2:

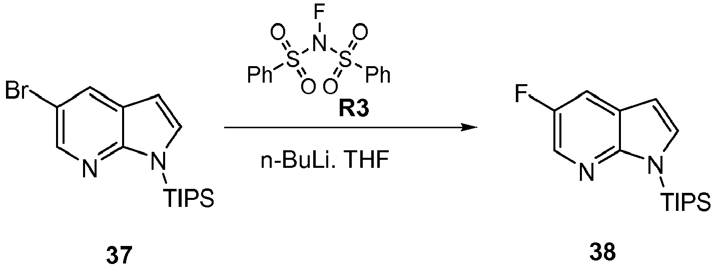

To a solution of 37 (30 g, 85.2 mmol) in THF (400 mL) was added n-BuLi (2.5 M, 34 mL, 85.2 mmol) drop wise at -70°C under N₂ and stirred for 10 min. The mixture turned cloudy and large white solid appeared. Then R3 (32.2 g, 102.2 mmol) in THF (100 mL) was added drop wise at - 70°C. The mixture was stirred at -70∼20°C for 16 h. TLC (PE: EtOAc=10:1) showed the reaction was completed. The mixture was poured into 500 mL aq. NH4CI, extracted with 500 mL EtOAc, washed with brine (100 mL), dried over Na₂SO₄, filtered, concentrated and purified by silica gel column (pure PE) to get 38 (32 g, 64.3%) as a white solid.
¹H NMR (CDCl₃) Bruker_B_400MHz: δ ppm 1.09 - 1.20 (m, 18 H) 1.75 - 1.95 (m, 3 H) 6.53 (dd, *J*=3.52, 0.78 Hz, 1 H) 7.37 (d, *J*=3.52 Hz, 1 H) 7.51 - 7.60 (m, 1 H) 8.11 - 8.18 (m, 1 H).

### Step 3:

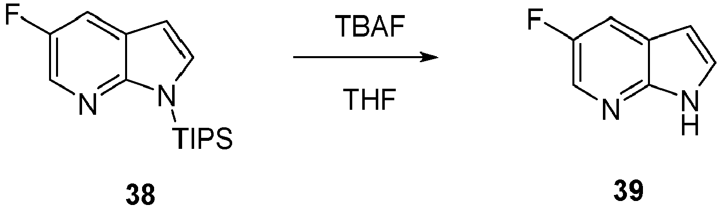

To a solution of 38 (27.9 g, 95.5 mmol) in THF (250 mL) was added TBAF (191.1 mL, 191.1 mmol) at 0°C under N₂. The resulting mixture was stirred for 2 hrs at 20°C. TLC (PE: EtOAc = 5:1) showed the reaction was completed. The reaction mixture was added H₂O (200 mL) and extracted with EtOAC (200 mL x 3). The combined organic layers were washed with brine (200 mL x 3), dried over Na₂SO₄, concentrated and purified by column chromatography (PE: EA=8:1∼1:1) to get 39 (11.4 g, yield: 87%).
¹HNMR (CDCl₃) Varian_D_400MHz: δ ppm 6.51 (dd, *J*=3.51, 2.01 Hz, 1 H) 7.42 (t, *J*=2.95 Hz, 1 H) 7.66 (dd, *J*=9.03, 2.51 Hz, 1 H) 8.22 (t, *J*=2.13 Hz, 1 H).

### Step 4:

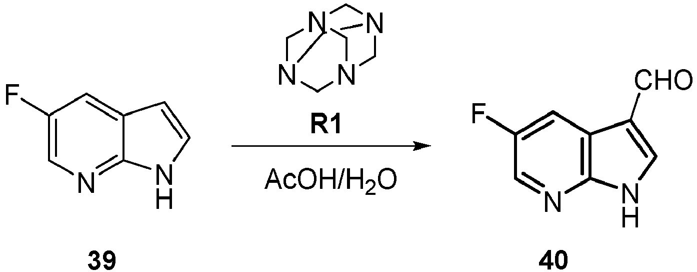

To a solution of 39 (18 g, 132.35 mmol) in AcOH/H₂O (20 mL/30 mL) was added R1 (25.9 g, 185.29 mmol) at 20°C. The resulting mixture was stirred for 8 hrs at 120°C in an autoclave. TLC (DCM: MeOH = 20:1) showed the reaction was completed. The reaction mixture was filtered to get 40 (15.1 g, Yield: 69%).
¹H NMR (DMSO) Bruker_B_400MHz: δ ppm 8.05 (s, 1 H) 8.32 (t, *J*=2.15 Hz, 1 H) 8.33 - 8.37 (m, 1 H) 10.03 (s, 1 H).

### Step 5:

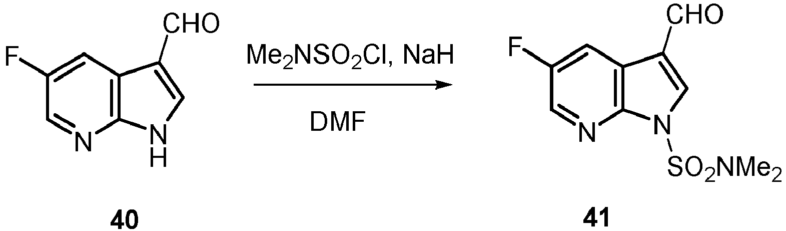

To a solution of 40 (13.4 g, 49.39 mmol) in DMF (150 mL) was added NaH (2.77 g, 69.15 mmol) at 0°C. The mixture was stirred for 0.5 hrs at 0°C. Then MeNSO₂Cl (8.48 g, 59.27 mmol) was added. The resulting mixture was stirred for 17 hrs at 20°C. TLC (DCM: MeOH = 20:1) showed the reaction was completed. The reaction mixture was added H₂O (150 mL) and extracted with EtOAc (150 mL x 3). The combined organic layers were washed with brine (150 mL x 3), dried over Na₂SO₄, concentrated to get 41 (16.8 g, yield: 75%).
¹H NMR (DMSO) Varian_D_400MHz: δ ppm 3.00 (s, 6 H) 8.28 (dd, *J*=8.53, 2.89 Hz, 1 H) 8.56 (dd, *J*=2.76, 1.51 Hz, 1 H) 8.85 (s, 1 H) 10.02 (s, 1 H).

### Step 6:

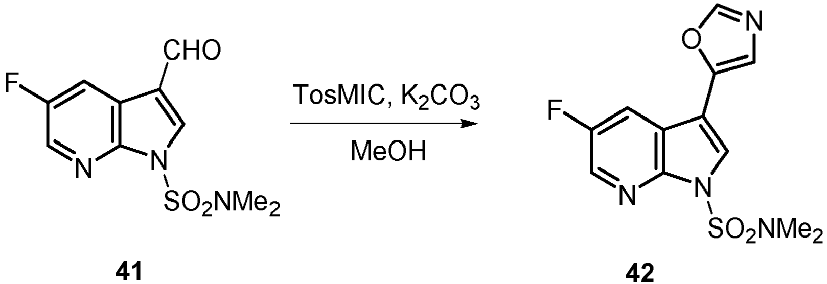

To a solution of 41 (11.5 g, 42.44 mmol) in MeOH (424 mL) was added TosMIC (9.1 g, 46.68 mmol) and K₂CO₃ (6.44 g, 17.74 mmol) at 20°C under N₂. Then the reaction was stirred for 8 h at 85°C refluxed. TLC (PE: EtOAc=1:1) showed the reaction was completed. The reaction mixture was added H₂O (200 mL) and extracted with EtOAc (200 mL x 3). The combined organic layers were washed with brine (200 mL x 3), dried over Na₂SO₄, concentrated and purified by column chromatography (PE: EA=10:1∼1:1) to get 42 (9.1 g, yield: 69%).
¹H NMR (CDCl₃) Varian_D_400MHz: δ ppm 3.08 (s, 6 H) 7.33 (s, 1 H) 7.89 (dd, *J*=8.28, 2.64 Hz, 1 H) 7.99 (d, *J*=11.29 Hz, 2 H) 8.37 (dd, *J*=2.45, 1.07 Hz, 1 H).

### Step 7:

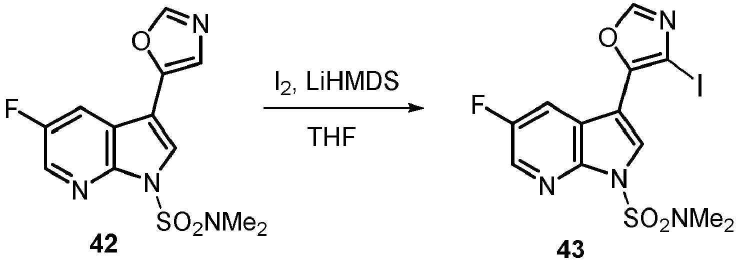

To a solution of 42 (4.2 g, 13.55 mmol) in THF (160 mL) was added LiHMDS (33.9 mL, 33.9 mmol) at -70°C under N₂. The mixture was stirred for 30 min at -70°C. Then a solution of I₂ (4.47 g, 17.61 mmol) in THF (20 mL) was added drop-wise. The mixture was stirred for 20 min at -70°C. TLC (PE: EtOAc=1:1) showed the reaction was completed. The reaction was quenched with water (200 mL), extracted with EtOAc (200 mL x 2), organic phase was washed with brine (200 mL x 2), dried over Na₂SO₄, concentrated and purified by column chromatography (PE: EA=10:1∼1:1) to get 43 (3.6 g, yield: 64%).
¹H NMR (CDCl₃) Varian_D_400MHz: δ ppm 3.10 (s, 6 H) 7.98 (s, 1 H) 8.07 (dd, *J*=8.53, 2.76 Hz, 1 H) 8.38 (dd, *J*=2.51, 1.00 Hz, 1 H) 8.50 (s, 1 H).

### Step 8:

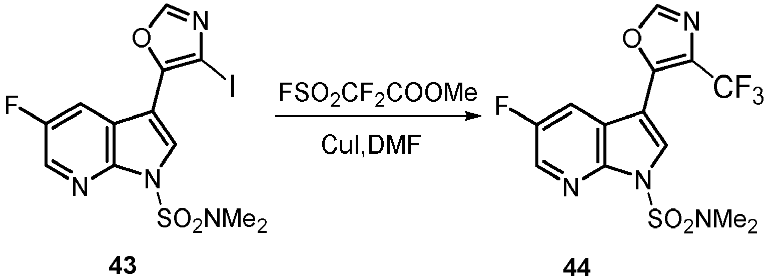

To a solution of 43 (972 mg, 2 mmol) in DMF (10 mL) was added FSO₂CF₂COOMe (1.1 g, 6 mmol) and CuI (1.1 g, 6 mmol), and stirred for 2 h at 90°C. LCMS showed the reaction was completed. The reaction was filtered and quenched with water (20 mL), extracted with EtOAc (20 mL x 2), organic phase was washed with brine (20 mL x 2), dried over Na₂SO₄, concentrated and purified by column chromatography (PE: EA=10:1∼1:1) to get 44 (480 mg, yield: 63%).
¹H NMR (CDCl₃) Bruker_B_400MHz: δ ppm 3.09 (s, 6 H) 7.97 (dd, *J*=8.47, 2.70 Hz, 1 H) 8.05 (s, 1 H) 8.10 (s, 1 H) 8.40 (dd, *J*=2.51, 1.00 Hz, 1 H).

### Step 9:

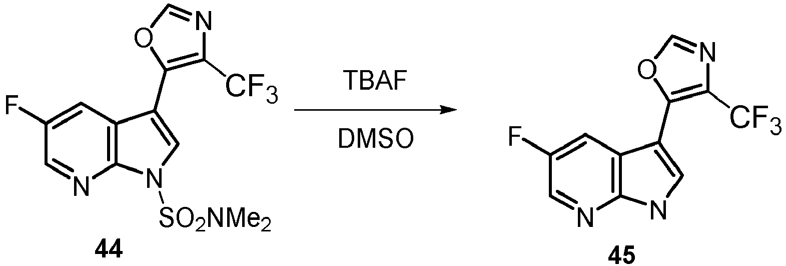

To a solution of 44 (570 mg, 1.49 mmol) in DMSO (8 mL) was added TBAF (1M in THF, 6 mL, 6 mmol), and stirred for 8 h at 40°C. TLC (PE: EtOAc=1:1) showed the reaction was completed. The reaction was quenched with water (20 mL), extracted with EtOAc (20 mL x 3). The organic layer was washed with brine (20 mL x 3), dried over Na₂SO₄, filtered and concentrated to afford the crude product. Then purified by column chromatography (PE: EA=10:1∼1:1) and HPLC to get 45 (150 mg, yield: 52%).
¹H NMR (CDCl₃) Bruker_B_400MHz: δ ppm 7.93 (s, 1 H) 8.09 (dd, *J*=9.03, 2.76 Hz, 1 H) 8.29 (t, *J*=2.07 Hz, 1 H) 8.38 (s, 1 H).

### Example 9: Preparation of 5-(5-fluoro-1H-pyrrolo[2,3-b]pyridin-3-yl)-4-iodooxazole

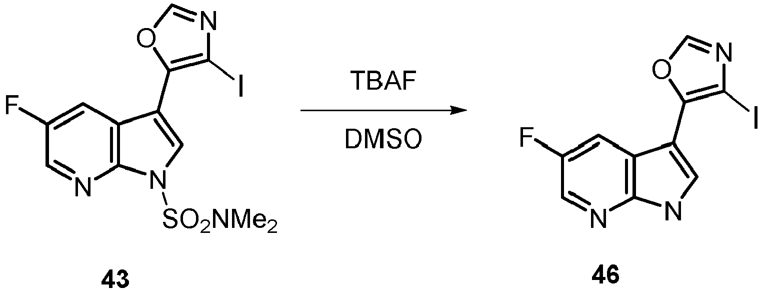

To a solution of 43 (1 g, 2.29 mmol) in DMSO (8 mL) was added TBAF (1M in THF, 9.2 mL, 9.2 mmol), and stirred for 8 h at 40°C. TLC (PE: EtOAc=1:1) showed the reaction was completed. The reaction was quenched with water (20 mL), extracted with EtOAc (20 mL x 3). The organic layer was washed with brine (20 mL x 3), dried over Na₂SO₄, filtered and concentrated to afford the crude product. Then purified by column chromatography (PE: EA=10:1∼1:1) and HPLC to get 46 (100 mg, yield: 19%).
¹H NMR (CDCl₃) Bruker_B_400MHz: δ ppm 8.05 (dd, *J*=9.41, 2.63 Hz, 1 H) 8.22 (d, *J*=2.89 Hz, 1 H) 8.35 (s, 1 H) 8.48 (s, 1 H).

### Example 10: Preparation of 4-bromo-5-(5-fluoro-1H-pyrrolo[2,3-b]pyridin-3-yl)oxazole

### Step 1:

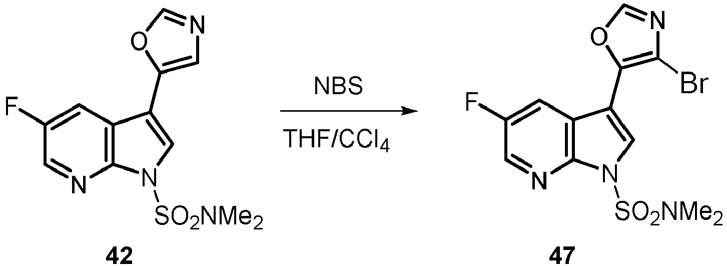

To a solution of 42 (6.5 g, 20.95 mmol) in THF/CCl₄ (60 mL /60 mL) was added NBS (4.5 g, 25.14 mmol) at 20°C under N₂. The resulting mixture was stirred for 2 h at 55°C. TLC (PE: EtOAc=1:1) showed the reaction was completed. The reaction was filtered and concentrated to afford the crude product. Then purified by column chromatography (PE: EA=10:1∼1:1) to get 47 (5.7 g, yield: 73%).
¹H NMR (400 MHz, DMSO-*d*₆): δ ppm 2.99 (s, 6 H) 8.22 - 8.25 (m, 1 H) 8.26 (s, 1 H) 8.56 - 8.59 (m, 1 H) 8.64 (s, 1 H).

### Step 2:

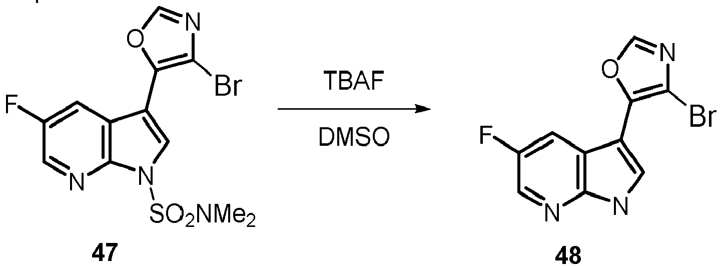

To a solution of 47 (340 mg, 0.88 mmol) in DMSO (5 mL) was added TBAF (1M in THF, 3.5 mL, 3.5 mmol), and stirred for 8 h at 40°C. TLC (PE: EtOAc=3:1) showed the reaction was completed. The reaction was quenched with water (20 mL), extracted with EtOAc (20 mL x 3). The organic layer was washed with brine (20 mL x 3), dried over Na₂SO₄, filtered and concentrated to afford the crude product. Then purified by column chromatography (PE: EA=10:1∼1:1) to get 48 (85 mg, yield: 32%).
¹H NMR (CDCl₃) Bruker_B_400MHz: δ ppm 8.07 (dd, *J*=9.35, 2.57 Hz, 1 H) 8.15 (d, *J*=2.89 Hz, 1 H) 8.36 - 8.38 (m, 1 H) 8.52 (s, 1 H).

### Example 11: Preparation of 4-(difluoromethyl)-5-(5-fluoro-1H-pyrrolo[2,3-b]pyridin-3-yl)oxazole

### Step 1:

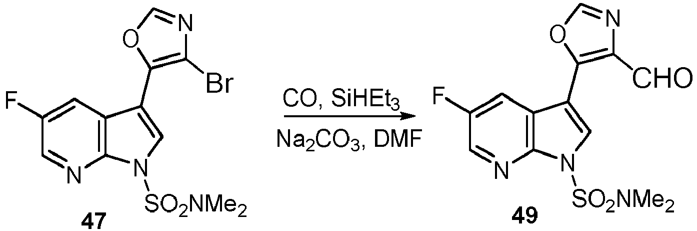

To a solution of 47 (5.7 mg, 14.7 mmol) in DMF (360 mL) was added SiHEt₃ (6.8 g, 60 mmol) and Na₂CO₃ (4.6 g, 4.41 mmol). The reaction was stirred for 10 h at 80°C, 50 psi under CO. TLC (PE: EtOAc=1:1) showed the reaction was completed. The reaction was filtered and concentrated to afford the crude product. Then purified by column chromatography (PE: EA=10:1∼1:1) to get 49 (2.4 g, yield: 48%).
¹H NMR (CDCl₃) Bruker_B_400MHz: δ ppm 3.01 (s, 6 H) 8.38 (dd, *J*=8.91, 2.76 Hz, 1 H) 8.60 (dd, *J*=2.57, 1.19 Hz, 1 H) 8.69 (s, 1 H) 9.16 (s, 1 H) 10.03 (s, 1 H).

### Step 2:

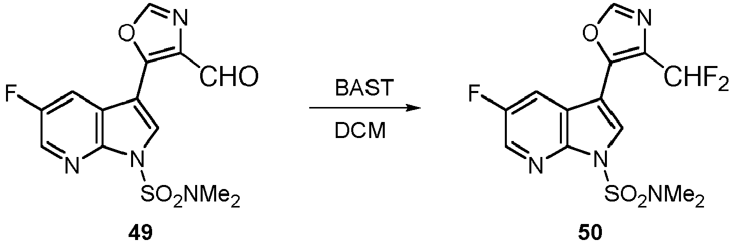

To a solution of 49 (2.9 g, 8.58 mmol) in DCM (10 mL) was added bis(2-methoxyethyl)aminosulfur trifluoride (BAST) (18.96 g, 85.8 mmol). The reaction was stirred for 1 h at 50°C. LCMS showed the reaction was completed. The reaction was quenched with aqueous NaHCO₃ (80 mL) carefully, extracted with DCM (80 mL x 3). The organic layer was washed with brine (30 mL x 3), dried over Na₂SO₄, filtered and concentrated to afford the crude product. Then purified by column chromatography (PE: EA=10:1∼1:1) to get 50 (1.1 g, yield: 36%).
¹H NMR (CDCl₃) Bruker_B_400MHz: δ ppm 3.08 - 3.10 (m, 6 H) 6.68 - 6.98 (m, 1 H) 7.99 - 8.04 (m, 2 H) 8.13 (s, 1 H) 8.37 - 8.41 (m, 1 H).

### Step 3:

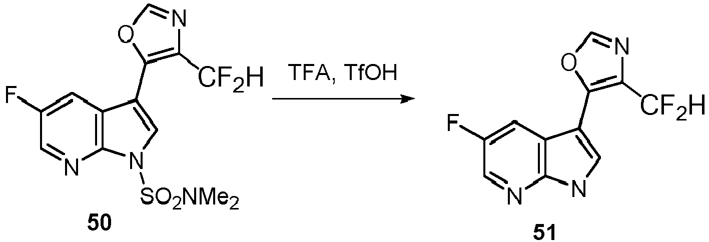

The reaction of 50 (950 mg, 2.64 mmol) in TFA/TfOH (5 mL/20 mL) was stirred for 17 h at 70°C. LCMS showed the material was remaining, but reaction was completed. The reaction was quenched with water (20 mL), extracted with EtOAc (20 mL x 3). The organic layer was adjusted for PH to 7 with NaHCO₃ aqueous, washed with brine (50 mL x 3), dried over Na₂SO₄, filtered and concentrated to afford the crude product. Then purified by column chromatography (PE: EA=10:1∼1:1) and HPLC (acid) to give the 51 (110 mg, yield: 16%).
¹H NMR (CDCl₃) Bruker_B_400MHz: δ ppm 6.81 - 7.11 (m, 1 H) 7.93 (s, 1 H) 8.07 (dd, *J*=9.10, 2.70 Hz, 1 H) 8.26 (d, *J*=2.13 Hz, 1 H) 8.31 (s, 1 H).

### Example 12: Preparation of 3-(4-(trifluoromethyl)thiazol-5-yl)-1H-indole-5-carbonitrile

### Step 1:

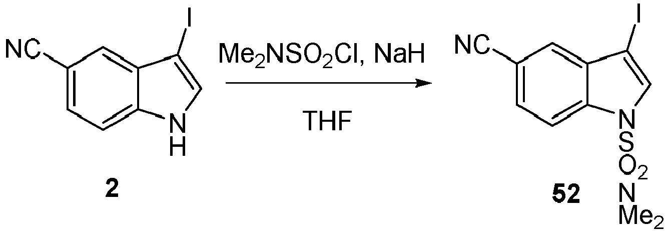

To a solution of compound 2 (77 g, 0.29 mol) in THF (700 mL) was added NaH (60% in oil, 13.8 g, 0.34 mol) at 0°C. After 60 mins, Me₂NSO₂Cl (49.3 g, 0.34 mol) was added at 0°C and the mixture was stirred at 20°C for 4 hrs. TLC (PE: EtOAc=5:1) showed the reaction was completed. The mixture was poured into H₂O and extracted with EtOAc (200 mL x 3), the organic layer was dried over Na₂SO₄, filtered and concentrated to give the crude product, which was purified by column chromatography (PE: EtOAc=5:1) to give the compound 52 (95 g, 88.7%) as a white solid.
¹H NMR: (CDCl₃) 400MHz: δ 2.90 (s, 6 H), 7.61 (d, *J* = 8.8 Hz, 1 H),7.69 (s, 1 H), 7.82 (s, 1 H), 8.02 (d, *J*=8.8 Hz,1 H).

### Step 2:

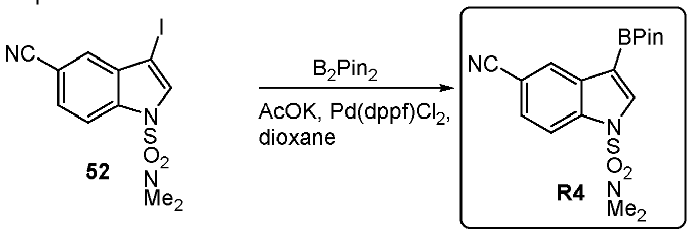

To a solution of compound 52 (5 g, 13.3 mmol) and B2Pin2 (10.2 g, 39.9 mmol) in Dioxane (50 mL) was added AcOK (3.2 g, 33.3 mmol) and Pd(dppf)Cl₂ (0.97 g, 1.33 mmol). The mixture was stirred at 90°C under N₂ for 10 hrs. TLC (PE: EtOAc=2:1) showed the reaction was completed. The mixture was poured into H₂O and extracted with EtOAc (50 mL x 3), the organic layer was dried over Na₂SO₄, filtered and concentrated to give a crude product, which was purified by column (PE: EtOAc=5:1) to get the compound R4 (3.8 g, 60%) as a yellow soil.
¹H NMR: (CDCl₃) 400MHz: δ 1.39 (s, 12 H), 2.88 (s, 6 H), 7.56 (d, J = 7.2 Hz, 1 H), 7.96 (s, 1 H), 7.99 (d, J = 7.2 Hz, 1 H), 8.40 (s, 1 H).

### Step 3:

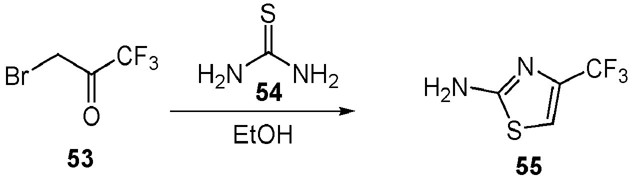

To a solution of 53 (20 g, 105 mmol) in EtOH (200 mL) was added and compound 54 (8 g, 105 mmol), the mixture was stirred at 52°C for 2 hrs. LCMS showed the reaction was completed. The mixture was concentrated, poured into H₂O, adjusted to pH=10-11 and extracted with EtOAc (50 mL x 3). The organic layer was dried over Na₂SO₄, filtered and concentrated to get 55 (15 g, 85.2%) as a yellow solid.
¹H NMR: GV-39287-3-3A CDCl3 400MHz: δ 6.26 (brs, 2 H), 6.89 (s, 1 H).

### Step 4:

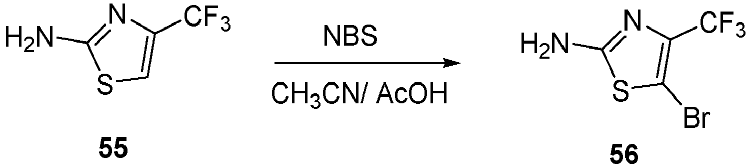

To a solution of 55 (3 g, 17.9 mmol) in CH₃CN/AcOH (20 mL /2 mL) was added NBS (3.5g, 19.7 mmol) at 0°C, the mixture was stirred at 25°C for 1 hour. TLC (PE: EtOAc=5:1) showed the reaction was completed. The mixture was poured into water and extracted with EtOAc (50 mL x 3). The organic layer was washed with sat. solution NaHCO₃, dried over Na₂SO₄, filtered and concentrated to give the crude product, which was purified by column chromatography (PE: EtOAc=10:1∼5:1) to get 56 (3.5 g, 80%) as a yellow solid.

### Step 5:

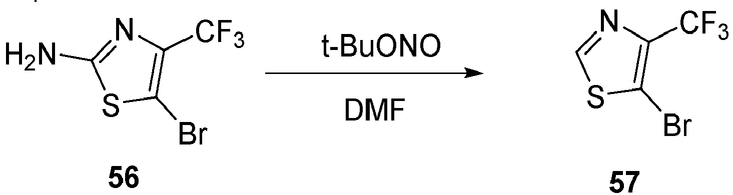

To a solution of 56 (15 g, 63 mmol) in DMF (200 mL) was added t-BuONO (7.5 g, 75 mmol) and the mixture was stirred at 80°C for 2 hrs. TLC (PE: EtOAc =3:1) showed the reaction was completed. The mixture was poured into water and extracted with EtOAc (10 mL x 3). The organic layer was dried over Na₂SO₄, filtered and concentrated to get 57 (8.3 g, 58.5%) as brown oil.
¹H NMR: (CDCl₃) 400MHz: δ 8.82 (s, 1 H).

### Step 6:

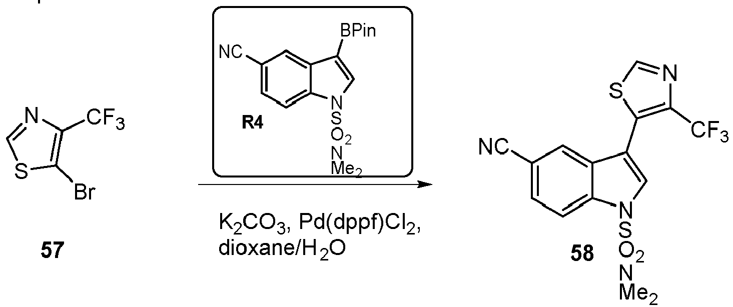

To a solution of 57 (2 g, 8.7 mmol) compound R4 (3.8 g, 10.4 mmol) in Dioxane (20 mL) and H₂O (2 mL) was added K₂CO₃ (3.5 g, 26.1 mmol) and Pd(dppf)Cl₂ (0.63 g, 0.87 mmol). The mixture was stirred at 90°C under N₂ for 10 hrs. TLC (PE: EtOAc=2:1) showed the reaction was completed. The mixture was poured into H₂O and extracted with EtOAc (20 mL x 3), the organic layer was dried over Na₂SO₄, filtered and concentrated to get the crude product, which was purified by column (PE: EtOAc=20:1-3:1) to obtain 58 (2 g, 58.8%) as a yellow soil.
¹H NMR: (CDCl₃) 400MHz: δ 2.93 (s, 6 H), 7.67 (d, J = 8.8 Hz, 1 H), 7.80 (s, 1 H), 7.95 (s, 1 H), 8.13 (d, J = 8.8 Hz, 1 H), 8.99 (s, 1 H).

### Step 7:

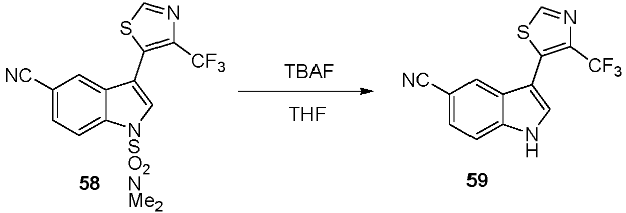

To a solution of 58 (0.8 g, 2 mmol) in THF (20 mL) was added TBAF (4 mL), and then the mixture was stirred at 80°C for 10 hrs. The mixture was poured into H₂O, extracted with EtOAc (10 mL x 3), and the organic layer was dried over Na₂SO₄, filtered and concentrated to give the crude product, which was purified by Pre-HPLC to obtain 59 (0.27 g, 58.6%) as a white solid. ¹H NMR: (DMSO) 400MHz: δ 7.57 (d, J = 8.4 Hz, 1 H), 7.68 (d, J = 8.4 Hz, 1 H), 7.90 (s, 1 H), 8.00 (s, 1 H), 9.31 (s, 1 H) 12.28 (brs, 1H).

The compounds provided in Table Q were prepared by similar methods as provided in synthesis examples.

**Table Q:**

| Compound No. | structure | LCMS retention time (RT) | LCMS method | melting point (MP) | NMR |
|---|---|---|---|---|---|
| P1 | 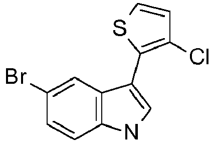 | 2.175 | 2b | | |
| P2 | 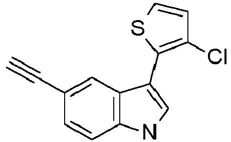 | 2.004 | 2b | | |
| P3 | 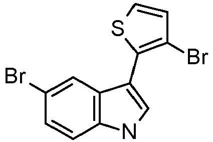 | 2.205 | 2b | | |
| P4 | 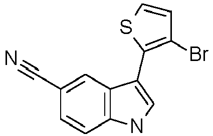 | 1.918 | 2b | | |
| P5 | 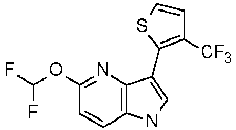 | | | | ¹H NMR CDCl₃ Bruker 400MHz: δ 8.69 (brs, 1H), 7.87-7.68 (m, 3H), 7.35 (d, 1H), 7.29 (d, 2H), 6.81 (d, 1H). |
| P6 | 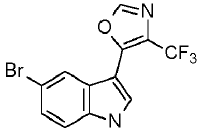 | 1.923 | 2b | | |
| P7 | 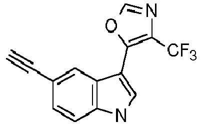 | 1.796 | 2b | | |
| P8 | 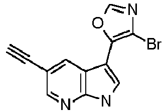 | | | | ¹H NMR MeOD Varian 400MHz: δ 3.59 (s, 1 H) 8.11 (s, 1 H) 8.26 (s, 1 H) 8.41 (d, *J*=1.96 Hz, 1 H) 8.49 (d, *J*=1.96 Hz, 1 H). |
| P9 | 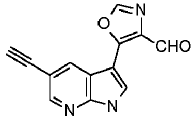 | | | | ¹H NMR MeOD Varian 400MHz: δ 3.62 (s, 1 H) 8.32 (d, *J*=0.98 Hz, 1 H) 8.44 (d, *J*=1.96 Hz, 1 H) 8.63 (d, *J*=0.98 Hz, 1 H) 8.78 (d, *J*=1.96 Hz, 1 H) 9.10 (s, 1 H). |
| P10 | 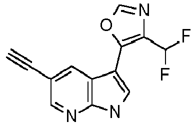 | | | | ¹H NMR MeOD Bruker 400MHz: δ 3.61 (s, 1 H) 6.81 - 7.12 (m, 1 H) 7.91 (s, 1 H) 8.33 (s, 1 H) 8.43 (s, 2 H). |
| P11 | 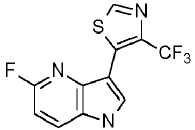 | | | | ¹H NMR MeOD 400MHz: δ 9.03 (s, 1 H), 8.01-7.97 (m, 1H), 7.89 (s, 1H), 6.91 (d, 1H). |
| P12 | 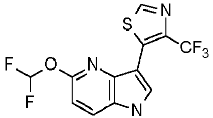 | | | | ¹H NMR MeOD 400MHz: δ 8.99 (s, 1 H), 7.95-7.90 (m, 2H), 7.65 (t, 1H), 6.85 (d, 1H). |
| P13 | 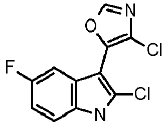 | 1.813 | 2b | | |
| P14 | 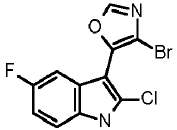 | 1.824 | 2b | | |
| P15 | 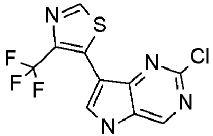 | | | | ¹H NMR CDCl₃ 400MHz: δ8.89 (s, 1H), 8.86 (s, 1H), 8.04 (s, 1H). |
| P16 | 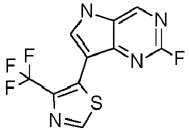 | | | | ¹H NMR CD₃CN 400MHz: δ8.99 (s, 1H), 8.86 (s, 1H), 8.10 (s, 1H). |
| P17 | 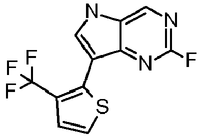 | | | | ¹H NMR CD₃CN 400MHz: δ8.83 (s, 1H), 8.04 (s, 1H), 7.57 (d, *J* = 5.6 Hz, 1H), 7.35 (d, J = 5.6 Hz, 1H). |
| P18 | 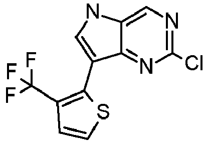 | | | | ¹H NMR CD₃CN 400MHz: δ8.87 (s, 1H), 8.02 (s, 1H), 7.59 (d, *J*=5.6 Hz, 1H), 7.36 (d, J = 5.6 Hz, 1H). |

### Biological data:

### Microtest

The active compounds were formulated separately as a stock solution having a concentration of 10000 ppm in dimethyl sulfoxide.

### 1. Activity against the grey mold Botrytis cinerea in the microtiterplate test (Botrci)

The stock solutions were mixed according to the ratio, pipetted onto a micro titer plate (MTP) and diluted with water to the stated concentrations. A spore suspension of Botrci cinerea in an aqueous biomalt or yeast-bactopeptone-sodiumacetate solution was then added. The plates were placed in a water vapor-saturated chamber at a temperature of 18°C. Using an absorption photometer, the MTPs were measured at 405 nm 7 days after the inoculation.

### 2. Activity against rice blast Pyricularia oryzae in the microtiterplate test (Pyrior)

The stock solutions were mixed according to the ratio, pipetted onto a micro titer plate (MTP) and diluted with water to the stated concentrations. A spore suspension of Pyricularia oryzae in an aqueous biomalt or yeast-bactopeptone-glycerine solution was then added. The plates were placed in a water vapor-saturated chamber at a temperature of 18°C. Using an absorption photometer, the MTPs were measured at 405 nm 7 days after the inoculation

### 3. Activity against leaf blotch on wheat caused by Septoria tritici (Septtr)

The stock solutions were mixed according to the ratio, pipetted onto a micro titer plate (MTP) and diluted with water to the stated concentrations. A spore suspension of Septoria tritici in an aqueous biomalt or yeast-bactopeptone-glycerine solution was then added. The plates were placed in a water vapor-saturated chamber at a temperature of 18°C. Using an absorption photometer, the MTPs were measured at 405 nm 7 days after the inoculation.

### 4. Activity against Fusarium culmorum (Fusacu)

The stock solutions were mixed according to the ratio, pipetted onto a micro titer plate (MTP) and diluted with water to the stated concentrations. A spore suspension of Fusarium culmorum in an aqueous biomalt or yeast-bactopeptone-glycerine solution was then added. The plates were placed in a water vapor-saturated chamber at a temperature of 18°C. Using an absorption photometer, the MTPs were measured at 405 nm 7 days after the inoculation.

The measured parameters were compared to the growth of the active compound-free control variant (100%) and the fungus-free and active compound-free blank value to determine the relative growth in % of the pathogens in the respective active compounds.

| Compound | growth (%) at 31ppm Botrci | growth (%) at 31ppm Pyrior | growth (%) at 31ppm Septtr | growth (%) at 31ppm Fusacu |
|---|---|---|---|---|
| Example 1 | | | 14 | 0 |
| Example 2 | 6 | | | 7 |
| Example 6 | 5 | | 12 | 1 |
| Example 8 | | | | 6 |
| Example 9 | | | | 11 |
| P1 | 0 | | 8 | 1 |
| P2 | 1 | | 12 | 1 |
| P3 | 0 | | 0 | 0 |
| P4 | | | | 17 |
| P5 | 1 | | 4 | 0 |
| P6 | 1 | | 2 | |
| P8 | | | 15 | 0 |
| P10 | | | | 6 |
| P17 | | | | 14 |

## Claims

1. Use of a compound of the formula (I) 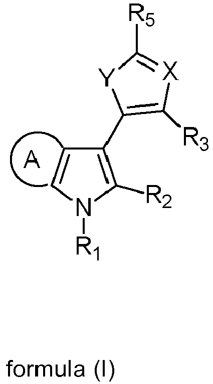 wherein,
X denotes CH or N;
Y denotes NH, O or S;
A, together with two carbon atoms of the pyrrole ring, is selected from the group consisting of 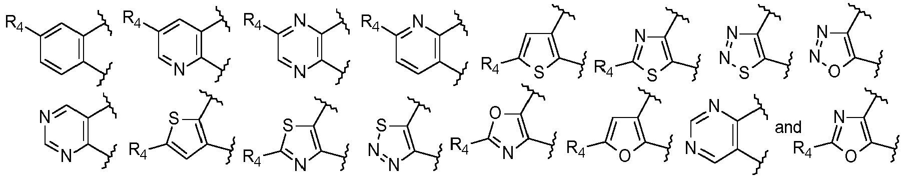 ;
R¹ is selected from the group consisting of H, -(CH₂)ₙ-OR⁶, -(CH₂)ₙ-C(=O)-R⁷, -C(=O)-(CH₂)ₙ-R⁸, -C(=O)-(CH₂)ₙ-OR⁹, -C(=O)-CR¹⁰(=CR¹¹), -C(=O)-(CH₂)ₙ-SR¹², -(CH₂)ₙ-O-C(=O)-R¹³, -(CH₂)ₙ-C(=O)-OR¹⁴, -C(=O)-O-(CH₂)ₙ-R¹⁵, -O-C(=O)-O-(CH₂)ₙ-R¹⁶, -C(=O)-NR¹⁷R¹⁸, -C(=S)-NR¹⁷R¹⁸, -N R¹⁷R¹⁸, -S(=O)ₙ-R¹⁹, -(CH₂)ₙ-Si-(R²⁰)ₙ and -(CH₂)ₙ-O-(CH₂)ₙ-Si-(R²¹)ₙ; whereby R¹ is unsubstituted or further substituted by 1, 2 or 3 identical or different groups R^{12a} which are independently selected from the group consisting of F, Cl, Br, I, OH, SH, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, C(=O)-(OH), -C(=O)-(NR¹⁷R¹⁸), -C(=O)-(H), -O-C(=O)-(C₁-C₈-alkyl), -C(=O)-(C₁-C₈-alkyl) and -C(=O)-O-(C₁-C₈-alkyl);
R² is selected from the group consisting of H; halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R² is unsubstituted or further substituted by 1, 2 or 3 identical or different groups R^{12b} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl,-CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl,-S(=O)₂-NH₂, -NH-S(=O)2-phenyl, -N(CH₃)-S(=O)2-phenyl, -Si-(C₁-C₈-alkyl)₃, -Si-(C₁-C₈-alkyl)₂-phenyl, -Si-(phenyl)₃, phenyl and -CH₂-phenyl;
R³ is selected from the group consisting of F, Cl, Br, I, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, C(=O)-(OH), C(=O)-(NR¹⁷R¹⁸), C(=O)-(H), O-C(=O)-(C₁-C₈-alkyl), -C(=O)-O-(C₁-C₈-alkyl), -O-C(=O)-(C₂-C₈-alkenyl), -C(=O)-O-(C₂-C₈-alkenyl), -O-C(=O)-(C₅-C₆-aryl), --C(=O)-O-C₅-C₆-aryl, C₃-C₈-cycloalkyl, O-C₃-C₃-cycloalkyl, -C(=O)-(C₁-C₈-alkyl), -C(=O)-(C₅-C₆-aryl), C₅-C₆-aryl, O-C₅-C₆-aryl and C₃-C₈-cycloalkenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(NR⁷R⁸), --C(=O)-(H), -O-C(=O)-(C₁-C₈-alkyl), -C(=O)-(C₁-C₈-alkyl) and -C(=O)-(O)(C₁-C₈-alkyl);
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ and R²¹ are independently of each other, selected from the group consisting of H, F, Cl, Br, I, CN, C₁-C₈-alkyl, C₂-C₈-alkenyl, C₂-C₈-alkynyl, C₃-C₈-cycloalkyl, C₅-C₆-aryl, C₅-C₆-heterocycloalkenyl, C₅-C₆-heterocycloalkyl or C₅-C₆-heteroaryl; wherein heterocycloalkenyl, heterocycloalkyl or heteroaryl contain besides carbon atoms as ring members, 1, 2 or 3 heteroatoms independently selected from O, N or S as ring members; or R¹⁷ together with R¹⁸ form a C₅-C₆-heterocycloalkenyl, C₅-C₆-heterocycloalkyl or C₅-C₆-heteroaryl;
R⁴ is selected from the group consisting of F, Br, I, CN, -C(=O)-OR¹⁴, -O-C₁-C₈-alkyl, CH₂F, C₂-C₈-alkyl, C₂-C₈-alkenyl, C₂-C₈-alkynyl, C₃-C₈-cycloalkyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁ C₆-alkyl) and C₃-C₈-cycloalkenyl whereby R⁴ is unsubstituted or further substituted by 1, 2 or 3 identical or different groups R^{12d} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl;
R⁵ is selected from the group consisting of H; halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R⁵ is unsubstituted or further substituted by 1, 2 or 3 identical or different groups R^{12e} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl,-CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl,-S(=O)₂-NH₂, -NH-S(=O)2-phenyl, -N(CH₃)-S(=O)2-phenyl, -Si-(C₁-C₈-alkyl)₃, -Si-(C₁-C₈-alkyl)₂-phenyl, -Si-(phenyl)₃, phenyl and -CH₂-phenyl; and
n is selected from 0, 1, 2, 3, 4 and 5;
or in the form of a stereoisomer or an agriculturally acceptable salt or a tautomer or an isotopic form of a N-oxide or a S-oxide or a prodrug thereof, as phytofungicide.

2. The use of the compound of formula (I) according to the claim 1, wherein R¹ is selected from the group consisting of H, -CH₂-OH, -CH₂-OCH₃, -CH₂-O-CH(CH₃)₂ -CH₂-O-(CH₂)₂-Si(CH₃)₃, -C(=O)-CH₃, -C(=O)-CH₂-CH₃, -C(=O)-(CH₂)₂-CH₃, -C(=O)-(CH₂)₃-CH₃, -C(=O)-(CH₂)₄-CH₃, -C(=O)-(CH₂)₅-CH₃, -C(=O)-C(CH₃)₃, -C(=O)-CH₂-OCH₃; -C(=O)-CH=CH-CH₃; -C(=O)-phenyl; -C(=O)-cyclopropyl; -C(=O)-CH₂-cyclopropyl; -C(=O)-CH₂-phenyl; -CH₂-O-C(=O)-CH₃; -CH₂-O-C(=O)-CH₂-CH₃; -CH₂-O-C(=O)-(CH₂)₃-CH₃; -CH₂-O-C(=O)-(CH₂)₄-CH₃; -CH₂-O-C(=O)-(CH₂)₅-CH₃; -CH₂-O-C(=O)-C(CH₃)₃; -CH₂-O-C(=O)-phenyl; -C(=O)-OCH₃; C(=O)-O-(CH₂)₂CH₃; -C(=O)-O-(CH₂)₃-CH₃; -C(=O)-O-(CH₂)₄-CH₃; -C(=O)-O-(CH₂)₅-CH₃;-C(=O)-OCH₂-CH(CH₃)₂; -C(=O)-OCH(CH₃)₂; -C(=O)-O-CH₂-CCl₃; -C(=O)-OC(CH₃)₃; -C(=O)-O-(CH₂)₆-CH₃; -C(=O)-O-CH₂-CH=CH; -C(=O)-O-CH₂-C=CH; -C(=O)O-phenyl; -C(=O)-O-CH₂-phenyl; -CH₂-C(=O)-OCH₃; -CH₂-C(=O)-OCH₂-CH₃; -CH₂-C(=O)-O(CH₂)₂-CH₃; -CH₂-C(=O)-O(CH₂)₃-CH₃; -CH₂-C(=O)-O(CH₂)₄-CH₃; -CH₂-C(=O)-O(CH₂)₅-CH₃; -CH₂-C(=O)-OC(CH₃)₃; -CH₂-C(=O)-O-CH₂-CH=CH; -CH₂-C(=O)-O-CH=CH-CH₃;-C(=O)-NH₂; -C(=O)-NH-CH₃; -C(=O)-N-(CH₃)₂; -C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)₂-CH₃;-S(=O)₂-CH₂-CH₃; -S(=O)₂-(CH₂)₂-CH₃; -S(=O)₂-(CH₂)₂-CH₃; -S(=O)₂-tert-butyl and -S(=O)₂-phenyl; whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, -C(=O)-(OH), -C(=O)-(H), -O-C(=O)-(C₁-C₈-alkyl), -C(=O)-(C₁-C₈-alkyl) and -C(=O)-(O)(C₁-C₈-alkyl).

3. The use of the compound of formula (I) according to the claim 1, wherein R² is selected from the group consisting of H, Cl, Br, F, I, CN, -C(=O)-O-CH₃, -C(=O)-O-CH₂-CH₃,-C(=O)-O-(CH₂)₃-CH₃ -C(=O)-O-(CH₂)₄-CH₃, -C(=O)-O-(CH₂)₅-CH₃, -C(=O)-O-CH(CH₃)₂,-C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl, ethynyl, propynyl; whereby R² is unsubstituted or further substituted by 1, 2 or 3 identical or different groups R^{12b} which are independently selected from the group consisting of Cl, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl,-CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl,-S(=O)₂-NH₂, -NH-S(=O)2-phenyl, -N(CH₃)-S(=O)2-phenyl, phenyl and -CH₂-phenyl.

4. The use of the compound of formula (I) according to the claim 1, wherein R³ is selected from the group consisting of Cl, F, Br, I, CN, -O-CH₃, -O-CH₂-CH₃, -C(=O)-O-CH₃; -C(=O)-O-CH₂-CH₃; -C(=O)-O-(CH₂)₂-CH_{3;} -C(=O)-O-(CH₂)₃-CH₃; -C(=O)-O-(CH₂)₄-CH₃; -C(=O)-O-(CH₂)₅-CH₃; -C(=O)-O-CH(CH₃)₂; -C(=O)-O-C(CH₃)₃; methyl, ethyl, propyl, butyl, iso-propyl, isobutyl, tert butyl, ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(H), -C(=O)-(C₁-C₈-alkyl) and -C(=O)-(O)(C₁-C₈-alkyl).

5. The use of the compound of formula (I) according to the claim 1, wherein R⁴ is selected from the group consisting of F, Br, I, CN, -C(=O)-OR¹⁴, -O-C₁-C₈-alkyl, CH₂F, C₂-C₈-alkenyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl), S(O₂)(C₁-C₆-alkyl), C₃-C₈-cycloalkenyl and C₂-C₈-alkynyl, whereby R⁴ is unsubstituted or further substituted by 1, 2 or 3 identical or different groups R^{12d} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl.

6. The use of the compound of formula (I) according to the claim 1, wherein R⁵ is selected from the group consisting of H, Cl, Br, F, I, CN, -C(=O)-O-CH₃, -C(=O)-O-CH₂-CH₃,-C(=O)-O-(CH₂)₃-CH₃ -C(=O)-O-(CH₂)₄-CH₃, -C(=O)-O-(CH₂)₅-CH₃, -C(=O)-O-CH(CH₃)₂,-C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl; whereby R⁵ is unsubstituted or further substituted by 1, 2 or 3 identical or different groups R^{12e} which are independently selected from the group consisting of Cl, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃,-S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl.

7. The use of the compound of formula (I) according to the any one of preceding claims, wherein,
X denotes CH or N;
Y denotes O or S;
A, together with two carbon atoms of the pyrrole ring, is selected from the group consisting of 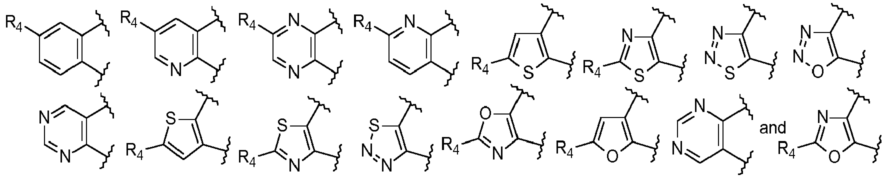 ; R¹ is H; R² is selected from the group consisting of H, F and Cl;
R³ is selected from the group consisting of F, Cl, Br, I, CN, C₁-C₈-alkyl and -C(=O)-(H); whereby R³ is unsubstituted or further substituted by 1, 2 or 3 identical or different groups R^{12c} which are independently selected from the group consisting of F, Cl, Br and I; and R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I; and
R⁵ is selected from the group consisting of H, F and Cl.

8. The use of the compound of the formula (I) according to the claim 1, wherein,
X denotes N and Y denotes O;
A, together with two carbon atoms of the pyrrole ring, is selected from the group consisting of 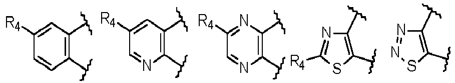 R¹ is H; R² is selected from the group consisting of H, F and Cl;
R³ is selected from the group consisting of F, Cl, Br, I, CN, C₁-C₈-alkyl and -C(=O)-(H);
whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and
R⁵ is selected from the group consisting of H, F and Cl.

9. The method of protecting a crop of useful plants susceptible to and/or under attack by fungi, which method comprises applying to said crop, treating a plant propagation material of said crop with, and/or applying to said fungi, the composition comprising at least one compound of formula (I) as defined in claim 1.

10. The use as claimed in any one of the preceding claims, which comprises treating the fungi, the plant, or the plant propagation material selected from the group consisting of seeds, roots, fruits, tubers, bulbs, rhizomes, shoots, sprouts and other parts of plants, including seedlings and young plants to be protected against fungi attack, the stored materials or harvest, or alternately, the locus or soil or soil substituents or surfaces therefrom, with an effective amount of at least one compound of formula (I), as defined in any one of the preceding claims.

11. A compound of the formula (I') 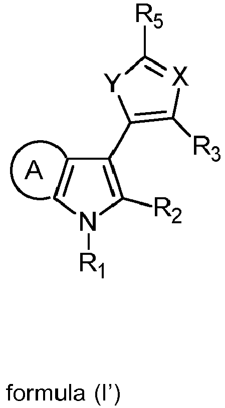 wherein,
X denotes CH or N;
Y denotes NH, O or S;
A, together with two carbon atoms of the pyrrole ring, is selected from the group consisting of 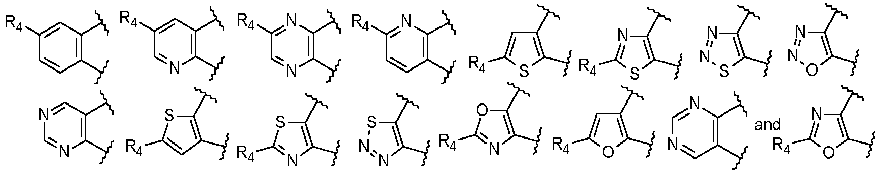 ;
R¹ is selected from the group consisting of H, -(CH₂)ₙOR⁶, -(CH₂)ₙ-C(=O)-R⁷, -C(=O)-(CH₂)ₙ-R⁸, -C(=O)-(CH₂)ₙ-OR⁹, -C(=O)-CR¹⁰(=CR¹¹), -C(=O)-(CH₂)ₙ-SR¹², -(CH₂)ₙ-O-C(=O)-R¹³, -(CH₂)ₙ-C(=O)-OR¹⁴, -C(=O)-O-(CH₂)ₙ-R¹⁵, -O-C(=O)-O-(CH₂)ₙ-R¹⁶, -C(=O)-NR¹⁷R¹⁸, -C(=S)-NR¹⁷R¹⁸, -N R¹⁷R¹⁸, -S(=O)ₙ-R¹⁹, -(CH₂)ₙ-Si-(R²⁰)ₙ and -(CH₂)ₙ-O-(CH₂)ₙ-Si-(R²¹)ₙ; whereby R¹ is unsubstituted or further substituted by 1, 2 or 3 identical or different groups R^{12a} which are independently selected from the group consisting of F, Cl, Br, I, OH, SH, CN, NO₂, NR¹⁷R¹⁸, -O-C₁-C₈-alkyl, -O-C₂-C₁₀-alkenyl, -O-C₂-C₁₀-alkynyl, C₁-C₈-alkyl, C₂-C₈-alkenyl, C₂-C₈-alkynyl, -C(=O)-(OH), -C(=O)-(NR¹⁷R¹⁸), -C(=O)-(H), -O-C(=O)-(C₁-C₈-alkyl), -C(=O)-(C₁-C₈-alkyl) and -C(=O)-(O)-(C₁-C₈-alkyl);
R² is selected from the group consisting of H, halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R² is unsubstituted or further substituted by 1, 2 or 3 identical or different groups R^{12b} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl,-CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl,-S(=O)₂-NH₂, -NH-S(=O)2-phenyl, -N(CH₃)-S(=O)2-phenyl, -Si-(C₁-C₈₋alkyl)₃, -Si-(C₁-C₈-alkyl)₂-phenyl, -Si-(phenyl)₃, phenyl and -CH₂-phenyl;
R³ is selected from the group consisting of F, Cl, Br, I, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, C=O(OH), C=O(NR¹⁷R¹⁸), C=O(H), OC=O(C₁-C₈-alkyl), C=O(O)(C₁-C₈-alkyl), OC=O(C₂-C₈-alkenyl), C=O(O)(C₂-C₈-alkenyl), OC=O(C₅-C₆-aryl), C=O(O)C₅-C₆-aryl, C₃-C₈-cycloalkyl, O-C₃-C₈-cycloalkyl, C=O(C₁-C₈-alkyl), C=O(C₅-C₆-aryl), C₅-C₆-aryl, O-C₅-C₆-aryl and C₃-C₈-cycloalkenyl; whereby R³ is unsubstituted or further substituted by 1, 2 or 3 identical or different groups R^{12c} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, C=O(OH), C=O(NR¹⁷R¹⁸), C=O(H), OC=O(C₁-C₈-alkyl), C=O(C₁-C₈-alkyl) and C=O(O)(C₁-C₈-alkyl);
R⁶, R⁷, R⁸, R⁹, R¹⁰, R¹¹, R¹², R¹³, R¹⁴, R¹⁵, R¹⁶, R¹⁷, R¹⁸, R¹⁹, R²⁰ and R²¹ are independently of each other, selected from the group consisting of H, F, Cl, Br, I, CN, C₁-C₈-alkyl, C₂-C₈-alkenyl, C₂-C₈-alkynyl, C₃-C₈-cycloalkyl, C₅-C₆-aryl, C₅-C₆-heterocycloalkenyl, C₅-C₆-heterocycloalkyl or C₅-C₆-heteroaryl; wherein heterocycloalkenyl, heterocycloalkyl or heteroaryl contain besides carbon atoms as ring members, 1, 2 or 3 heteroatoms independently selected from O, N or S as ring members; or R¹⁷ together with R¹⁸ form a C₅-C₆-heterocycloalkenyl, C₅-C₆-heterocycloalkyl or C₅-C₆-heteroaryl;
R⁴ is selected from the group consisting of F, Br, I, CN, -C(=O)-OR¹⁴, -O-C₁-C₈-alkyl, CH₂F, C₂-C₈-alkyl, C₂-C₈-alkenyl, C₂-C₈-alkynyl, C₃-C₈-cycloalkyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁-C₆-alkyl) and C₃-C₈-cycloalkenyl and C₃-C₈-cycloalkenyl whereby R⁴ is unsubstituted or further substituted by 1, 2 or 3 identical or different groups R^{12d} which are independently selected from the group consisting of F, Cl, Br, I, OH, SH, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl;
R⁵ is selected from the group consisting of H, halogen, CN, -C(=O)-OR¹⁴, C₁-C₈-alkyl, C₂-C₈-alkenyl, C₂-C₈-alkynyl, C₃-C₈-cycloalkyl and C₃-C₈-cycloalkenyl whereby R⁵ is unsubstituted or further substituted by 1, 2 or 3 identical or different groups R^{12e} which are independently selected from the group consisting of Cl, Br, F, I, CN, -OH, -SH, -NH₂, -NH(C₁-C₈)alkyl, -N(C₁-C₈-alkyl)₂, -N(C₁-C₈-alkyl)-phenyl, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl,-CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl,-S(=O)₂-NH₂, -NH-S(=O)2-phenyl, -N(CH₃)-S(=O)2-phenyl, -Si-(C₁-C₈-alkyl)₃, - -Si-(C₁-C₈-alkyl)₂-phenyl, -Si-(phenyl)₃, phenyl and -CH₂-phenyl; and
n is selected from 0, 1, 2, 3, 4 and 5;
or in the form of a stereoisomer or an agriculturally acceptable salt or a tautomer or an isotopic form of a N-oxide or a S-oxide or a prodrug thereof.

12. The compound of formula (I') according to the claim 11, wherein
R¹ is selected from the group consisting H, -CH₂-OH, -CH₂-OCH₃, -CH₂-O-CH(CH₃)_{2,}-CH₂-O-(CH₂)₂-Si(CH₃)₃, -C(=O)-CH₃, -C(=O)-CH₂-CH₃, -C(=O)-(CH₂)₂-CH₃, -C(=O)-(CH₂)₃-CH₃, -C(=O)-(CH₂)₄-CH₃, -C(=O)-(CH₂)₅-CH₃, -C(=O)-C(CH₃)₃, -C(=O)-CH₂-OCH₃; -C(=O)-CH=CH-CH₃; -C(=O)-phenyl; -C(=O)-cyclopropyl; -C(=O)-CH₂-cyclopropyl; -C(=O)-CH₂-phenyl; -CH₂-O-C(=O)-CH₃; -CH₂-O-C(=O)-CH₂-CH₃; -CH₂-O-C(=O)-(CH₂)₃-CH₃; -CH₂-O-C(=O)-(CH₂)₄-CH₃; -CH₂-O-C(=O)-(CH₂)₅-CH₃; -CH₂-O-C(=O)-C(CH₃)₃; -CH₂-O-C(=O)-phenyl; -C(=O)-OCH₃; C(=O)-O-(CH₂)₂CH₃; -C(=O)-O-(CH₂)₃-CH₃; -C(=O)-O-(CH₂)₄-CH₃;-C(=O)-O-(CH₂)₅-CH₃; -C(=O)-OCH₂-CH(CH₃)₂; -C(=O)-OCH(CH₃)₂; -C(=O)-O-CH₂-CCl₃;-C(=O)-OC(CH₃)₃; -C(=O)-O-(CH₂)₆-CH₃; -C(=O)-O-CH₂-CH=CH; -C(=O)-O-CH₂-C=CH;-C(=O)O-phenyl; -C(=O)-O-CH₂-phenyl; -CH₂-C(=O)-OCH₃; -CH₂-C(=O)-OCH₂-CH₃; -CH₂-C(=O)-O(CH₂)₂-CH₃; -CH₂-C(=O)-O(CH₂)₃-CH₃; -CH₂-C(=O)-O(CH₂)₄-CH₃; -CH₂-C(=O)-O(CH₂)₅-CH₃; -CH₂-C(=O)-OC(CH₃)₃; -CH₂-C(=O)-O-CH₂-CH=CH; -CH₂-C(=O)-O-CH=CH-CH₃; -C(=O)-NH₂; -C(=O)-NH-CH₃; -C(=O)-N-(CH₃)₂; -C(=O)-pyridyl; -C(=O)-pyrimidinyl; -C(=S)-NH₂; -C(=S)-NH-CH₃; -C(=S)-N-(CH₃)₂; -C(=S)-pyridyl; -C(=S)-pyrimidinyl; -S(=O)₂-CH₃; -S(=O)₂-CH₂-CH₃; -S(=O)₂-(CH₂)₂-CH₃; -S(=O)₂-(CH₂)₂-CH₃;-S(=O)₂-tert-butyl and -S(=O)₂-phenyl; whereby R¹ is unsubstituted or further substituted by 1, 2 or 3 identical or different groups R^{12a} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH), -C(=O)-(H), -OC(=O)-(C₁-C₈-alkyl), -C(=O)-(C₁-C₈-alkyl) and -C(=O)-(O)(C₁-C₈-alkyl);
R² is selected from the group consisting H, Cl, Br, F, I, CN, -C(=O)-O-CH₃, -C(=O)-O-CH₂-CH₃, -C(=O)-O-(CH₂)₃-CH₃ -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, ethynyl, propynyl, cyclopropyl, cyclobutyl, cyclopentyl, cyclohexyl, cyclopropenyl, cyclobutenyl, cyclopentenyl and cyclohexenyl; whereby R² is unsubstituted or further substituted by 1, 2 or 3 identical or different groups R^{12b} which are independently selected from the group consisting of Cl, Br, F, I, CN, -NH₂, -C(=O)-H, -C(=O)-C₁-C₈-alkyl, -C(=O)-OH, -C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl;
R³ is selected from the group consisting of Cl, F, Br, I, CN, -O-CH₃, -O-CH₂-CH₃, -C(=O)-O-CH₃; -C(=O)-O-CH₂-CH₃; -C(=O)-O-(CH₂)₂-CH_{3;} -C(=O)-O-(CH₂)₃-CH₃; -C(=O)-O-(CH₂)₄-CH₃; -C(=O)-O-(CH₂)₅-CH₃; -C(=O)-O-CH(CH₃)₂; -C(=O)-O-C(CH₃)₃; methyl, ethyl, propyl, butyl, isopropyl, isobutyl, tert butyl, ethenyl, propenyl, butenyl, isopropenyl, isobutenyl, ethynyl, propynyl, butynyl, cyclopropyl, cyclobutyl, cyclopentyl, cyclohexyl, cyclopropenyl, cyclobutenyl, cyclopentenyl and cyclohexenyl; whereby R³ is unsubstituted or further substituted by 1, 2 or 3 identical or different groups R^{12c} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl, C₂-C₈-alkynyl, -C(=O)-(OH),-C(=O)-(H), -C(=O)-(C₁-C₈-alkyl) and -C(=O)-(O)(C₁-C₈-alkyl);
R⁴ is selected from the group consisting of H, F, Cl, Br, I, CN, -C(=O)-OR¹⁴, CH₂F, -O-C₁-C₈-alkyl, C₁-C₈-alkenyl, -O-CN, -N-CN, -S-CN, C=(NOR⁶), C=O(NR¹⁷R¹⁸), C=O(H), OH, SH, NO₂, NR¹⁷R¹⁸, S-(C₁-C₆-alkyl) and S(O₂)(C₁-C₆-alkyl) and C₃-C₈-cycloalkenyl and C₂-C₆-alkynyl, whereby R⁴ is unsubstituted or further substituted by 1, 2 or 3 identical or different groups R^{12d} which are independently selected from the group consisting of F, Cl, Br, I, CN, NO₂, NR¹⁷R¹⁸, -O-C₁-C₈-alkyl, -O-C₂-C₈-alkenyl, -O-C₂-C₈-alkynyl, C₁-C₈-alkyl, C₂-C₈-alkenyl and C₂-C₈-alkynyl; and
R⁵ is selected from the group consisting H, Cl, Br, F, I, CN, -C(=O)-O-CH₃, -C(=O)-O-CH₂-CH₃, -C(=O)-O-(CH₂)₃-CH₃, -C(=O)-O-(CH₂)₄-CH₃, -C(=O)-O-(CH₂)₅-CH₃, -C(=O)-O-CH(CH₃)₂, -C(=O)-O-C(CH₃)₃, methyl, ethyl, propyl, butyl, hexyl, heptyl, isopropyl, isobutyl, isopentyl, tert butyl, cyclopropyl, cyclobutyl, cyclopentyl, cyclohexyl, cyclopropenyl, cyclobutenyl, cyclopentenyl and cyclohexenyl; whereby R⁵ is unsubstituted or further substituted by 1, 2 or 3 identical or different groups R^{12e} which are independently selected from the group consisting of Cl, Br, F, I, CN, -NH₂, -C(=O)-H, -C(=O)-C₁-C₈-alkyl, -C(=O)-OH,-C(=O)-O-C₁-C₈-alkyl, -C(=O)-O-phenyl, -CH₂-C(=O)-OH, -CH₂-C(=O)-C₁-C₈-alkyl, -CH₂-C(=O)-O-C₁-C₈-alkyl, -C(=O)-NH₂, -C(=O)-NH(C₁-C₈)alkyl, -C(=O)-NH-phenyl, -NH-C(=O)-C(CH₃)₃, -S(=O)-C₁-C₈-alkyl, -S(=O)-phenyl, -S(=O)₂-C₁-C₈-alkyl, -S(=O)₂-phenyl, -S(=O)₂-NH₂, -NH-S(=O)2-phenyl, -N(CH₃)-S(=O)2-phenyl, phenyl and -CH₂-phenyl;

13. The compound of formula (I') according to claim 11 or 12, wherein,
X denotes CH or N;
Y denotes O or S;
A, together with two carbon atoms of the pyrrole ring, is selected from the group consisting of 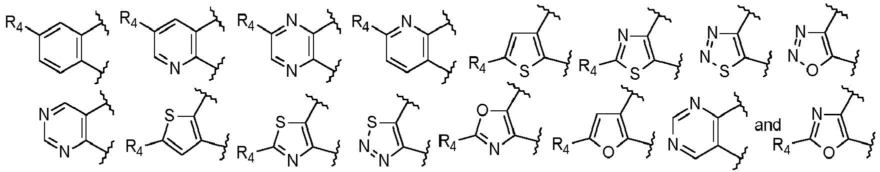 ; R¹ is H; R² is selected from the group consisting of H, F and Cl;
R³ is selected from the group consisting of F, Cl, Br, I, CN, C₁-C₈-alkyl and -C(=O)-(H);
whereby R³ is unsubstituted or further substituted by 1, 2 or 3 identical or different groups R^{12c} which are independently selected from the group consisting of F, Cl, Br and I; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are independently selected from the group consisting of F, Cl, Br and I; and
R⁵ is selected from the group consisting of H, F and Cl.

14. The compound of formula (I') according to any one of claims 11 to 13, wherein,
X denotes N and Y denotes O;
A, together with two carbon atoms of the pyrrole ring, is selected from the group consisting of 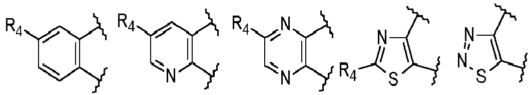 ; R¹ is H; R² is selected from the group consisting of H, F and Cl;
R³ is selected from the group consisting of F, Cl, Br, I, CN, C₁-C₈-alkyl and -C(=O)-(H);
whereby R³ is unsubstituted or further substituted by 1, 2 or 3 identical or different groups R^{12c} which are selected from the group consisting of F and Cl; and
R⁴ is selected from the group consisting of F, Br, I, CN, -O-C₁-C₈-alkyl, CH₂F, C₂-C₈-alkyl and C₂-C₈-alkynyl; whereby R⁴ is unsubstituted or further substituted by 1, 2 or 3 identical or different groups R^{12d} which are selected from the group consisting of F and Cl; and
R⁵ is selected from the group consisting of H, F and Cl.

15. An agrochemical mixture comprising at least one compound of formula (I) as defined in any of claims 1 to 10 or a compound of formula (I') according to any one of claims 11 to14, optionally in the form of a stereoisomer or an agriculturally acceptable salt or a tautomer or an isotopic form or a N-oxide or a S-oxide or a prodrug thereof.

16. A composition comprising at least one compound of formula (I) as defined in any of claims 1 to 10 or a compound of formula (I') according to any one of claims 11 to 14, optionally in the form of a stereoisomer or an agriculturally acceptable salt or a tautomer or an isotopic form of a N-oxide or a S-oxide or a prodrug thereof, and an auxiliary.
